# EUROPEAN PATENT APPLICATION

(11) **EP 2 803 733 A1**
(43) Date of publication of application: **19.11.2014**
(21) Application number: 13167540.7
(22) Date of filing: 13.05.2013
(51) Int. Cl.: C12Q 1/68

(54) **Method for determining the risk of developing severe dyspnea after radiation therapy**

(71) Applicant: Universiteit Maastricht, 6211 LK Maastricht (NL); Academisch Ziekenhuis Maastricht, 6229 HX Maastricht (NL); STICHTING MAASTRICHT RADIATION ONCOLOGY "MAASTRO CLINIC", 6229 ET Maastricht (NL)
(72) Inventor: Lambin, Philippe, 1470 Genappe-Bousval (BE); Nalbantov, Georgi Ilkov, 6227 TJ Maastricht (NL); Smeets, Hubertus Julius Maria, 6211 NS Maastricht (NL); Voets, An Mieke, 3740 Bilzen (BE)
(74) Representative: Habets, Winand

(57) **Abstract**

The invention is in the art of medical treatments, in particular the treatment of tumors with ionizing radiation. It provides means and methods for predicting whether a subject is likely to develop radiation damage upon radiotherapy. The invention provides tools that allow individualized and optimized radiation treatment of a subject in need of a radiation treatment. The invention also provides methods of determining the risk of developing severe dyspnea after radiation treatment. More in particular, the invention relates to an in vitro method for predicting the risk of developing radiation induced lung toxicity comprising the steps of obtaining mitochondrial DNA from a sample of a subject, determining the number of non-synonymous variations present in at least one gene encoding a mitochondrial protein, attributing a value to the number of non-synonymous variations, wherein a higher value corresponds to a higher risk of developing radiation induced lung toxicity.

SEQUENCE LISTING

<110>  Universiteit Maastricht, Academisch Ziekenhuis Maastricht and
Stichting Maastricht Radiation Oncology "Maastro-Clinic"

<120>  Method for determining the risk of developing severe dyspnea
       after radiation therapy.

<130>  229 EPP1

<160>  14

<170>  PatentIn version 3.5

<210>  1
<211>  16569
<212>  DNA
<213>  homo sapiens


<220>
<221>  b
<222>  (3107)..(3107)
<223>  b is a gap in order to keep this sequence aligned with previous
       reference sequences.

<400>  1
gatcacaggt ctatcaccct attaaccact cacgggagct ctccatgcat ttggtatttt      60

cgtctggggg gtatgcacgc gatagcattg cgagacgctg gagccggagc accctatgtc     120

gcagtatctg tctttgattc ctgcctcatc ctattattta tcgcacctac gttcaatatt     180

acaggcgaac atacttacta aagtgtgtta attaattaat gcttgtagga cataataata     240

acaattgaat gtctgcacag ccactttcca cacagacatc ataacaaaaa atttccacca     300

aaccccccct cccccgcttc tggccacagc acttaaacac atctctgcca aaccccaaaa     360

acaaagaacc ctaacaccag cctaaccaga tttcaaattt tatcttttgg cggtatgcac     420

ttttaacagt caccccccaa ctaacacatt attttcccct cccactccca tactactaat     480

ctcatcaata caacccccgc ccatcctacc cagcacacac acaccgctgc taaccccata     540

ccccgaacca accaaacccc aaagacaccc cccacagttt atgtagctta cctcctcaaa     600

gcaatacact gaaaatgttt agacgggctc acatcacccc ataaacaaat aggtttggtc     660

ctagcctttc tattagctct tagtaagatt acacatgcaa gcatccccgt tccagtgagt     720

tcaccctcta aatcaccacg atcaaaagga acaagcatca agcacgcagc aatgcagctc     780

aaaacgctta gcctagccac acccccacgg gaaacagcag tgattaacct ttagcaataa     840

acgaaagttt aactaagcta tactaacccc agggttggtc aatttcgtgc cagccaccgc     900

ggtcacacga ttaacccaag tcaatagaag ccggcgtaaa gagtgtttta gatcaccccc     960

tccccaataa agctaaaact cacctgagtt gtaaaaaact ccagttgaca caaaatagac    1020

tacgaaagtg gctttaacat atctgaacac acaatagcta agacccaaac tgggattaga    1080

taccccacta tgcttagccc taaacctcaa cagttaaatc aacaaaactg ctcgccagaa 1140

cactacgagc cacagcttaa aactcaaagg acctggcggt gcttcatatc cctctagagg 1200

agcctgttct gtaatcgata aaccccgatc aacctcacca cctcttgctc agcctatata 1260

ccgccatctt cagcaaaccc tgatgaaggc tacaaagtaa gcgcaagtac ccacgtaaag 1320

acgttaggtc aaggtgtagc ccatgaggtg gcaagaaatg ggctacattt tctaccccag 1380

aaaactacga tagcccttat gaaacttaag ggtcgaaggt ggatttagca gtaaactaag 1440

agtagagtgc ttagttgaac agggccctga agcgcgtaca caccgcccgt caccctcctc 1500

aagtatactt caaaggacat ttaactaaaa cccctacgca tttatataga ggagacaagt 1560

cgtaacatgg taagtgtact ggaaagtgca cttggacgaa ccagagtgta gcttaacaca 1620

aagcacccaa cttacactta ggagatttca acttaacttg accgctctga gctaaaccta 1680

gccccaaacc cactccacct tactaccaga caaccttagc caaaccattt acccaaataa 1740

agtataggcg atagaaattg aaacctggcg caatagatat agtaccgcaa gggaaagatg 1800

aaaaattata accaagcata atatagcaag gactaacccc tataccttct gcataatgaa 1860

ttaactagaa ataactttgc aaggagagcc aaagctaaga cccccgaaac cagacgagct 1920

acctaagaac agctaaaaga gcacacccgt ctatgtagca aaatagtggg aagatttata 1980

ggtagaggcg acaaacctac cgagcctggt gatagctggt tgtccaagat agaatcttag 2040

ttcaacttta aatttgccca cagaaccctc taaatcccct tgtaaattta actgttagtc 2100

caaagaggaa cagctctttg gacactagga aaaaaccttg tagagagagt aaaaaattta 2160

acacccatag taggcctaaa agcagccacc aattaagaaa gcgttcaagc tcaacaccca 2220

ctacctaaaa aatcccaaac atataactga actcctcaca cccaattgga ccaatctatc 2280

accctataga agaactaatg ttagtataag taacatgaaa acattctcct ccgcataagc 2340

ctgcgtcaga ttaaaacact gaactgacaa ttaacagccc aatatctaca atcaaccaac 2400

aagtcattat taccctcact gtcaacccaa cacaggcatg ctcataagga aaggttaaaa 2460

aaagtaaaag gaactcggca aatcttaccc cgcctgttta ccaaaaacat cacctctagc 2520

atcaccagta ttagaggcac cgcctgccca gtgacacatg tttaacggcc gcggtaccct 2580

aaccgtgcaa aggtagcata atcacttgtt ccttaaatag ggacctgtat gaatggctcc 2640

acgagggttc agctgtctct tacttttaac cagtgaaatt gacctgcccg tgaagaggcg 2700

ggcataacac agcaagacga gaagacccta tggagcttta atttattaat gcaaacagta 2760

cctaacaaac ccacaggtcc taaactacca aacctgcatt aaaaatttcg gttggggcga 2820

cctcggagca gaacccaacc tccgagcagt acatgctaag acttcaccag tcaaagcgaa 2880

ctactatact caattgatcc aataacttga ccaacggaac aagttaccct agggataaca 2940

gcgcaatcct attctagagt ccatatcaac aatagggttt acgacctcga tgttggatca 3000

ggacatcccg atggtgcagc cgctattaaa ggttcgtttg ttcaacgatt aaagtcctac 3060

gtgatctgag ttcagaccgg agtaatccag gtcggtttct atctacbttc aaattcctcc 3120

ctgtacgaaa ggacaagaga aataaggcct acttcacaaa gcgccttccc ccgtaaatga 3180

tatcatctca acttagtatt atacccacac ccacccaaga acagggtttg ttaagatggc 3240

agagcccggt aatcgcataa aacttaaaac tttacagtca gaggttcaat tcctcttctt 3300

aacaacatac ccatggccaa cctcctactc ctcattgtac ccattctaat cgcaatggca 3360

ttcctaatgc ttaccgaacg aaaaattcta ggctatatac aactacgcaa aggccccaac 3420

gttgtaggcc cctacgggct actacaaccc ttcgctgacg ccataaaact cttcaccaaa 3480

gagcccctaa aacccgccac atctaccatc accctctaca tcaccgcccc gaccttagct 3540

ctcaccatcg ctcttctact atgaaccccc ctccccatac ccaaccccct ggtcaacctc 3600

aacctaggcc tcctatttat tctagccacc tctagcctag ccgtttactc aatcctctga 3660

tcagggtgag catcaaactc aaactacgcc ctgatcggcg cactgcgagc agtagcccaa 3720

acaatctcat atgaagtcac cctagccatc attctactat caacattact aataagtggc 3780

tcctttaacc tctccaccct tatcacaaca caagaacacc tctgattact cctgccatca 3840

tgacccttgg ccataatatg atttatctcc acactagcag agaccaaccg aacccccttc 3900

gaccttgccg aaggggagtc cgaactagtc tcaggcttca acatcgaata cgccgcaggc 3960

cccttcgccc tattcttcat agccgaatac acaaacatta ttataataaa caccctcacc 4020

actacaatct tcctaggaac aacatatgac gcactctccc ctgaactcta cacaacatat 4080

tttgtcacca agaccctact tctaacctcc ctgttcttat gaattcgaac agcatacccc 4140

cgattccgct acgaccaact catacacctc ctatgaaaaa acttcctacc actcacccta 4200

gcattactta tatgatatgt ctccataccc attacaatct ccagcattcc ccctcaaacc 4260

taagaaatat gtctgataaa agagttactt tgatagagta aataatagga gcttaaaccc 4320

ccttatttct aggactatga gaatcgaacc catccctgag aatccaaaat tctccgtgcc 4380

acctatcaca ccccatccta aagtaaggtc agctaaataa gctatcgggc ccataccccg 4440

aaaatgttgg ttataccctt cccgtactaa ttaatcccct ggcccaaccc gtcatctact 4500

ctaccatctt tgcaggcaca ctcatcacag cgctaagctc gcactgattt tttacctgag 4560

taggcctaga aataaacatg ctagctttta ttccagttct aaccaaaaaa ataaaccctc 4620

gttccacaga agctgccatc aagtatttcc tcacgcaagc aaccgcatcc ataatccttc 4680

taatagctat cctcttcaac aatatactct ccggacaatg aaccataacc aatactacca 4740

atcaatactc atcattaata atcataatag ctatagcaat aaaactagga atagccccct 4800

ttcacttctg agtcccagag gttacccaag gcacccctct gacatccggc ctgcttcttc 4860

tcacatgaca aaaactagcc cccatctcaa tcatatacca aatctctccc tcactaaacg 4920

taagccttct cctcactctc tcaatcttat ccatcatagc aggcagttga ggtggattaa 4980

accaaaccca gctacgcaaa atcttagcat actcctcaat tacccacata ggatgaataa 5040

tagcagttct accgtacaac cctaacataa ccattcttaa tttaactatt tatattatcc 5100

taactactac cgcattccta ctactcaact taaactccag caccacgacc ctactactat 5160

ctcgcacctg aaacaagcta acatgactaa cacccttaat tccatccacc ctcctctccc 5220

taggaggcct gcccccgcta accggctttt tgcccaaatg ggccattatc gaagaattca 5280

caaaaaacaa tagcctcatc atccccacca tcatagccac catcaccctc cttaacctct 5340

acttctacct acgcctaatc tactccacct caatcacact actccccata tctaacaacg 5400

taaaaataaa atgacagttt gaacatacaa aacccacccc attcctcccc acactcatcg 5460

cccttaccac gctactccta cctatctccc cttttatact aataatctta tagaaattta 5520

ggttaaatac agaccaagag ccttcaaagc cctcagtaag ttgcaatact taatttctgt 5580

aacagctaag gactgcaaaa ccccactctg catcaactga acgcaaatca gccactttaa 5640

ttaagctaag cccttactag accaatggga cttaaaccca caaacactta gttaacagct 5700

aagcacccta atcaactggc ttcaatctac ttctcccgcc gccgggaaaa aaggcgggag 5760

aagccccggc aggtttgaag ctgcttcttc gaatttgcaa ttcaatatga aaatcacctc 5820

ggagctggta aaaagaggcc taacccctgt ctttagattt acagtccaat gcttcactca 5880

gccattttac ctcaccccca ctgatgttcg ccgaccgttg actattctct acaaaccaca 5940

aagacattgg aacactatac ctattattcg gcgcatgagc tggagtccta ggcacagctc 6000

taagcctcct tattcgagcc gagctgggcc agccaggcaa ccttctaggt aacgaccaca 6060

tctacaacgt tatcgtcaca gcccatgcat ttgtaataat cttcttcata gtaataccca 6120

tcataatcgg aggctttggc aactgactag ttcccctaat aatcggtgcc cccgatatgg 6180

cgtttccccg cataaacaac ataagcttct gactcttacc tccctctctc ctactcctgc 6240

tcgcatctgc tatagtggag gccggagcag gaacaggttg aacagtctac cctcccttag 6300

cagggaacta ctcccaccct ggagcctccg tagacctaac catcttctcc ttacacctag 6360

caggtgtctc ctctatctta ggggccatca atttcatcac aacaattatc aatataaaac 6420

cccctgccat aacccaatac caaacgcccc tcttcgtctg atccgtccta atcacagcag 6480

tcctacttct cctatctctc ccagtcctag ctgctggcat cactatacta ctaacagacc 6540

gcaacctcaa caccaccttc ttcgaccccg ccggaggagg agaccccatt ctataccaac 6600

acctattctg atttttcggt caccctgaag tttatattct tatcctacca ggcttcggaa 6660

taatctccca tattgtaact tactactccg gaaaaaaaga accatttgga tacataggta 6720

tggtctgagc tatgatatca attggcttcc tagggtttat cgtgtgagca caccatatat 6780

ttacagtagg aatagacgta gacacacgag catatttcac ctccgctacc ataatcatcg 6840

ctatccccac cggcgtcaaa gtatttagct gactcgccac actccacgga agcaatatga 6900

aatgatctgc tgcagtgctc tgagccctag gattcatctt tcttttcacc gtaggtggcc 6960

tgactggcat tgtattagca aactcatcac tagacatcgt actacacgac acgtactacg 7020

ttgtagccca cttccactat gtcctatcaa taggagctgt atttgccatc ataggaggct 7080

tcattcactg atttccccta ttctcaggct acaccctaga ccaaacctac gccaaaatcc 7140

atttcactat catattcatc ggcgtaaatc taactttctt cccacaacac tttctcggcc 7200

tatccggaat gccccgacgt tactcggact accccgatgc atacaccaca tgaaacatcc 7260

tatcatctgt aggctcattc atttctctaa cagcagtaat attaataatt ttcatgattt 7320

gagaagcctt cgcttcgaag cgaaaagtcc taatagtaga agaaccctcc ataaacctgg 7380

agtgactata tggatgcccc ccaccctacc acacattcga agaacccgta tacataaaat 7440

ctagacaaaa aaggaaggaa tcgaaccccc caaagctggt ttcaagccaa ccccatggcc 7500

tccatgactt tttcaaaaag gtattagaaa aaccatttca taactttgtc aaagttaaat 7560

tataggctaa atcctatata tcttaatggc acatgcagcg caagtaggtc tacaagacgc 7620

tacttcccct atcatagaag agcttatcac ctttcatgat cacgccctca taatcatttt 7680

ccttatctgc ttcctagtcc tgtatgccct tttcctaaca ctcacaacaa aactaactaa 7740

tactaacatc tcagacgctc aggaaataga aaccgtctga actatcctgc ccgccatcat 7800

cctagtcctc atcgccctcc catccctacg catcctttac ataacagacg aggtcaacga 7860

tccctccctt accatcaaat caattggcca ccaatggtac tgaacctacg agtacaccga 7920

ctacggcgga ctaatcttca actcctacat acttccccca ttattcctag aaccaggcga 7980

cctgcgactc cttgacgttg acaatcgagt agtactcccg attgaagccc ccattcgtat 8040

aataattaca tcacaagacg tcttgcactc atgagctgtc cccacattag gcttaaaaac 8100

agatgcaatt cccggacgtc taaaccaaac cactttcacc gctacacgac cgggggtata 8160

ctacggtcaa tgctctgaaa tctgtggagc aaaccacagt ttcatgccca tcgtcctaga 8220

attaattccc ctaaaaatct ttgaaatagg gcccgtattt accctatagc accccctcta 8280

ccccctctag agcccactgt aaagctaact tagcattaac cttttaagtt aaagattaag 8340

agaaccaaca cctctttaca gtgaaatgcc ccaactaaat actaccgtat ggcccaccat 8400

aattaccccc atactcctta cactattcct catcacccaa ctaaaaatat taaacacaaa 8460

ctaccaccta cctccctcac caaagcccat aaaaataaaa aattataaca aaccctgaga 8520

accaaaatga acgaaaatct gttcgcttca ttcattgccc ccacaatcct aggcctaccc 8580

gccgcagtac tgatcattct atttccccct ctattgatcc ccacctccaa atatctcatc 8640

aacaaccgac taatcaccac ccaacaatga ctaatcaaac taacctcaaa acaaatgata 8700

accatacaca acactaaagg acgaacctga tctcttatac tagtatcctt aatcattttt 8760

attgccacaa ctaacctcct cggactcctg cctcactcat ttacaccaac cacccaacta 8820

tctataaacc tagccatggc catcccctta tgagcgggca cagtgattat aggctttcgc 8880

tctaagatta aaaatgccct agcccacttc ttaccacaag gcacacctac accccttatc 8940

cccatactag ttattatcga aaccatcagc ctactcattc aaccaatagc cctggccgta 9000

cgcctaaccg ctaacattac tgcaggccac ctactcatgc acctaattgg aagcgccacc 9060

ctagcaatat caaccattaa ccttccctct acacttatca tcttcacaat tctaattcta 9120

ctgactatcc tagaaatcgc tgtcgcctta atccaagcct acgttttcac acttctagta 9180

agcctctacc tgcacgacaa cacataatga cccaccaatc acatgcctat catatagtaa 9240

aacccagccc atgaccccta acaggggccc tctcagccct cctaatgacc tccggcctag 9300

ccatgtgatt tcacttccac tccataacgc tcctcatact aggcctacta accaacacac 9360

taaccatata ccaatgatgg cgcgatgtaa cacgagaaag cacataccaa ggccaccaca 9420

caccacctgt ccaaaaaggc cttcgatacg ggataatcct atttattacc tcagaagttt 9480

ttttcttcgc aggatttttc tgagcctttt accactccag cctagcccct accccccaat 9540

taggagggca ctggccccca acaggcatca ccccgctaaa tcccctagaa gtcccactcc 9600

taaacacatc cgtattactc gcatcaggag tatcaatcac ctgagctcac catagtctaa 9660

tagaaaacaa ccgaaaccaa ataattcaag cactgcttat tacaatttta ctgggtctct 9720

attttaccct cctacaagcc tcagagtact tcgagtctcc cttcaccatt tccgacggca 9780

tctacggctc aacatttttt gtagccacag gcttccacgg acttcacgtc attattggct 9840

caactttcct cactatctgc ttcatccgcc aactaatatt tcactttaca tccaaacatc 9900

actttggctt cgaagccgcc gcctgatact ggcattttgt agatgtggtt tgactatttc 9960

tgtatgtctc catctattga tgagggtctt actcttttag tataaatagt accgttaact 10020

tccaattaac tagttttgac aacattcaaa aaagagtaat aaacttcgcc ttaattttaa 10080

taatcaacac cctcctagcc ttactactaa taattattac attttgacta ccacaactca 10140

acggctacat agaaaaatcc accccttacg agtgcggctt cgaccctata tcccccgccc 10200

gcgtcccttt ctccataaaa ttcttcttag tagctattac cttcttatta tttgatctag 10260

aaattgccct ccttttaccc ctaccatgag ccctacaaac aactaacctg ccactaatag 10320

ttatgtcatc cctcttatta atcatcatcc tagccctaag tctggcctat gagtgactac 10380

aaaaaggatt agactgaacc gaattggtat atagtttaaa caaaacgaat gatttcgact 10440

cattaaatta tgataatcat atttaccaaa tgcccctcat ttacataaat attatactag 10500

catttaccat ctcacttcta ggaatactag tatatcgctc acacctcata tcctccctac 10560

tatgcctaga aggaataata ctatcgctgt tcattatagc tactctcata accctcaaca 10620

cccactccct cttagccaat attgtgccta ttgccatact agtctttgcc gcctgcgaag 10680

cagcggtggg cctagcccta ctagtctcaa tctccaacac atatggccta gactacgtac 10740

ataacctaaa cctactccaa tgctaaaact aatcgtccca acaattatat tactaccact 10800

gacatgactt tccaaaaaac acataatttg aatcaacaca accacccaca gcctaattat 10860

tagcatcatc cctctactat tttttaacca aatcaacaac aacctattta gctgttcccc 10920

aaccttttcc tccgaccccc taacaacccc cctcctaata ctaactacct gactcctacc 10980

cctcacaatc atggcaagcc aacgccactt atccagtgaa ccactatcac gaaaaaaact 11040

ctacctctct atactaatct ccctacaaat ctccttaatt ataacattca cagccacaga 11100

actaatcata ttttatatct tcttcgaaac cacacttatc cccaccttgg ctatcatcac 11160

ccgatgaggc aaccagccag aacgcctgaa cgcaggcaca tacttcctat tctacaccct 11220

agtaggctcc cttcccctac tcatcgcact aatttacact cacaacaccc taggctcact 11280

aaacattcta ctactcactc tcactgccca agaactatca aactcctgag ccaacaactt 11340

aatatgacta gcttacacaa tagcttttat agtaaagata cctctttacg gactccactt 11400

atgactccct aaagcccatg tcgaagcccc catcgctggg tcaatagtac ttgccgcagt 11460

actcttaaaa ctaggcggct atggtataat acgcctcaca ctcattctca accccctgac 11520

aaaacacata gcctacccct tccttgtact atccctatga ggcataatta taacaagctc 11580

catctgccta cgacaaacag acctaaaatc gctcattgca tactcttcaa tcagccacat 11640

agccctcgta gtaacagcca ttctcatcca aaccccctga agcttcaccg gcgcagtcat 11700

tctcataatc gcccacgggc ttacatcctc attactattc tgcctagcaa actcaaacta 11760

cgaacgcact cacagtcgca tcataatcct ctctcaagga cttcaaactc tactcccact 11820

aatagctttt tgatgacttc tagcaagcct cgctaacctc gccttacccc ccactattaa 11880

cctactggga gaactctctg tgctagtaac cacgttctcc tgatcaaata tcactctcct 11940

acttacagga ctcaacatac tagtcacagc cctatactcc ctctacatat ttaccacaac 12000

acaatggggc tcactcaccc accacattaa caacataaaa ccctcattca cacgagaaaa 12060

caccctcatg ttcatacacc tatcccccat tctcctccta tccctcaacc ccgacatcat 12120

taccgggttt tcctcttgta aatatagttt aaccaaaaca tcagattgtg aatctgacaa 12180

cagaggctta cgacccctta tttaccgaga aagctcacaa gaactgctaa ctcatgcccc 12240

catgtctaac aacatggctt tctcaacttt taaaggataa cagctatcca ttggtcttag 12300

gccccaaaaa ttttggtgca actccaaata aaagtaataa ccatgcacac tactataacc 12360

accctaaccc tgacttccct aattcccccc atccttacca ccctcgttaa ccctaacaaa 12420

aaaaactcat acccccatta tgtaaaatcc attgtcgcat ccacctttat tatcagtctc 12480

ttccccacaa caatattcat gtgcctagac caagaagtta ttatctcgaa ctgacactga 12540

gccacaaccc aaacaaccca gctctcccta agcttcaaac tagactactt ctccataata 12600

ttcatccctg tagcattgtt cgttacatgg tccatcatag aattctcact gtgatatata 12660

aactcagacc caaacattaa tcagttcttc aaatatctac tcatcttcct aattaccata 12720

ctaatcttag ttaccgctaa caacctattc caactgttca tcggctgaga gggcgtagga 12780

attatatcct tcttgctcat cagttgatga tacgcccgag cagatgccaa cacagcagcc 12840

attcaagcaa tcctatacaa ccgtatcggc gatatcggtt tcatcctcgc cttagcatga 12900

tttatcctac actccaactc atgagaccca caacaaatag cccttctaaa cgctaatcca 12960

agcctcaccc cactactagg cctcctccta gcagcagcag gcaaatcagc ccaattaggt 13020

ctccacccct gactcccctc agccatagaa ggccccaccc cagtctcagc cctactccac 13080

tcaagcacta tagttgtagc aggaatcttc ttactcatcc gcttccaccc cctagcagaa 13140

aatagcccac taatccaaac tctaacacta tgcttaggcg ctatcaccac tctgttcgca 13200

gcagtctgcg cccttacaca aaatgacatc aaaaaaatcg tagccttctc cacttcaagt 13260

caactaggac tcataatagt tacaatcggc atcaaccaac cacacctagc attcctgcac 13320

atctgtaccc acgccttctt caaagccata ctatttatgt gctccgggtc catcatccac 13380

aaccttaaca atgaacaaga tattcgaaaa ataggaggac tactcaaaac catacctctc 13440

acttcaacct ccctcaccat tggcagccta gcattagcag gaataccttt cctcacaggt 13500

ttctactcca aagaccacat catcgaaacc gcaaacatat catacacaaa cgcctgagcc 13560

ctatctatta ctctcatcgc tacctccctg acaagcgcct atagcactcg aataattctt 13620

ctcaccctaa caggtcaacc tcgcttcccc acccttacta acattaacga aaataacccc 13680

accctactaa accccattaa acgcctggca gccggaagcc tattcgcagg atttctcatt 13740

actaacaaca tttcccccgc atcccccttc caaacaacaa tccccctcta cctaaaactc 13800

acagccctcg ctgtcacttt cctaggactt ctaacagccc tagacctcaa ctacctaacc 13860

aacaaactta aaataaaatc cccactatgc acattttatt tctccaacat actcggattc 13920

taccctagca tcacacaccg cacaatcccc tatctaggcc ttcttacgag ccaaaacctg 13980

cccctactcc tcctagacct aacctgacta gaaaagctat tacctaaaac aatttcacag 14040

caccaaatct ccacctccat catcacctca acccaaaaag gcataattaa actttacttc 14100

ctctctttct tcttcccact catcctaacc ctactcctaa tcacataacc tattcccccg 14160

agcaatctca attacaatat atacaccaac aaacaatgtt caaccagtaa ctactactaa 14220

tcaacgccca taatcataca aagcccccgc accaatagga tcctcccgaa tcaaccctga 14280

cccctctcct tcataaatta ttcagcttcc tacactatta aagtttacca caaccaccac 14340

cccatcatac tctttcaccc acagcaccaa tcctacctcc atcgctaacc ccactaaaac 14400

actcaccaag acctcaaccc ctgaccccca tgcctcagga tactcctcaa tagccatcgc 14460

tgtagtatat ccaaagacaa ccatcattcc ccctaaataa attaaaaaaa ctattaaacc 14520

catataacct cccccaaaat tcagaataat aacacacccg accacaccgc taacaatcaa 14580

tactaaaccc ccataaatag gagaaggctt agaagaaaac cccacaaacc ccattactaa 14640

acccacactc aacagaaaca aagcatacat cattattctc gcacggacta caaccacgac 14700

caatgatatg aaaaaccatc gttgtatttc aactacaaga acaccaatga ccccaatacg 14760

caaaactaac cccctaataa aattaattaa ccactcattc atcgacctcc ccaccccatc 14820

caacatctcc gcatgatgaa acttcggctc actccttggc gcctgcctga tcctccaaat 14880

caccacagga ctattcctag ccatgcacta ctcaccagac gcctcaaccg ccttttcatc 14940

aatcgcccac atcactcgag acgtaaatta tggctgaatc atccgctacc ttcacgccaa 15000

tggcgcctca atattcttta tctgcctctt cctacacatc gggcgaggcc tatattacgg 15060

atcatttctc tactcagaaa cctgaaacat cggcattatc ctcctgcttg caactatagc 15120

aacagccttc ataggctatg tcctcccgtg aggccaaata tcattctgag gggccacagt 15180

aattacaaac ttactatccg ccatcccata cattgggaca gacctagttc aatgaatctg 15240

aggaggctac tcagtagaca gtcccaccct cacacgattc tttacctttc acttcatctt 15300

gcccttcatt attgcagccc tagcaacact ccacctccta ttcttgcacg aaacgggatc 15360

aaacaacccc ctaggaatca cctcccattc cgataaaatc accttccacc cttactacac 15420

aatcaaagac gccctcggct tacttctctt ccttctctcc ttaatgacat taacactatt 15480

ctcaccagac ctcctaggcg acccagacaa ttatacccta gccaacccct taaacacccc 15540

tccccacatc aagcccgaat gatatttcct attcgcctac acaattctcc gatccgtccc 15600

taacaaacta ggaggcgtcc ttgccctatt actatccatc ctcatcctag caataatccc 15660

catcctccat atatccaaac aacaaagcat aatatttcgc ccactaagcc aatcacttta 15720

ttgactccta gccgcagacc tcctcattct aacctgaatc ggaggacaac cagtaagcta 15780

cccttttacc atcattggac aagtagcatc cgtactatac ttcacaacaa tcctaatcct 15840

aataccaact atctccctaa ttgaaaacaa aatactcaaa tgggcctgtc cttgtagtat 15900

aaactaatac accagtcttg taaaccggag atgaaaacct ttttccaagg acaaatcaga 15960

gaaaaagtct ttaactccac cattagcacc caaagctaag attctaattt aaactattct 16020

ctgttctttc atggggaagc agatttgggt accacccaag tattgactca cccatcaaca 16080

accgctatgt atttcgtaca ttactgccag ccaccatgaa tattgtacgg taccataaat 16140

acttgaccac ctgtagtaca taaaaaccca atccacatca aaaccccctc cccatgctta 16200

caagcaagta cagcaatcaa ccctcaacta tcacacatca actgcaactc caaagccacc 16260

cctcacccac taggatacca acaaacctac ccacccttaa cagtacatag tacataaagc 16320

catttaccgt acatagcaca ttacagtcaa atcccttctc gtccccatgg atgacccccc 16380

tcagataggg gtcccttgac caccatcctc cgtgaaatca atatcccgca caagagtgct 16440

actctcctcg ctccgggccc ataacacttg ggggtagcta aagtgaactg tatccgacat 16500

ctggttccta cttcagggtc ataaagccta aatagcccac acgttcccct taaataagac 16560

atcacgatg 16569

<210> 2
<211> 318
<212> PRT
<213> homo sapiens

<400> 2

Met Pro Met Ala Asn Leu Leu Leu Leu Ile Val Pro Ile Leu Ile Ala
1               5                   10                  15

Met Ala Phe Leu Met Leu Thr Glu Arg Lys Ile Leu Gly Tyr Met Gln
            20                  25                  30

Leu Arg Lys Gly Pro Asn Val Val Gly Pro Tyr Gly Leu Leu Gln Pro
        35                  40                  45

Phe Ala Asp Ala Met Lys Leu Phe Thr Lys Glu Pro Leu Lys Pro Ala
    50                  55                  60

Thr Ser Thr Ile Thr Leu Tyr Ile Thr Ala Pro Thr Leu Ala Leu Thr
65                  70                  75                  80

Ile Ala Leu Leu Leu Trp Thr Pro Leu Pro Met Pro Asn Pro Leu Val
                85                  90                  95

Asn Leu Asn Leu Gly Leu Leu Phe Ile Leu Ala Thr Ser Ser Leu Ala
            100                 105                 110

Val Tyr Ser Ile Leu Trp Ser Gly Trp Ala Ser Asn Ser Asn Tyr Ala
        115                 120                 125

Leu Ile Gly Ala Leu Arg Ala Val Ala Gln Thr Ile Ser Tyr Glu Val
    130                 135                 140

Thr Leu Ala Ile Ile Leu Leu Ser Thr Leu Leu Met Ser Gly Ser Phe
145                 150                 155                 160

Asn Leu Ser Thr Leu Ile Thr Thr Gln Glu His Leu Trp Leu Leu Leu

165                 170                 175

Pro Ser Trp Pro Leu Ala Met Met Trp Phe Ile Ser Thr Leu Ala Glu
            180                 185                 190

Thr Asn Arg Thr Pro Phe Asp Leu Ala Glu Gly Glu Ser Glu Leu Val
        195                 200                 205

Ser Gly Phe Asn Ile Glu Tyr Ala Ala Gly Pro Phe Ala Leu Phe Phe
    210                 215                 220

Met Ala Glu Tyr Thr Asn Ile Ile Met Met Asn Thr Leu Thr Thr Thr
225                 230                 235                 240

Ile Phe Leu Gly Thr Thr Tyr Asp Ala Leu Ser Pro Glu Leu Tyr Thr
                245                 250                 255

Thr Tyr Phe Val Thr Lys Thr Leu Leu Leu Thr Ser Leu Phe Leu Trp
            260                 265                 270

Ile Arg Thr Ala Tyr Pro Arg Phe Arg Tyr Asp Gln Leu Met His Leu
        275                 280                 285

Leu Trp Lys Asn Phe Leu Pro Leu Thr Leu Ala Leu Leu Met Trp Tyr
    290                 295                 300

Val Ser Met Pro Ile Thr Ile Ser Ser Ile Pro Pro Gln Thr
305                 310                 315

<210>  3
<211>  347
<212>  PRT
<213>  homo sapiens

<400>  3

Met Asn Pro Leu Ala Gln Pro Val Ile Tyr Ser Thr Ile Phe Ala Gly
1               5                   10                  15

Thr Leu Ile Thr Ala Leu Ser Ser His Trp Phe Phe Thr Trp Val Gly
            20                  25                  30

Leu Glu Met Asn Met Leu Ala Phe Ile Pro Val Leu Thr Lys Lys Met
        35                  40                  45

Asn Pro Arg Ser Thr Glu Ala Ala Ile Lys Tyr Phe Leu Thr Gln Ala
    50                  55                  60

Thr Ala Ser Met Ile Leu Leu Met Ala Ile Leu Phe Asn Asn Met Leu

65                  70                  75                  80

Ser Gly Gln Trp Thr Met Thr Asn Thr Thr Asn Gln Tyr Ser Ser Leu
                85                  90                  95

Met Ile Met Met Ala Met Ala Met Lys Leu Gly Met Ala Pro Phe His
            100                 105                 110

Phe Trp Val Pro Glu Val Thr Gln Gly Thr Pro Leu Thr Ser Gly Leu
        115                 120                 125

Leu Leu Leu Thr Trp Gln Lys Leu Ala Pro Ile Ser Ile Met Tyr Gln
    130                 135                 140

Ile Ser Pro Ser Leu Asn Val Ser Leu Leu Leu Thr Leu Ser Ile Leu
145                 150                 155                 160

Ser Ile Met Ala Gly Ser Trp Gly Gly Leu Asn Gln Thr Gln Leu Arg
                165                 170                 175

Lys Ile Leu Ala Tyr Ser Ser Ile Thr His Met Gly Trp Met Met Ala
            180                 185                 190

Val Leu Pro Tyr Asn Pro Asn Met Thr Ile Leu Asn Leu Thr Ile Tyr
        195                 200                 205

Ile Ile Leu Thr Thr Thr Ala Phe Leu Leu Leu Asn Leu Asn Ser Ser
    210                 215                 220

Thr Thr Thr Leu Leu Leu Ser Arg Thr Trp Asn Lys Leu Thr Trp Leu
225                 230                 235                 240

Thr Pro Leu Ile Pro Ser Thr Leu Leu Ser Leu Gly Gly Leu Pro Pro
                245                 250                 255

Leu Thr Gly Phe Leu Pro Lys Trp Ala Ile Ile Glu Glu Phe Thr Lys
            260                 265                 270

Asn Asn Ser Leu Ile Ile Pro Thr Ile Met Ala Thr Ile Thr Leu Leu
        275                 280                 285

Asn Leu Tyr Phe Tyr Leu Arg Leu Ile Tyr Ser Thr Ser Ile Thr Leu
    290                 295                 300

Leu Pro Met Ser Asn Asn Val Lys Met Lys Trp Gln Phe Glu His Thr
305                 310                 315                 320

Lys Pro Thr Pro Phe Leu Pro Thr Leu Ile Ala Leu Thr Thr Leu Leu
                325                 330                 335

Leu Pro Ile Ser Pro Phe Met Leu Met Ile Leu
            340                 345

<210>  4
<211>  513
<212>  PRT
<213>  homo sapiens

<400>  4

Met Phe Ala Asp Arg Trp Leu Phe Ser Thr Asn His Lys Asp Ile Gly
1               5                   10                  15

Thr Leu Tyr Leu Leu Phe Gly Ala Trp Ala Gly Val Leu Gly Thr Ala
            20                  25                  30

Leu Ser Leu Leu Ile Arg Ala Glu Leu Gly Gln Pro Gly Asn Leu Leu
        35                  40                  45

Gly Asn Asp His Ile Tyr Asn Val Ile Val Thr Ala His Ala Phe Val
    50                  55                  60

Met Ile Phe Phe Met Val Met Pro Ile Met Ile Gly Gly Phe Gly Asn
65                  70                  75                  80

Trp Leu Val Pro Leu Met Ile Gly Ala Pro Asp Met Ala Phe Pro Arg
                85                  90                  95

Met Asn Asn Met Ser Phe Trp Leu Leu Pro Pro Ser Leu Leu Leu Leu
            100                 105                 110

Leu Ala Ser Ala Met Val Glu Ala Gly Ala Gly Thr Gly Trp Thr Val
        115                 120                 125

Tyr Pro Pro Leu Ala Gly Asn Tyr Ser His Pro Gly Ala Ser Val Asp
    130                 135                 140

Leu Thr Ile Phe Ser Leu His Leu Ala Gly Val Ser Ser Ile Leu Gly
145                 150                 155                 160

Ala Ile Asn Phe Ile Thr Thr Ile Ile Asn Met Lys Pro Pro Ala Met
                165                 170                 175

Thr Gln Tyr Gln Thr Pro Leu Phe Val Trp Ser Val Leu Ile Thr Ala
            180                 185                 190

Val Leu Leu Leu Leu Ser Leu Pro Val Leu Ala Ala Gly Ile Thr Met
        195                 200                 205

Leu Leu Thr Asp Arg Asn Leu Asn Thr Thr Phe Phe Asp Pro Ala Gly
    210                 215                 220

Gly Gly Asp Pro Ile Leu Tyr Gln His Leu Phe Trp Phe Phe Gly His
225                 230                 235                 240

Pro Glu Val Tyr Ile Leu Ile Leu Pro Gly Phe Gly Met Ile Ser His
                245                 250                 255

Ile Val Thr Tyr Tyr Ser Gly Lys Lys Glu Pro Phe Gly Tyr Met Gly
            260                 265                 270

Met Val Trp Ala Met Met Ser Ile Gly Phe Leu Gly Phe Ile Val Trp
        275                 280                 285

Ala His His Met Phe Thr Val Gly Met Asp Val Asp Thr Arg Ala Tyr
    290                 295                 300

Phe Thr Ser Ala Thr Met Ile Ile Ala Ile Pro Thr Gly Val Lys Val
305                 310                 315                 320

Phe Ser Trp Leu Ala Thr Leu His Gly Ser Asn Met Lys Trp Ser Ala
                325                 330                 335

Ala Val Leu Trp Ala Leu Gly Phe Ile Phe Leu Phe Thr Val Gly Gly
            340                 345                 350

Leu Thr Gly Ile Val Leu Ala Asn Ser Ser Leu Asp Ile Val Leu His
        355                 360                 365

Asp Thr Tyr Tyr Val Val Ala His Phe His Tyr Val Leu Ser Met Gly
    370                 375                 380

Ala Val Phe Ala Ile Met Gly Gly Phe Ile His Trp Phe Pro Leu Phe
385                 390                 395                 400

Ser Gly Tyr Thr Leu Asp Gln Thr Tyr Ala Lys Ile His Phe Thr Ile
                405                 410                 415

Met Phe Ile Gly Val Asn Leu Thr Phe Phe Pro Gln His Phe Leu Gly
            420                 425                 430

Leu Ser Gly Met Pro Arg Arg Tyr Ser Asp Tyr Pro Asp Ala Tyr Thr
        435                 440                 445

Thr Trp Asn Ile Leu Ser Ser Val Gly Ser Phe Ile Ser Leu Thr Ala
    450                 455                 460

Val Met Leu Met Ile Phe Met Ile Trp Glu Ala Phe Ala Ser Lys Arg
465                 470                 475                 480

Lys Val Leu Met Val Glu Glu Pro Ser Met Asn Leu Glu Trp Leu Tyr
                485                 490                 495

Gly Cys Pro Pro Pro Tyr His Thr Phe Glu Glu Pro Val Tyr Met Lys
            500                 505                 510

Ser

<210>  5
<211>  227
<212>  PRT
<213>  homo sapiens

<400>  5

Met Ala His Ala Ala Gln Val Gly Leu Gln Asp Ala Thr Ser Pro Ile
1               5                   10                  15

Met Glu Glu Leu Ile Thr Phe His Asp His Ala Leu Met Ile Ile Phe
            20                  25                  30

Leu Ile Cys Phe Leu Val Leu Tyr Ala Leu Phe Leu Thr Leu Thr Thr
        35                  40                  45

Lys Leu Thr Asn Thr Asn Ile Ser Asp Ala Gln Glu Met Glu Thr Val
    50                  55                  60

Trp Thr Ile Leu Pro Ala Ile Ile Leu Val Leu Ile Ala Leu Pro Ser
65                  70                  75                  80

Leu Arg Ile Leu Tyr Met Thr Asp Glu Val Asn Asp Pro Ser Leu Thr
                85                  90                  95

Ile Lys Ser Ile Gly His Gln Trp Tyr Trp Thr Tyr Glu Tyr Thr Asp
            100                 105                 110

Tyr Gly Gly Leu Ile Phe Asn Ser Tyr Met Leu Pro Pro Leu Phe Leu
        115                 120                 125

Glu Pro Gly Asp Leu Arg Leu Leu Asp Val Asp Asn Arg Val Val Leu
    130                 135                 140

Pro Ile Glu Ala Pro Ile Arg Met Met Ile Thr Ser Gln Asp Val Leu
145                 150                 155                 160

His Ser Trp Ala Val Pro Thr Leu Gly Leu Lys Thr Asp Ala Ile Pro
                165                 170                 175

Gly Arg Leu Asn Gln Thr Thr Phe Thr Ala Thr Arg Pro Gly Val Tyr
            180                 185                 190

Tyr Gly Gln Cys Ser Glu Ile Cys Gly Ala Asn His Ser Phe Met Pro
        195                 200                 205

Ile Val Leu Glu Leu Ile Pro Leu Lys Ile Phe Glu Met Gly Pro Val
    210                 215                 220

Phe Thr Leu
225

<210>  6
<211>  68
<212>  PRT
<213>  homo sapiens

<400>  6

Met Pro Gln Leu Asn Thr Thr Val Trp Pro Thr Met Ile Thr Pro Met
1               5                   10                  15

Leu Leu Thr Leu Phe Leu Ile Thr Gln Leu Lys Met Leu Asn Thr Asn
            20                  25                  30

Tyr His Leu Pro Pro Ser Pro Lys Pro Met Lys Met Lys Asn Tyr Asn
        35                  40                  45

Lys Pro Trp Glu Pro Lys Trp Thr Lys Ile Cys Ser Leu His Ser Leu
    50                  55                  60

Pro Pro Gln Ser
65

<210>  7
<211>  226
<212>  PRT
<213>  homo sapiens

<400>  7

Met Asn Glu Asn Leu Phe Ala Ser Phe Ile Ala Pro Thr Ile Leu Gly
1               5                   10                  15

Leu Pro Ala Ala Val Leu Ile Ile Leu Phe Pro Pro Leu Leu Ile Pro
            20                  25                  30

Thr Ser Lys Tyr Leu Ile Asn Asn Arg Leu Ile Thr Thr Gln Gln Trp
        35                  40                  45

Leu Ile Lys Leu Thr Ser Lys Gln Met Met Thr Met His Asn Thr Lys
    50                  55                  60

Gly Arg Thr Trp Ser Leu Met Leu Val Ser Leu Ile Ile Phe Ile Ala
65                  70                  75                  80

Thr Thr Asn Leu Leu Gly Leu Leu Pro His Ser Phe Thr Pro Thr Thr
                85                  90                  95

Gln Leu Ser Met Asn Leu Ala Met Ala Ile Pro Leu Trp Ala Gly Thr
            100                 105                 110

Val Ile Met Gly Phe Arg Ser Lys Ile Lys Asn Ala Leu Ala His Phe
        115                 120                 125

Leu Pro Gln Gly Thr Pro Thr Pro Leu Ile Pro Met Leu Val Ile Ile
    130                 135                 140

Glu Thr Ile Ser Leu Leu Ile Gln Pro Met Ala Leu Ala Val Arg Leu
145                 150                 155                 160

Thr Ala Asn Ile Thr Ala Gly His Leu Leu Met His Leu Ile Gly Ser
                165                 170                 175

Ala Thr Leu Ala Met Ser Thr Ile Asn Leu Pro Ser Thr Leu Ile Ile
            180                 185                 190

Phe Thr Ile Leu Ile Leu Leu Thr Ile Leu Glu Ile Ala Val Ala Leu
        195                 200                 205

Ile Gln Ala Tyr Val Phe Thr Leu Leu Val Ser Leu Tyr Leu His Asp
    210                 215                 220

Asn Thr
225

<210>  8
<211>  261
<212>  PRT
<213>  homo sapiens

<400>  8

Met Thr His Gln Ser His Ala Tyr His Met Val Lys Pro Ser Pro Trp
1               5                   10                  15

Pro Leu Thr Gly Ala Leu Ser Ala Leu Leu Met Thr Ser Gly Leu Ala
            20                  25                  30

Met Trp Phe His Phe His Ser Met Thr Leu Leu Met Leu Gly Leu Leu
        35                  40                  45

Thr Asn Thr Leu Thr Met Tyr Gln Trp Trp Arg Asp Val Thr Arg Glu
    50                  55                  60

Ser Thr Tyr Gln Gly His His Thr Pro Pro Val Gln Lys Gly Leu Arg
65                  70                  75                  80

Tyr Gly Met Ile Leu Phe Ile Thr Ser Glu Val Phe Phe Phe Ala Gly
                85                  90                  95

Phe Phe Trp Ala Phe Tyr His Ser Ser Leu Ala Pro Thr Pro Gln Leu
            100                 105                 110

Gly Gly His Trp Pro Pro Thr Gly Ile Thr Pro Leu Asn Pro Leu Glu
        115                 120                 125

Val Pro Leu Leu Asn Thr Ser Val Leu Leu Ala Ser Gly Val Ser Ile
    130                 135                 140

Thr Trp Ala His His Ser Leu Met Glu Asn Asn Arg Asn Gln Met Ile
145                 150                 155                 160

Gln Ala Leu Leu Ile Thr Ile Leu Leu Gly Leu Tyr Phe Thr Leu Leu
                165                 170                 175

Gln Ala Ser Glu Tyr Phe Glu Ser Pro Phe Thr Ile Ser Asp Gly Ile
            180                 185                 190

Tyr Gly Ser Thr Phe Phe Val Ala Thr Gly Phe His Gly Leu His Val
        195                 200                 205

Ile Ile Gly Ser Thr Phe Leu Thr Ile Cys Phe Ile Arg Gln Leu Met
    210                 215                 220

Phe His Phe Thr Ser Lys His His Phe Gly Phe Glu Ala Ala Ala Trp
225                 230                 235                 240

Tyr Trp His Phe Val Asp Val Val Trp Leu Phe Leu Tyr Val Ser Ile
                245                 250                 255

Tyr Trp Trp Gly Ser
            260

<210>  9
<211>  115
<212>  PRT
<213>  homo sapiens

<400>  9

Met Asn Phe Ala Leu Ile Leu Met Ile Asn Thr Leu Leu Ala Leu Leu
1               5                   10                  15

Leu Met Ile Ile Thr Phe Trp Leu Pro Gln Leu Asn Gly Tyr Met Glu
            20                  25                  30

Lys Ser Thr Pro Tyr Glu Cys Gly Phe Asp Pro Met Ser Pro Ala Arg
        35                  40                  45

Val Pro Phe Ser Met Lys Phe Phe Leu Val Ala Ile Thr Phe Leu Leu
    50                  55                  60

Phe Asp Leu Glu Ile Ala Leu Leu Leu Pro Leu Pro Trp Ala Leu Gln
65                  70                  75                  80

Thr Thr Asn Leu Pro Leu Met Val Met Ser Ser Leu Leu Leu Ile Ile
                85                  90                  95

Ile Leu Ala Leu Ser Leu Ala Tyr Glu Trp Leu Gln Lys Gly Leu Asp
            100                 105                 110

Trp Thr Glu
        115

<210>  10
<211>  98
<212>  PRT
<213>  homo sapiens

<400>  10

Met Pro Leu Ile Tyr Met Asn Ile Met Leu Ala Phe Thr Ile Ser Leu
1               5                   10                  15

Leu Gly Met Leu Val Tyr Arg Ser His Leu Met Ser Ser Leu Leu Cys
            20                  25                  30

Leu Glu Gly Met Met Leu Ser Leu Phe Ile Met Ala Thr Leu Met Thr
        35                  40                  45

Leu Asn Thr His Ser Leu Leu Ala Asn Ile Val Pro Ile Ala Met Leu
    50                  55                  60

Val Phe Ala Ala Cys Glu Ala Ala Val Gly Leu Ala Leu Leu Val Ser
65                  70                  75                  80

Ile Ser Asn Thr Tyr Gly Leu Asp Tyr Val His Asn Leu Asn Leu Leu
                85                  90                  95

Gln Cys

<210>  11
<211>  459
<212>  PRT
<213>  homo sapiens

<400>  11

Met Leu Lys Leu Ile Val Pro Thr Ile Met Leu Leu Pro Leu Thr Trp
1               5                   10                  15

Leu Ser Lys Lys His Met Ile Trp Ile Asn Thr Thr Thr His Ser Leu
            20                  25                  30

Ile Ile Ser Ile Ile Pro Leu Leu Phe Phe Asn Gln Ile Asn Asn Asn
        35                  40                  45

Leu Phe Ser Cys Ser Pro Thr Phe Ser Ser Asp Pro Leu Thr Thr Pro
    50                  55                  60

Leu Leu Met Leu Thr Thr Trp Leu Leu Pro Leu Thr Ile Met Ala Ser
65                  70                  75                  80

Gln Arg His Leu Ser Ser Glu Pro Leu Ser Arg Lys Lys Leu Tyr Leu
                85                  90                  95

Ser Met Leu Ile Ser Leu Gln Ile Ser Leu Ile Met Thr Phe Thr Ala
            100                 105                 110

Thr Glu Leu Ile Met Phe Tyr Ile Phe Phe Glu Thr Thr Leu Ile Pro
        115                 120                 125

Thr Leu Ala Ile Ile Thr Arg Trp Gly Asn Gln Pro Glu Arg Leu Asn
    130                 135                 140

Ala Gly Thr Tyr Phe Leu Phe Tyr Thr Leu Val Gly Ser Leu Pro Leu
145                 150                 155                 160

Leu Ile Ala Leu Ile Tyr Thr His Asn Thr Leu Gly Ser Leu Asn Ile
                165                 170                 175

Leu Leu Leu Thr Leu Thr Ala Gln Glu Leu Ser Asn Ser Trp Ala Asn
            180                 185                 190

Asn Leu Met Trp Leu Ala Tyr Thr Met Ala Phe Met Val Lys Met Pro
        195                 200                 205

Leu Tyr Gly Leu His Leu Trp Leu Pro Lys Ala His Val Glu Ala Pro
    210                 215                 220

Ile Ala Gly Ser Met Val Leu Ala Ala Val Leu Leu Lys Leu Gly Gly
225                 230                 235                 240

Tyr Gly Met Met Arg Leu Thr Leu Ile Leu Asn Pro Leu Thr Lys His
                245                 250                 255

Met Ala Tyr Pro Phe Leu Val Leu Ser Leu Trp Gly Met Ile Met Thr
            260                 265                 270

Ser Ser Ile Cys Leu Arg Gln Thr Asp Leu Lys Ser Leu Ile Ala Tyr
        275                 280                 285

Ser Ser Ile Ser His Met Ala Leu Val Val Thr Ala Ile Leu Ile Gln
    290                 295                 300

Thr Pro Trp Ser Phe Thr Gly Ala Val Ile Leu Met Ile Ala His Gly
305                 310                 315                 320

Leu Thr Ser Ser Leu Leu Phe Cys Leu Ala Asn Ser Asn Tyr Glu Arg
                325                 330                 335

Thr His Ser Arg Ile Met Ile Leu Ser Gln Gly Leu Gln Thr Leu Leu
            340                 345                 350

Pro Leu Met Ala Phe Trp Trp Leu Leu Ala Ser Leu Ala Asn Leu Ala
        355                 360                 365

Leu Pro Pro Thr Ile Asn Leu Leu Gly Glu Leu Ser Val Leu Val Thr
    370                 375                 380

Thr Phe Ser Trp Ser Asn Ile Thr Leu Leu Leu Thr Gly Leu Asn Met
385                 390                 395                 400

Leu Val Thr Ala Leu Tyr Ser Leu Tyr Met Phe Thr Thr Thr Gln Trp
                405                 410                 415

Gly Ser Leu Thr His His Ile Asn Asn Met Lys Pro Ser Phe Thr Arg
        420                 425                 430

Glu Asn Thr Leu Met Phe Met His Leu Ser Pro Ile Leu Leu Leu Ser
        435                 440                 445

Leu Asn Pro Asp Ile Ile Thr Gly Phe Ser Ser
    450                 455

<210>  12
<211>  603
<212>  PRT
<213>  homo sapiens

<400>  12

Met Thr Met His Thr Thr Met Thr Thr Leu Thr Leu Thr Ser Leu Ile
1               5                   10                  15

Pro Pro Ile Leu Thr Thr Leu Val Asn Pro Asn Lys Lys Asn Ser Tyr
            20                  25                  30

Pro His Tyr Val Lys Ser Ile Val Ala Ser Thr Phe Ile Ile Ser Leu
        35                  40                  45

Phe Pro Thr Thr Met Phe Met Cys Leu Asp Gln Glu Val Ile Ile Ser
    50                  55                  60

Asn Trp His Trp Ala Thr Thr Gln Thr Thr Gln Leu Ser Leu Ser Phe
65                  70                  75                  80

Lys Leu Asp Tyr Phe Ser Met Met Phe Ile Pro Val Ala Leu Phe Val
                85                  90                  95

Thr Trp Ser Ile Met Glu Phe Ser Leu Trp Tyr Met Asn Ser Asp Pro
            100                 105                 110

Asn Ile Asn Gln Phe Phe Lys Tyr Leu Leu Ile Phe Leu Ile Thr Met
        115                 120                 125

Leu Ile Leu Val Thr Ala Asn Asn Leu Phe Gln Leu Phe Ile Gly Trp
    130                 135                 140

Glu Gly Val Gly Ile Met Ser Phe Leu Leu Ile Ser Trp Trp Tyr Ala
145                 150                 155                 160

Arg Ala Asp Ala Asn Thr Ala Ala Ile Gln Ala Ile Leu Tyr Asn Arg
                165                 170                 175

Ile Gly Asp Ile Gly Phe Ile Leu Ala Leu Ala Trp Phe Ile Leu His
            180                 185                 190

Ser Asn Ser Trp Asp Pro Gln Gln Met Ala Leu Leu Asn Ala Asn Pro
        195                 200                 205

Ser Leu Thr Pro Leu Leu Gly Leu Leu Leu Ala Ala Ala Gly Lys Ser
    210                 215                 220

Ala Gln Leu Gly Leu His Pro Trp Leu Pro Ser Ala Met Glu Gly Pro
225                 230                 235                 240

Thr Pro Val Ser Ala Leu Leu His Ser Ser Thr Met Val Val Ala Gly
                245                 250                 255

Ile Phe Leu Leu Ile Arg Phe His Pro Leu Ala Glu Asn Ser Pro Leu
            260                 265                 270

Ile Gln Thr Leu Thr Leu Cys Leu Gly Ala Ile Thr Thr Leu Phe Ala
        275                 280                 285

Ala Val Cys Ala Leu Thr Gln Asn Asp Ile Lys Lys Ile Val Ala Phe
    290                 295                 300

Ser Thr Ser Ser Gln Leu Gly Leu Met Met Val Thr Ile Gly Ile Asn
305                 310                 315                 320

Gln Pro His Leu Ala Phe Leu His Ile Cys Thr His Ala Phe Phe Lys
                325                 330                 335

Ala Met Leu Phe Met Cys Ser Gly Ser Ile Ile His Asn Leu Asn Asn
            340                 345                 350

Glu Gln Asp Ile Arg Lys Met Gly Gly Leu Leu Lys Thr Met Pro Leu
        355                 360                 365

Thr Ser Thr Ser Leu Thr Ile Gly Ser Leu Ala Leu Ala Gly Met Pro
    370                 375                 380

Phe Leu Thr Gly Phe Tyr Ser Lys Asp His Ile Ile Glu Thr Ala Asn
385                 390                 395                 400

Met Ser Tyr Thr Asn Ala Trp Ala Leu Ser Ile Thr Leu Ile Ala Thr
                405                 410                 415

Ser Leu Thr Ser Ala Tyr Ser Thr Arg Met Ile Leu Leu Thr Leu Thr
            420                 425                 430

Gly Gln Pro Arg Phe Pro Thr Leu Thr Asn Ile Asn Glu Asn Asn Pro
        435                 440                 445

Thr Leu Leu Asn Pro Ile Lys Arg Leu Ala Ala Gly Ser Leu Phe Ala
    450                 455                 460

Gly Phe Leu Ile Thr Asn Asn Ile Ser Pro Ala Ser Pro Phe Gln Thr
465                 470                 475                 480

Thr Ile Pro Leu Tyr Leu Lys Leu Thr Ala Leu Ala Val Thr Phe Leu
                485                 490                 495

Gly Leu Leu Thr Ala Leu Asp Leu Asn Tyr Leu Thr Asn Lys Leu Lys
            500                 505                 510

Met Lys Ser Pro Leu Cys Thr Phe Tyr Phe Ser Asn Met Leu Gly Phe
        515                 520                 525

Tyr Pro Ser Ile Thr His Arg Thr Ile Pro Tyr Leu Gly Leu Leu Thr
    530                 535                 540

Ser Gln Asn Leu Pro Leu Leu Leu Leu Asp Leu Thr Trp Leu Glu Lys
545                 550                 555                 560

Leu Leu Pro Lys Thr Ile Ser Gln His Gln Ile Ser Thr Ser Ile Ile
                565                 570                 575

Thr Ser Thr Gln Lys Gly Met Ile Lys Leu Tyr Phe Leu Ser Phe Phe
            580                 585                 590

Phe Pro Leu Ile Leu Thr Leu Leu Leu Ile Thr
        595                 600

<210>  13
<211>  174
<212>  PRT
<213>  homo sapiens

<400>  13

Met Met Tyr Ala Leu Phe Leu Leu Ser Val Gly Leu Val Met Gly Phe
1               5                   10                  15

Val Gly Phe Ser Ser Lys Pro Ser Pro Ile Tyr Gly Gly Leu Val Leu
            20                  25                  30

Ile Val Ser Gly Val Val Gly Cys Val Ile Ile Leu Asn Phe Gly Gly
        35                  40                  45

Gly Tyr Met Gly Leu Met Val Phe Leu Ile Tyr Leu Gly Gly Met Met
    50                  55                  60

Val Val Phe Gly Tyr Thr Thr Ala Met Ala Ile Glu Glu Tyr Pro Glu
65                  70                  75                  80

Ala Trp Gly Ser Gly Val Glu Val Leu Val Ser Val Leu Val Gly Leu
                85                  90                  95

Ala Met Glu Val Gly Leu Val Leu Trp Val Lys Glu Tyr Asp Gly Val
            100                 105                 110

Val Val Val Val Asn Phe Asn Ser Val Gly Ser Trp Met Ile Tyr Glu
        115                 120                 125

Gly Glu Gly Ser Gly Leu Ile Arg Glu Asp Pro Ile Gly Ala Gly Ala
    130                 135                 140

Leu Tyr Asp Tyr Gly Arg Trp Leu Val Val Val Thr Gly Trp Thr Leu
145                 150                 155                 160

Phe Val Gly Val Tyr Ile Val Ile Glu Ile Ala Arg Gly Asn
                165                 170

<210>  14
<211>  380
<212>  PRT
<213>  Homo sapiens

<400>  14

Met Thr Pro Met Arg Lys Thr Asn Pro Leu Met Lys Leu Ile Asn His
1               5                   10                  15

Ser Phe Ile Asp Leu Pro Thr Pro Ser Asn Ile Ser Ala Trp Trp Asn
            20                  25                  30

Phe Gly Ser Leu Leu Gly Ala Cys Leu Ile Leu Gln Ile Thr Thr Gly
        35                  40                  45

Leu Phe Leu Ala Met His Tyr Ser Pro Asp Ala Ser Thr Ala Phe Ser
    50                  55                  60

Ser Ile Ala His Ile Thr Arg Asp Val Asn Tyr Gly Trp Ile Ile Arg
65                  70                  75                  80

Tyr Leu His Ala Asn Gly Ala Ser Met Phe Phe Ile Cys Leu Phe Leu
                85                  90                  95

His Ile Gly Arg Gly Leu Tyr Tyr Gly Ser Phe Leu Tyr Ser Glu Thr
            100                 105                 110

Trp Asn Ile Gly Ile Ile Leu Leu Leu Ala Thr Met Ala Thr Ala Phe
        115                 120                 125

Met Gly Tyr Val Leu Pro Trp Gly Gln Met Ser Phe Trp Gly Ala Thr
    130                 135                 140

Val Ile Thr Asn Leu Leu Ser Ala Ile Pro Tyr Ile Gly Thr Asp Leu
145                 150                 155                 160

Val Gln Trp Ile Trp Gly Gly Tyr Ser Val Asp Ser Pro Thr Leu Thr
                165                 170                 175

Arg Phe Phe Thr Phe His Phe Ile Leu Pro Phe Ile Ile Ala Ala Leu
            180                 185                 190

Ala Thr Leu His Leu Leu Phe Leu His Glu Thr Gly Ser Asn Asn Pro
        195                 200                 205

Leu Gly Ile Thr Ser His Ser Asp Lys Ile Thr Phe His Pro Tyr Tyr
    210                 215                 220

Thr Ile Lys Asp Ala Leu Gly Leu Leu Leu Phe Leu Leu Ser Leu Met
225                 230                 235                 240

Thr Leu Thr Leu Phe Ser Pro Asp Leu Leu Gly Asp Pro Asp Asn Tyr
                245                 250                 255

Thr Leu Ala Asn Pro Leu Asn Thr Pro Pro His Ile Lys Pro Glu Trp
            260                 265                 270

Tyr Phe Leu Phe Ala Tyr Thr Ile Leu Arg Ser Val Pro Asn Lys Leu
        275                 280                 285

Gly Gly Val Leu Ala Leu Leu Leu Ser Ile Leu Ile Leu Ala Met Ile
    290                 295                 300

Pro Ile Leu His Met Ser Lys Gln Gln Ser Met Met Phe Arg Pro Leu
305                 310                 315                 320

Ser Gln Ser Leu Tyr Trp Leu Leu Ala Ala Asp Leu Leu Ile Leu Thr
                325                 330                 335

Trp Ile Gly Gly Gln Pro Val Ser Tyr Pro Phe Thr Ile Ile Gly Gln

340                 345                 350

Val Ala Ser Val Leu Tyr Phe Thr Thr Ile Leu Ile Leu Met Pro Thr
        355                 360                 365

Ile Ser Leu Ile Glu Asn Lys Met Leu Lys Trp Ala
    370                 375                 380

## Description

### Field of the invention

The invention is in the art of medical treatments, in particular the treatment of tumors with ionizing radiation. It provides means and methods for predicting whether a subject is likely to develop radiation damage upon radiotherapy. The invention provides tools that allow individualized and optimized radiation treatment of a subject in need of a radiation treatment. The invention also provides methods for determining the risk of developing severe dyspnea after radiation treatment.

### Background of the invention

Radiation-induced lung toxicity (RILT) is the most radiation-dose limiting factor for lung cancer patients. It occurs in about 15-20% of such patients and can seriously affect the quality of life and can even be lethal (Mehta, V. Radiation pneumonitis and pulmonary fibrosis in non-small-cell lung cancer: pulmonary function, prediction, and prevention. Int J Radiat Oncol Biol Phys 63, 5-24 (2005)).

However, RILT varies significantly between patients at similar dose levels to the lung. The current radiation practice aims at limiting the toxicity rate to a value between 5% and 15% based on risk estimates for the general population. Thus, most lung cancer patients receive sub-maximal doses (Kong, F.M., et al. Final toxicity results of a radiation-dose escalation study in patients with non-small-cell lung cancer (NSCLC): predictors for radiation pneumonitis and fibrosis. Int J Radiat Oncol Biol Phys 65, 1075-1086 (2006)).

The relevance of genetic predisposition for individual differences in radiotherapy (RT) toxicity has been recognized for long (Barnett, G.C., et al. Normal tissue reactions to radiotherapy: towards tailoring treatment dose by genotype. Nat Rev Cancer 9, 134-142 (2009)). Studies using candidate gene approaches have searched for associations between single nucleotide polymorphisms (SNPs) in multiple genes and radiotoxicity, resulting in conflicting findings (Quarmby, S., et al. Association of transforming growth factor beta-1 single nucleotide polymorphisms with radiation-induced damage to normal tissues in breast cancer patients. Int J Radiat Biol 79, 137-143 (2003), Andreassen, C.N., Alsner, J., Overgaard, M. & Overgaard, J. Prediction of normal tissue radiosensitivity from polymorphisms in candidate genes. Radiother Oncol 69, 127-135 (2003), Azria, D., et al. Single nucleotide polymorphisms, apoptosis, and the development of severe late adverse effects after radiotherapy. Clin Cancer Res 14, 6284-6288 (2008), Barnett, G.C., et al. Independent validation of genes and polymorphisms reported to be associated with radiation toxicity: a prospective analysis study. Lancet Oncol 13, 65-77 (2012), Voets, A.M., et al. No association between TGF-beta1 polymorphisms and radiation-induced lung toxicity in a European cohort of lung cancer patients. Radiotherapy and oncology : Journal of the European Society for Therapeutic Radiology and Oncology 105, 296-298 (2012)).

There is still a need in the art for more reliable markers that predict the risk of developing radiation damage in particular in the treatment of radiation-curable diseases such as thoracic cancers, such as for instance lung cancer. Such markers would be useful in guiding the physician towards a prediction of the radiation dose that may be applied to a certain subject without inducing damage to the lungs.

### Summary of the invention

Two sets of 321 and 66 lung cancer patients receiving radiation treatment at two university hospitals were studied herein. We found that an increased number of non-synonymous variations within mitochondrial genes encoding proteins, correlated with an increased risk for RILT.

We conclude that mitochondrial DNA variation can successfully predict whether lung cancer patients will develop radiation induced lung toxicity (RILT) after ionizing radiotherapy. In particular the non-synonymous variations (nucleotide variations resulting in a change of an amino acid in a protein encoded by a mitochondrial gene) appeared to have a predicting power for the development of RILT after radiation therapy.

Hence, the invention relates to a method for predicting the risk of developing radiation induced lung toxicity comprising the steps of: obtaining mitochondrial DNA from a sample of a subject and determining the number of non-synonymous variations present in at least one gene encoding a mitochondrial protein, wherein a higher number of non-synonymous variations corresponds to a higher risk of developing radiation induced lung toxicity.

When the data obtained in our populations were expressed in a ROC curve, this resulted in an AUC of 0.770 for one set and 0.725 for the other set. This shows that the method as disclosed herein provides a valuable and reliable predictive tool for a risk factor of developing RILT. The method according to the invention has a significantly better predictive power than the current gold standard (mean lung dose, AUC 0.457 in our dataset). Incorporation of mtDNA information in personalized RT planning might eventually allow for escalating doses in patients predicted to be at low risk for RILT, improving their chance of disease free survival.

### Detailed description of the invention

Lung cancer patients undergoing ionizing radiation therapy have an increased chance of developing lung toxicity, so-called radiation induced lung toxicity (RILT). The term RILT is herein defined as the development of dyspnea after irradiation. In the examples as provided herein the term RILT is used to indicate dyspnea grade ≥2 (CTCAEv3.0) within 6 months after radiation treatment.

Although higher radiation doses to the lung tumor are associated with improved local control and overall survival , the current radiation practice is based on reducing the risk of developing secondary complication in the lungs (such as dyspnea) during or after radiation therapy to approximately 5-15%. However, in practice only a small number of patients are hypersensitive to radiation. Thus, identifying patients that are hypersensitive to radiation would allow for an improved treatment of the non-hypersensitive subjects wherein an increased dose may be given to patients that are not at risk of developing RILT thereby increasing their chances on a complete cure.

We found that mitochondrial DNA variation can successfully predict whether lung cancer patients will develop radiation induced lung toxicity (RILT) after ionizing radiotherapy. We use the definition of RILT according to the CTCAEv3.0 scoring system. In essence, RILT is defined as an increase in dyspnea (shortness of breath) score. Approximately 15% of all lung cancer patients develop RILT after radiation therapy.

Even more importantly, the risk of RILT may be used to determine the total dose to be applied in the therapy of a given patient. Since the success of the therapy depends on the dose provided, the treating physician is always looking for the highest possible dose to be administered.

The more accurate a prediction of RILT is, the higher the dose can be, thereby improving the success rate of the treatment.

We present herein a predictive model based on mitochondrial DNA (mtDNA) data that achieves an accuracy of 77%, exceeding the accuracy of existing models which achieve an accuracy of about 50-60%, which is hardly better than random.

Mitochondrial DNA is the DNA located in organelles called mitochondria, structures within eukaryotic cells that convert the chemical energy from food into a form that cells can use, adenosine triphosphate (ATP). Most of the rest of the DNA present in eukaryotic cells can be found in the cell nucleus.

In humans, mitochondrial DNA can be regarded as the smallest chromosome coding for only 37 genes and containing only about 16,600 base pairs. Human mitochondrial DNA was the first significant part of the human genome to be sequenced. In most species, including humans, mtDNA is inherited solely from the mother.

The DNA sequence of mtDNA has been determined from a large number of organisms and individuals (including some organisms that are extinct), and the comparison of those DNA sequences represents a mainstay of phylogenetics, in that it allows biologists to elucidate the evolutionary relationships among species. It also permits an examination of the relatedness of populations, and so has become important in anthropology and field biology.

For human mitochondrial DNA 100-10,000 separate copies of mtDNA are usually present per cell. In mammals, each double-stranded circular mtDNA molecule consists of 15,000-17,000 base pairs. The two strands of mtDNA are differentiated by their nucleotide content with the guanine rich strand referred to as the heavy strand or H-strand, and the cytosine rich strand referred to as the light strand or L-strand. The heavy strand encodes 28 genes, and the light strand encodes 9 genes for a total of 37 genes. Of the 37 genes, 13 are for proteins (polypeptides), 22 are for transfer RNA (tRNA) and two are for the small and large subunits of ribosomal RNA (rRNA).

The revised Cambridge Reference Sequence (rCRS) of the human mitochondrial DNA is used herein as published under NCBI number NC_012920 (Genbank's RefSeq database). It is the Cambridge Reference Sequence as provided by Anderson et al., Nature (1981) 290; 457-465 as revised by Andrews et al., Nature Genetics (1999) 2;147. The rCRS is referred herein as the reference sequence and the nucleotide sequence of its L-strand is provided herein as SEQ ID NO: 1.

Variation in the mtDNA sequence is common and every individual carries between 10 and 60 variants. We now found that certain types of mtDNA variants are associated with radiation induced lung toxicity (RILT). We also found that certain types of mtDNA variations were indicative for the risk of developing RILT.

The terms "predicting the risk" "indicative of the risk" or "risk factor" or equivalent, as used herein, refer to assessing the probability according to which the subject as referred to herein will experience RILT. More preferably, the risk/probability of developing RILT within a certain time window is predicted. In a preferred embodiment of the present invention, the predictive window, preferably, is an interval of at least 3 months, such as at least 6 months, at least 9 months, at least 1 year, at least 2 years, at least 3 years, at least 4 years, at least 5 years, at least 10 years, at least 15 years or any intermitting time range. In a particular preferred embodiment of the present invention, the predictive window, preferably, is an interval of at least 6 months. Preferably, said predictive window is calculated from the time point at which radiotherapy started.

Mitochondrial DNA, extracted from blood lymphocytes of patients before they underwent radiotherapy, was sequenced using the commercially available Affymetrix Mitochondrial DNA resequencing chip 2.0 (Mitochip). The sequence obtained was compared with the reference sequence (SEQ ID NO:1) and variants were scored and validated.

It was found that in particular the non-synonymous variants in any of the protein encoding genes in the mitochondrial genome correlated with the development of RILT upon radiation.

The mtDNA may be obtained from all kinds of samples by using standard techniques, commercially available in the art. Preferred is the use of samples containing nucleated cells, preferably blood cells, more preferably, white blood cells. Also preferred is the use of a sample obtained before the start of the irradiation.

The sequence of the mtDNA may be determined with routine methods which are commercially available in the art. The obtained mtDNA sequences may then be compared with the reference sequence (SEQ ID NO:1) and variations may be determined.

Due to the degeneracy of the genetic code, not every variation in the nucleotide sequence will automatically lead to an alteration or variant in the protein sequence. The variations in the nucleotide sequence that lead to alterations (variants) of the encoded proteins are termed herein "non-synonymous" variations. Silent variations that do not result in the alteration of the primary protein sequence are termed "synonymous" variants or variations.

The 13 mtDNA genes encoding proteins are termed ND1, ND2, CO1, CO2, ATP8, ATP6, CO3, ND3, ND4L, ND4, ND5, ND6, and CytB. Their amino acid sequence (SEQ ID NO: 2 - 14) is provided in tables 1 - 13. The position in the mtDNA genome of these 13 genes is shown in table 14.

A person skilled in the art will be able to determine whether a certain variation in the mtDNA found in a particular subject is within the coding sequence of a mitochondrial gene encoding a protein. For that purpose, he may align the sequence obtained from the subject with the reference sequence and determine whether the variant is within the range between the first and the last coding sequence of any of the genes listed in table 14.

A variant nucleotide is defined herein as a nucleotide that is different from its counterpart nucleotide at the same position in the reference sequence. So, a nucleotide G at position 3600 in the sequence of the mtDNA of a subject is a variant nucleotide since position 3600 in the reference sequence (SEQ ID NO:1) is a C.

If the variant nucleotide is within the coding sequence of any of the genes encoding a protein (protein genes) then the triplet encoding the corresponding amino acid may be determined. The skilled person will know how to determine the corresponding amino acid position. In detail: he may first determine the position of the variant (N) relative to the start codon by subtracting the first coding nucleotide position (FCNP) position from the position number of N. For example, a variation at position N=3340 is within the ND1 gene (Table 14) at relative position 3340-3308 = 32.

Next, he may determine the triplet codon number for that position by dividing the absolute value of the relative position number by 3, adding 1 to the outcome and rounding down the value towards zero. For example, relative positions 30 to 32 are within triplet number 11 of the ND1 gene. Triplet 11 encodes amino acid number 11 of the ND1 gene.

A formula for that calculation in Microsoft Excel would read: ROUNDDOWN(((ABS(N-FNP))/3+1);0). More suitable computer programs for converting nucleotide variations into amino acid variations are available in the art and are preferred.

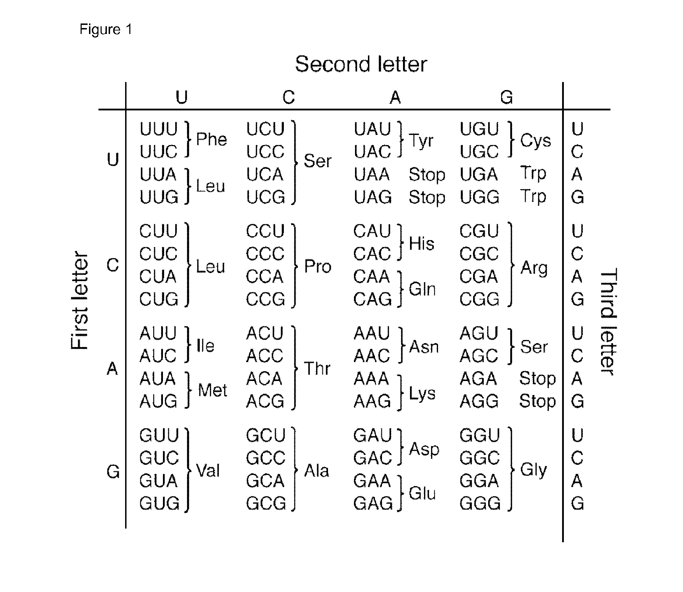

Once the triplet codon number has been determined, it may be determined whether the nucleotide variation will result in an amino acid variation by comparing the encoded amino acid with the reference sequence (SEQ ID NO: 1) and the sequence in tables 1 - 13. Care is to be taken in this process to use the genetic code for mitochondrial DNA which is slightly different from the code for nuclear DNA. The codon usage table for mitochondrial DNA is known and provided herein in figure 1. If the nucleotide variation does not alter the amino acid encoded, the variation is referred to as a synonymous variation, otherwise it is considered a non-synonymous variation.

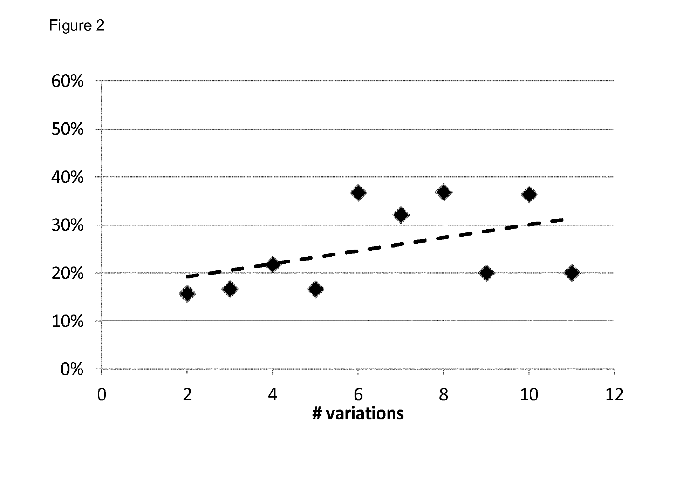

We found that there is a positive correlation between the number of non-synonymous variations and the risk of developing RILT after radiation. The raw data obtained with the largest population of 321 patients are shown in Table 16 below. The results obtained with the smaller population of 66 patients were comparable if not identical. It is shown in table 16 that the number of non-synonymous variations in these patients ranges from 0 to 14. When the patients were grouped according to their number of non-synonymous variations, and groups with less than 3 members were left out, it can be seen that the risk on RILT increases with increasing number of non-synonymous variations (table 17 and figure 2).

**Table 17. The risk on RILT increases with increasing number of non-synonymous variations.**

| **# non-synonymous variations** | **% patients developing RILT** |
|---|---|
| 2 | 16% |
| 3 | 17% |
| 4 | 22% |
| 5 | 17% |
| 6 | 37% |
| 7 | 32% |
| 8 | 37% |
| 9 | 20% |
| 10 | 36% |
| 11 | 20% |

In a ROC curve produced with our data as disclosed herein, the use of this "marker" resulted in an accuracy of 60% which is an improvement over prior art methods.

Hence, the invention relates to a method for predicting the risk of developing radiation induced lung toxicity comprising the steps of obtaining mitochondrial DNA from a sample of a subject and determining the number of non-synonymous variations present in at least one gene encoding a mitochondrial protein, wherein a higher number of non-synonymous variations corresponds to a higher risk of developing radiation induced lung toxicity.

When this method is combined with methods employing other or further risk factors, this may advantageously be done in a so-called nomogram. Therein, a value is attributed to a certain risk factor (such as the number of non-synonymous variations in the mitochondrial DNA) that is then converted in a risk factor, wherein a higher value indicates a higher risk.

The invention therefore also relates to a a method for predicting the risk of developing radiation induced lung toxicity comprising the steps of obtaining mitochondrial DNA from a sample of a subject, determining the number of non-synonymous variations present in at least one gene encoding a mitochondrial protein and attributing a value to the number of non-synonymous variations, wherein a higher value corresponds to a higher risk of developing radiation induced lung toxicity.

We further found that the method could be improved by scoring only those non-synonymous variations that occurred in the mtDNA genome at positions that were less conserved in evolution. For the determination of the evolutionary conservation of the individual positions in the mtDNA genome, we used a database provided online at http://mtsnp.tmig.or.jp/mtsnp/search_mtSAP_evaluation_e.html. Therein, the entire mtDNA genome of 61 mammalian species can be found. We used those data to determine the evolutionary conservation of each amino acid encoded by the reference sequence of the mtDNA (SEQ ID NO:1). The results are shown in tables 1 - 13 in the column Percentage Conservation (%cons).

Particularly good results were thus obtained when the non-synonymous variants that were well conserved in the reference sequence were excluded from the analysis. It was found that amino acid variants on positions that were evolutionary less conserved in the reference sequence were better pedictors for RILT than the variants on well conserved positions.

For example, amino acid number 11 of the ATP6 gene (Table 1) is 13.1% conserved and would therefore have a better predictive value than amino acid number 1 which is 98.4% conserved.

The invention therefore relates to a method as described above wherein said non-synonymous variations are selected from the group of variants that are less than 100% conserved.

Less than 100% in the present context means 99% or less, such as 98%, 97%, 96%, 95%, 94%, 93%, 92%, 91%, 90%, 89%, 88%, 87%, 86%, 85%, 84%, 83%, 82%, 81%, 80% or less conserved, such as 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, 50% or less, including any number between 49 and 10.

It was found that there is an optimum number of conservation to be used in the present invention. If the percentage of conservation decreases, the number of non-synonymous amino acid variations that can be used in the analysis also decreases, making the prediction increasingly more unreliable. On the other hand, the predictive value of the individual variant increases with decreasing percentage of conservation. That mechanism results in an optimal percentage of conservation that yields the optimal predictive value. In our test population that optimal value was found to be between less than 85-95%, such as less than or equal to 90% (<=90%).

Since our test population is sufficiently large, this percentage conservation is expected to be an adequate cut-off value for performing the method in any other population.

In a preferred embodiment, the invention therefore relates to a method as described above wherein said non-synonymous variations occur at positions that are at most 90% conserved (<=90% conserved).

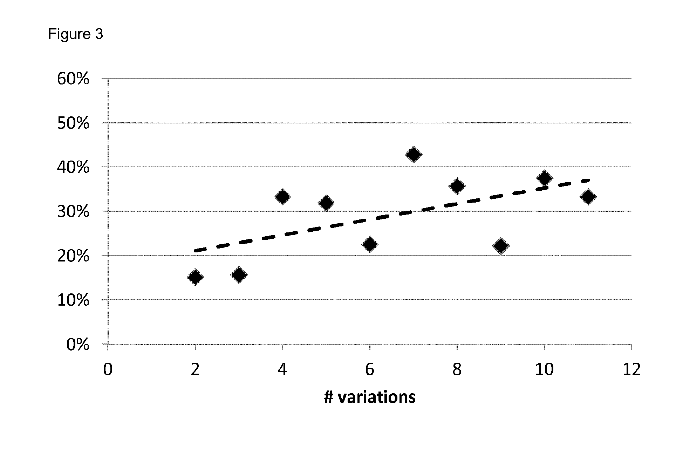

This is illustrated by the data presented in Table 18 and figure 3.

**Table 18: The risk on RILT increases with increasing number of non-synonymous variations at positions <=90% conserved.**

| **# non-synonymous variations at positions <=90% conserved** | **% patients developing RILT** |
|---|---|
| 2 | 16% |
| 3 | 17% |
| 4 | 22% |
| 5 | 17% |
| 6 | 37% |
| 7 | 32% |
| 8 | 37% |
| 9 | 20% |
| 10 | 36% |
| 11 | 20% |

The method was found to be particularly useful in a population of patients suffering from lung cancer. Hence, in a preferred embodiment, the invention relates to a method as described above wherein the subject is diagnosed with lung cancer.

The predictive value of the method as disclosed above, could even be further improved when the value attributed to the number of non-synonymous variations was added to a value obtained from clinical parameters. The most useful clinical parameter in this respect was the baseline dyspnea score.

The term baseline dyspnea score in this context means the shortness-of-breath before the start of the radiation therapy, scored qualitatively on a 0-5 scale according to the Common Toxicity Criteria version 3.0 (CTCAEv3.0). Of course, different CTC versions may also be used, as well as different approaches to score dyspnea, such as scoring according to other schemes (example: RTOG) or using quantitative measures to score dyspnea (such as measuring the movement of patients though time, using for instance a pedometer device).

The invention therefore relates to a method as described above, additionally comprising a step of attributing a value to the baseline dyspnea score of the subject and adding that value to the value obtained from the number of non-synonymous variations to obtain an aggregated value, wherein a higher aggregated value corresponds to a higher risk of developing radiation induced lung toxicity.

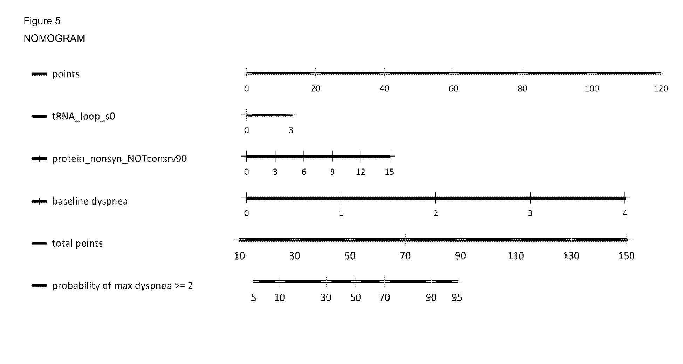

The values attributed to the number of non-synonymous variations and the value attributed to the baseline dyspnea score of the subject may advantageously be represented in a nomogram. Such a nomogram is shown in figure 5.

A nomogram is a tool to estimate outcome probability by assigning points (upperscale) to each predictor value. The sum of these scores corresponds to an event probability (bottom two scales).

We also found that a method as described above could even be further improved when variations in the genes encoding tRNAs were also taken into account, in particular those variations that occur in the loops of the tRNAs.

Table 15 shows the nucleotide positions of loops of tRNAs. There are three loops; the D-loop, the T-loop and the anti-CD loop. The D-loop in tRNA contains the modified nucleotide dihydrouridine. It is composed of 7 to 11 bases and is closed by a Watson Crick base pair. The TψC-loop (generally called the T-loop) contains thymine, a base usually found in DNA and pseudouracil (ψ). The D-loop and T-loop form a tertiary interaction in tRNA. The anticodon loop consists of 7 nucleotides that encompass the three nucleotides that correspond to the three bases of the codon of the mRNA.

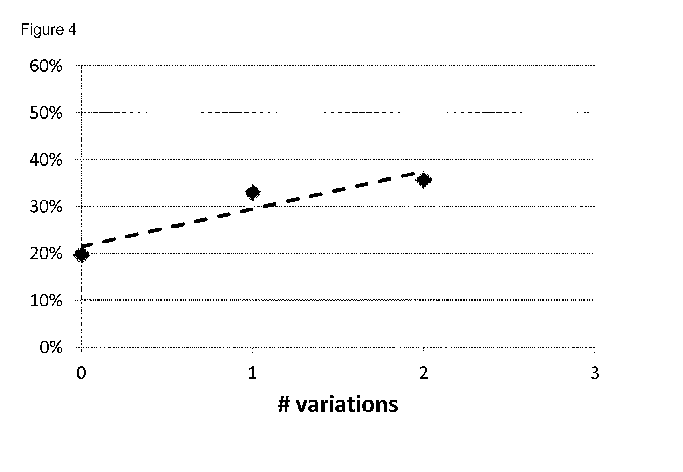

The correlation between number of variants in the loops of tRNA genes is shown in table 19 and figure 4.

**Table 19: The risk on RILT increases with increasing number of variations at positions encoding the loops of tRNAs.**

| **# variations at positions encoding tRNA loops** | **% patients developing RILT** |
|---|---|
| 0 | 20% |
| 1 | 33% |
| 2 | 36% |

In a preferred embodiment, the invention therefore relates to a method as described above wherein the number of variant nucleotides in any of the three loops is counted and a value attributed to the number of variants. The higher the number of variants, the higher the attributed value. The value thus obtained may then be added to the aggregated value obtained from the non-synonymous variants and the baseline dyspnea score.

In a particularly preferred embodiment, the invention therefore relates to a method as described above, additionally comprising a step of determining the number of variations in tRNA loop positions, attributing a value to the number of variations in tRNA loop positions and adding that value to the value obtained from the number of non-synonymous variations or the aggregated value.

In an even more preferred embodiment, the treatment status of the subject is also taken into account. Patients treated with chemotherapy had a higher risk of RILT after the radiation treatment. This risk increased with the dose of chemotherapy. Treatment may therefore also successfully be integrated in a method according to the invention. For that purpose, a value is attributed to the treatment status, proportional to the dose of chemotherapy and that value is added to the value obtained from the number of non-synonymous variations or the aggregated value.

As detailed above, the mitochondrial genome consists of 13 genes encoding 13 different proteins. The method may be performed by considering only the non-synonymous variations in one of these genes, at each position or at some selected positions, such as less conserved positions. However, the accuracy and reliability of the method improves when more than one gene is included in the analysis, such as 2 genes or more than 2 genes, such as 3 or 4, 5, 6, 7, 8, 9, 10, 11, 12 or all protein encoding genes.

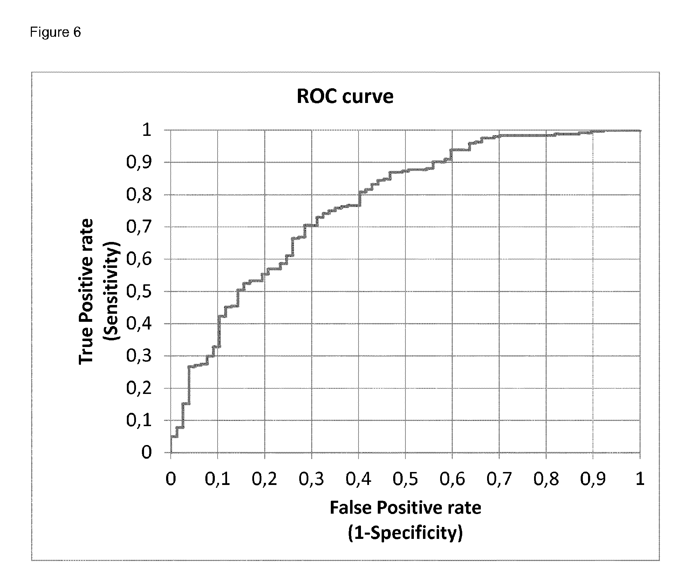

Figure 6 shows a ROC curve obtained when a preferred method according to the invention was applied to the population of the present study. At a certain point in the ROC curve, 64 patients are predicted to not have RILT after radiation treatment whereas 5 patients do.

Therefore, when using this point for predicting whether a patient could receive higher radiation doses, these patients have a relative risk of 8% for developing RILT. This is significant improvement (p = 0.012, Chi-square equality-of-proportions test) from the 24% of patients developing RILT in the whole training dataset. Furthermore, at another point in the ROC curve, 23 patients were predicted to develop RILT and could be selected for treatment with expensive radio-protectors, such as amifostine, or proton therapy. Although at that point 5 (22%) patients were wrongly predicted to be radiosensitive, this proportion is significantly less (p<0.001, Chi-square test) than the 76% of patients not developing RILT in the whole training dataset. The expenses of (over)treating such small proportion of patients are substantially smaller than when all patients would be included (increased relative benefit).

The possibility to predict if patients will be hypersensitive to radiotherapy or not will allow more optimal delivery and ultimately the usage of higher radiation doses (and thus better tumor control and outcome) in patients that are not hypersensitive. This invention will predominantly be useful in radiotherapy centers and clinics that treat lung cancer patients.

### Legend to the figures

Figure 1. Codon table for mitochondrial DNA.
Figure 2. Scatter plot with trendline. The figure shows the data presented in table 17. It can be seen that the risk on RILT increases with increasing number of non-synonymous variations.
Figure 3. Scatter plot with trendline. The figure shows the data presented in table 18. It can be seen that the risk on RILT increases with increasing number of non-synonymous variations at positions that are 90% or less conserved.
Figure 4. Scatter plot with trendline. The figure shows the data presented in table 19. It can be seen that the risk on RILT increases with increasing number variations in the genes encoding tRNAs at position encoding the tRNA loops.
Figure 5. The nomogram is a model visualizing a diagram for determining the probability of RILT. The figure shows a model for determining the probability of dyspnea >= 2. The uppermost horizontal line indicates the number of points to be attributed to a certain parameter. For example: if 0 variations are found in the tRNA loop of an individual, then 0 points are scored. When 15 non-synonymous variations are found at positions less than 90% conserved, this accounts for an additional 42 points. A baseline dyspnea score of 1 accounts for 27 points. The total points for this individual would then be 42 plus 14 plus 0 is 56 total points. This individual would then score a probability of about 60% of RILT, calculated based on the total points horizontal line and the respective value of max. dyspnea >=2 line.
Figure 6: ROC CURVE obtained from the largest population tested herein.

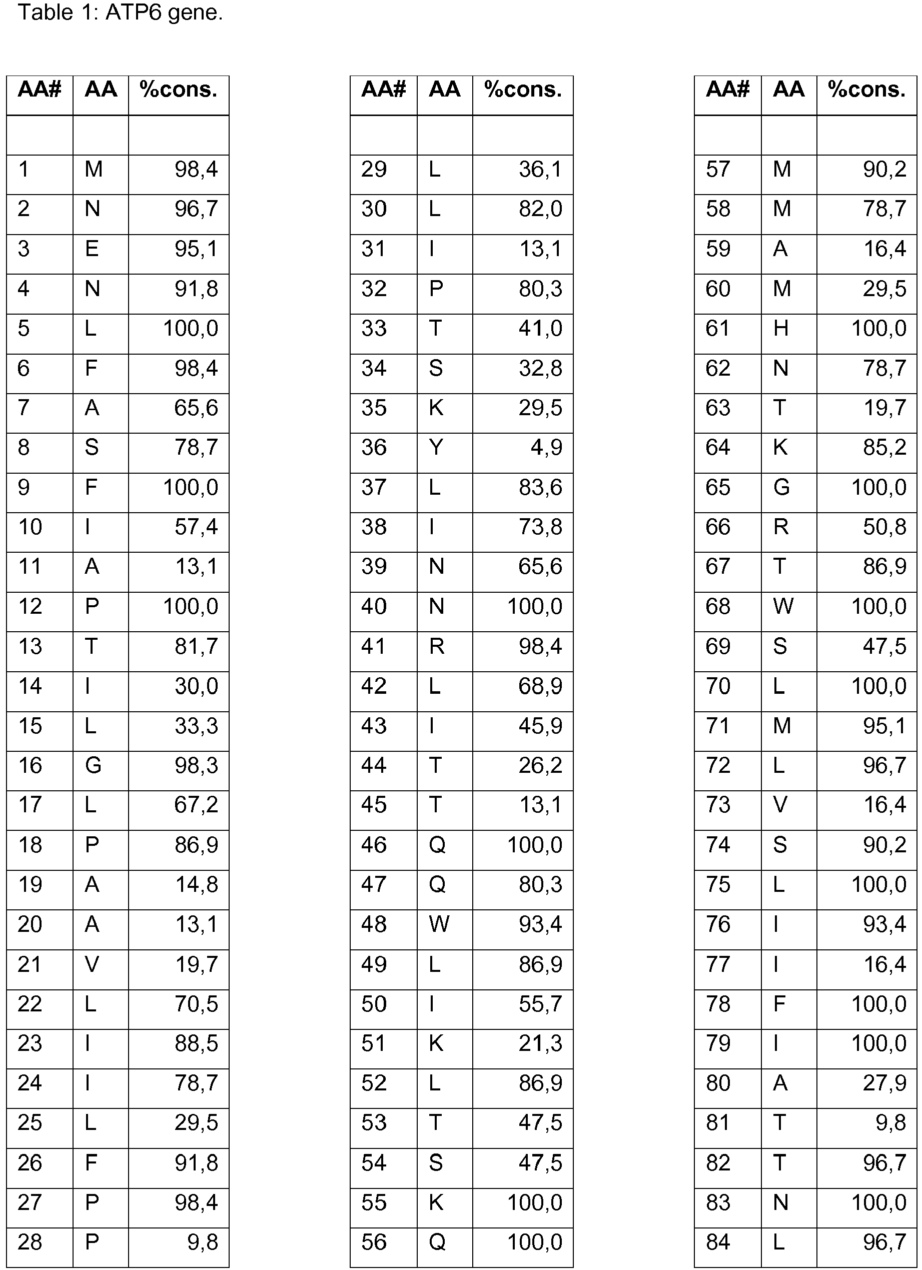

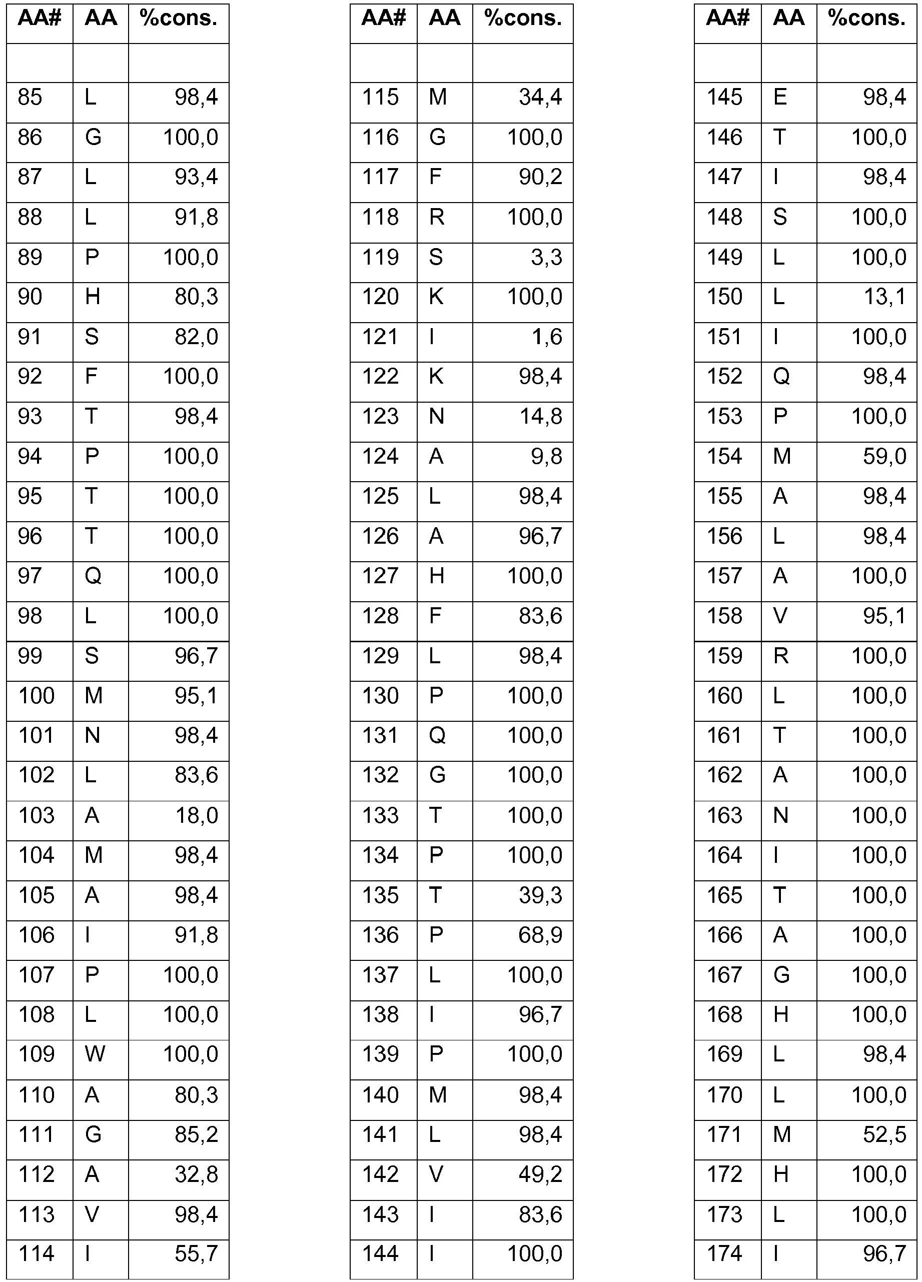

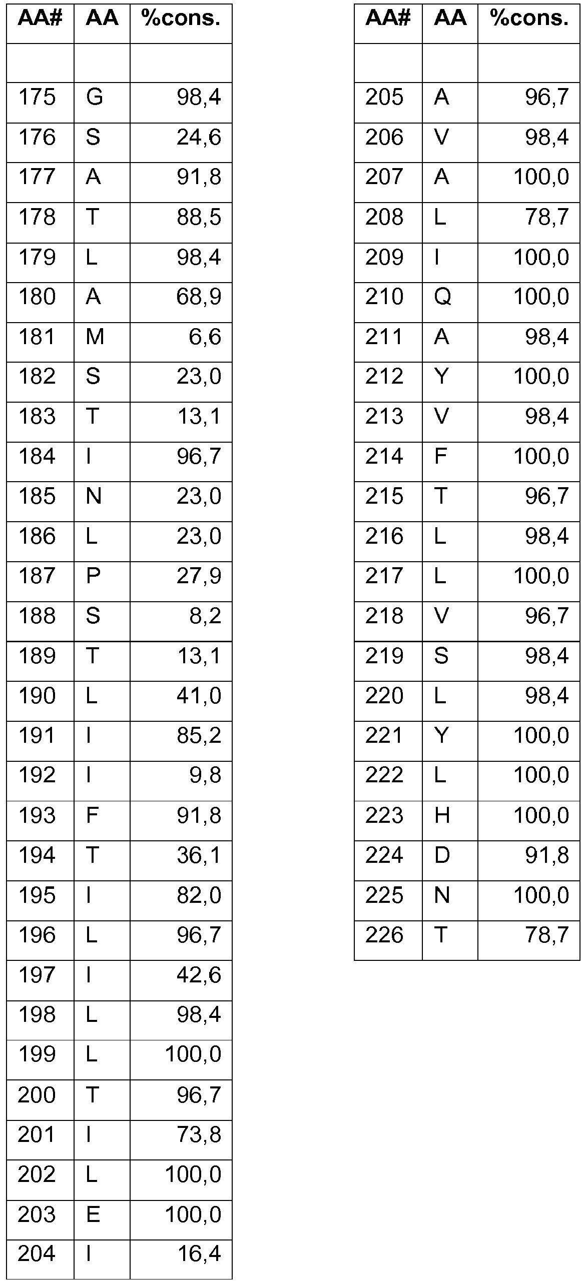

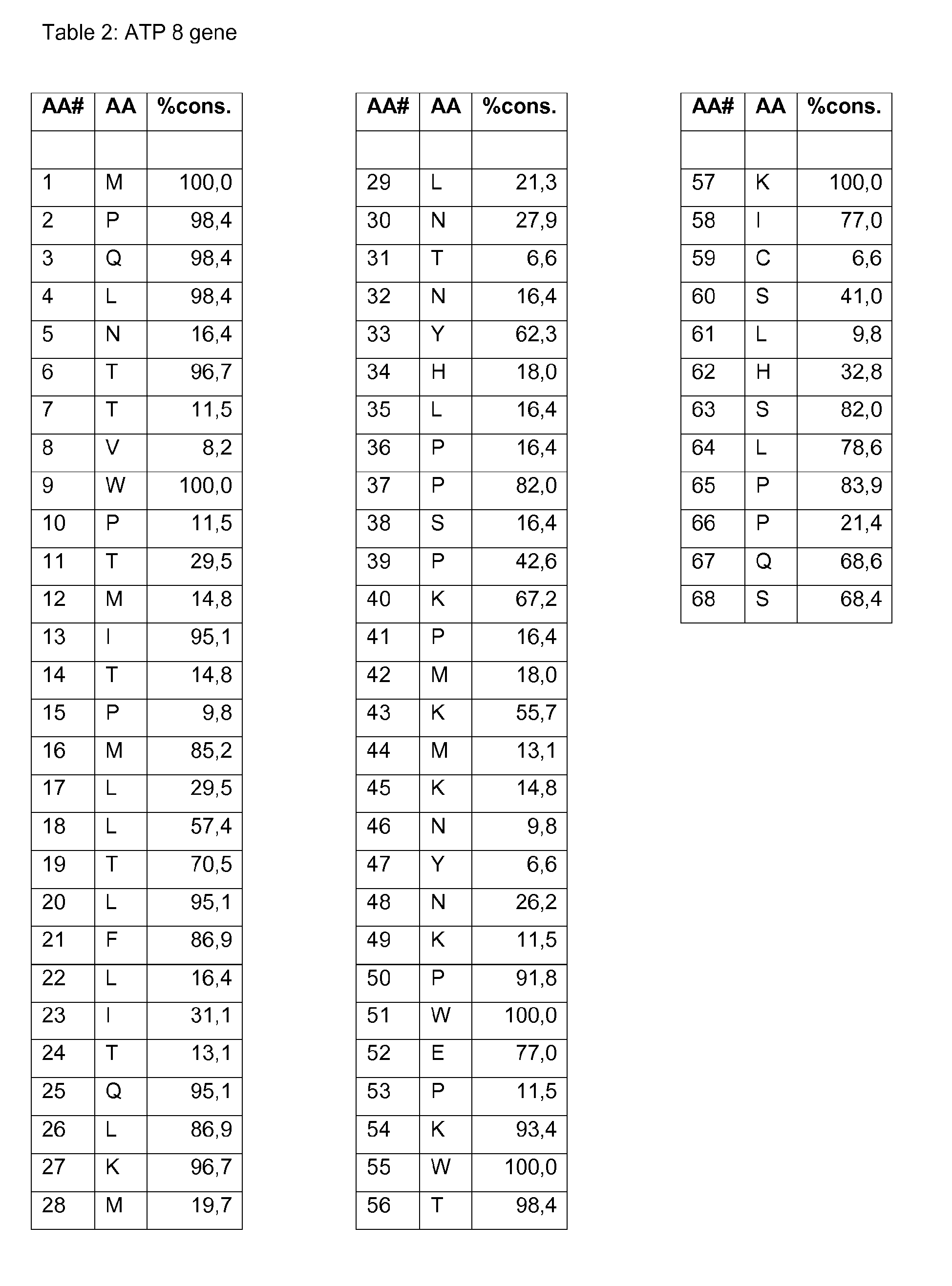

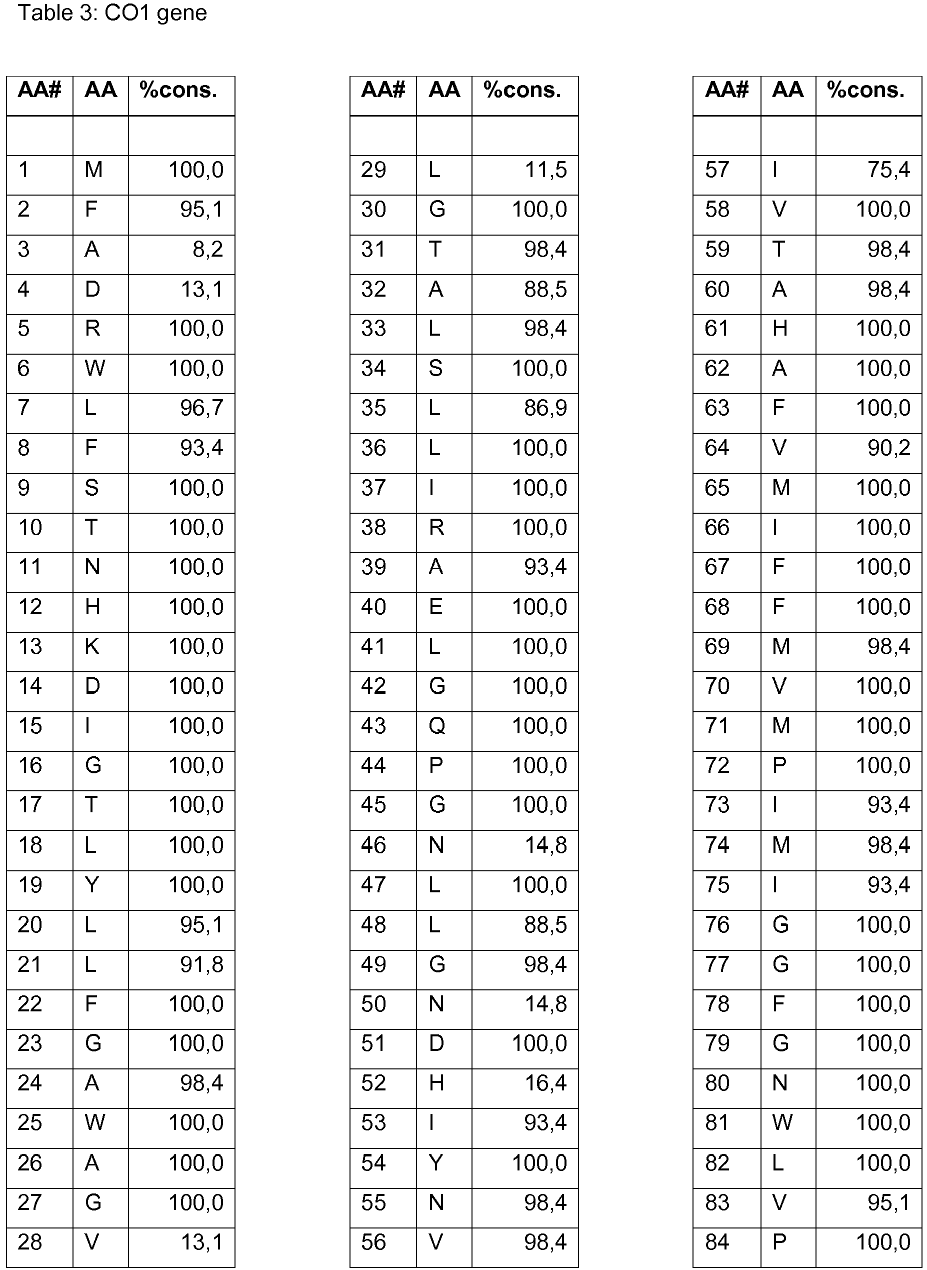

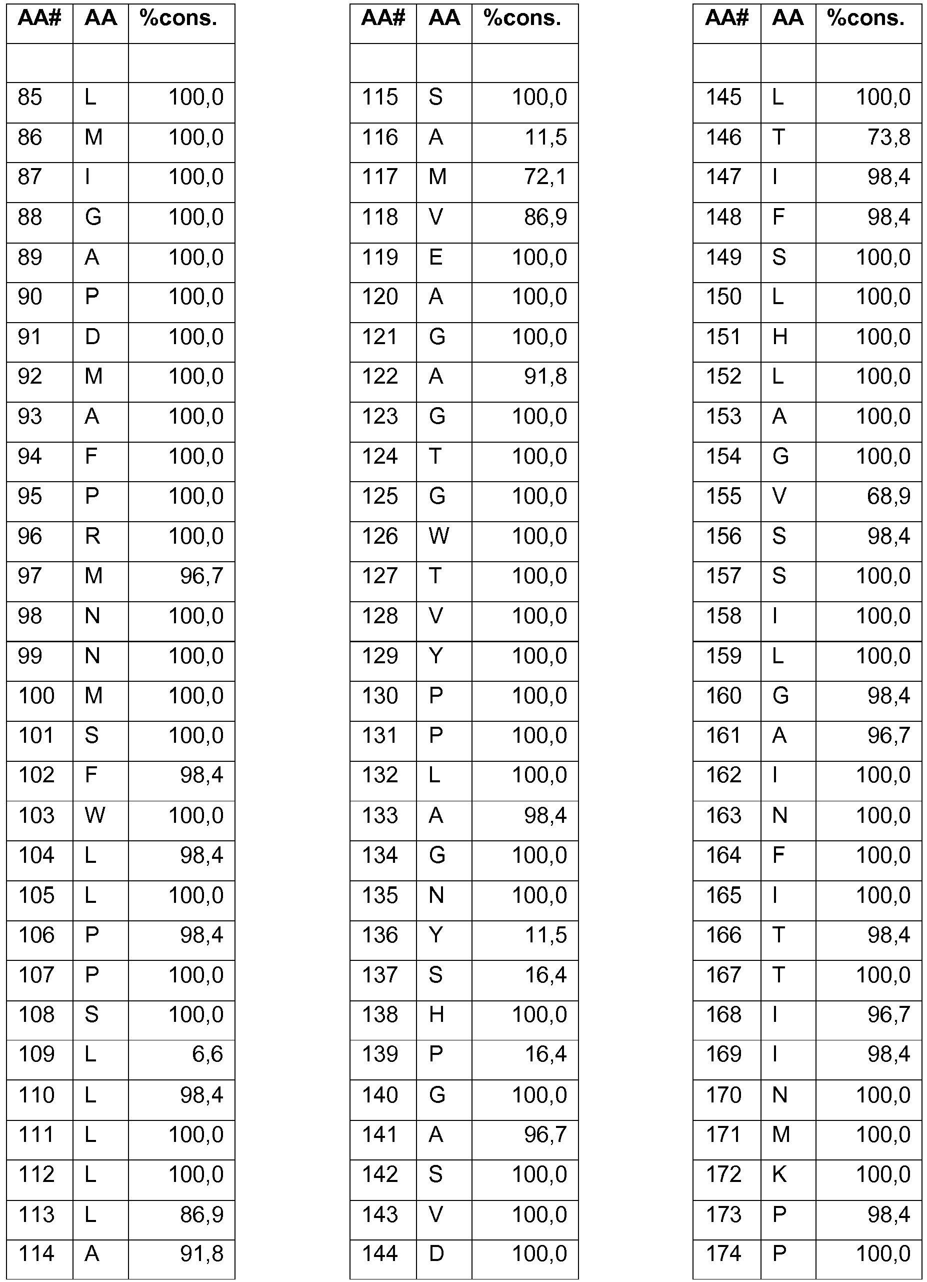

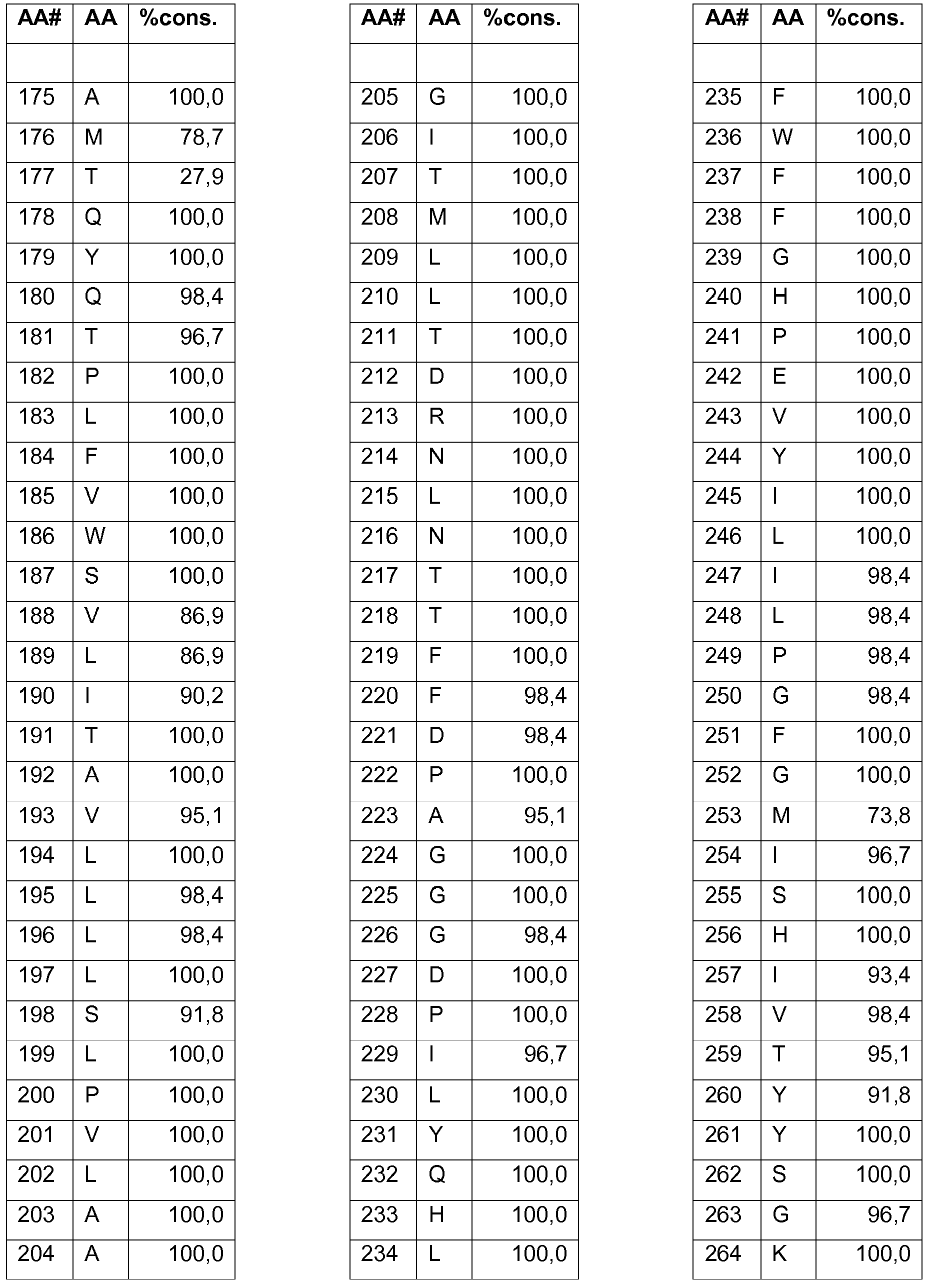

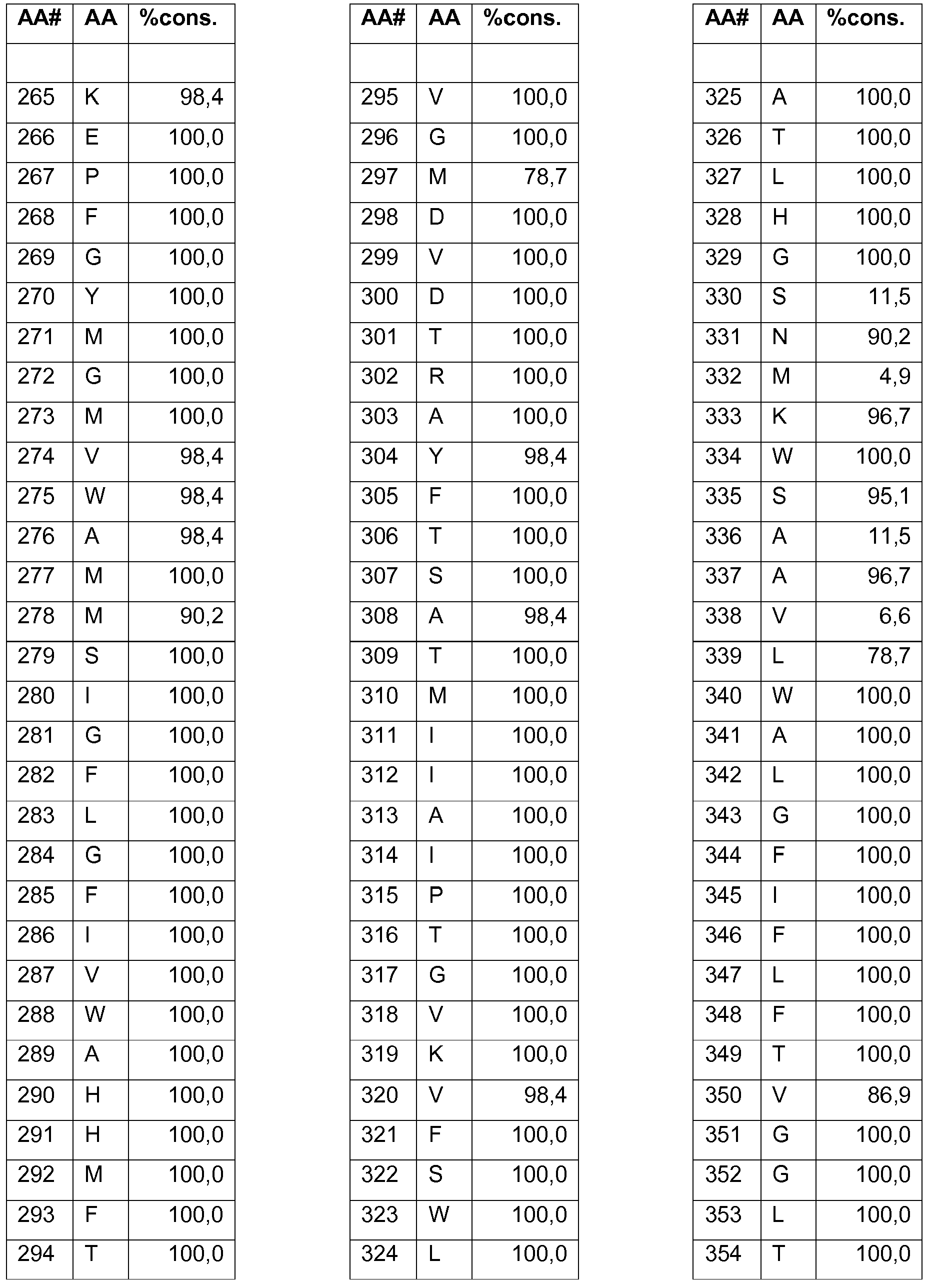

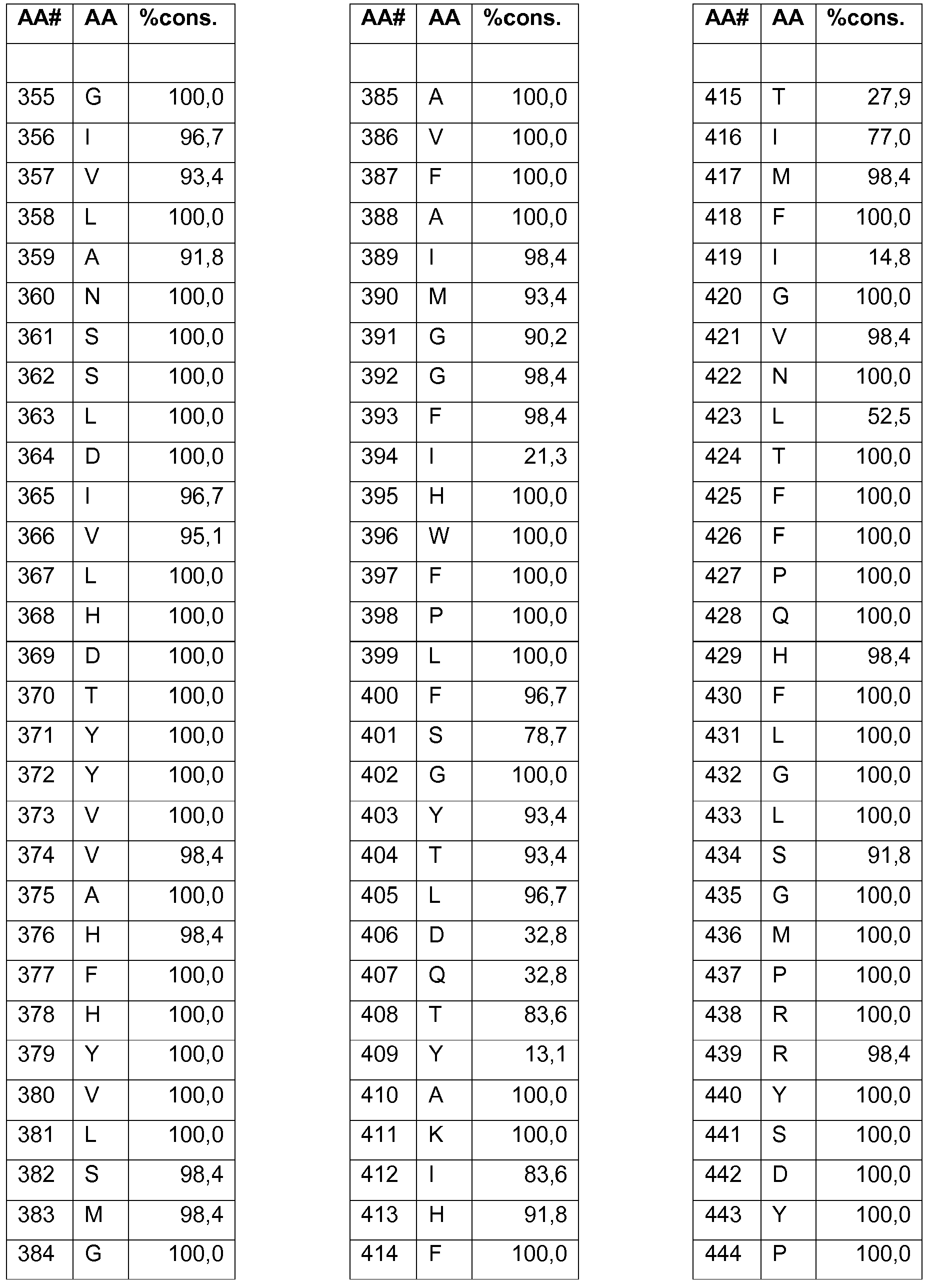

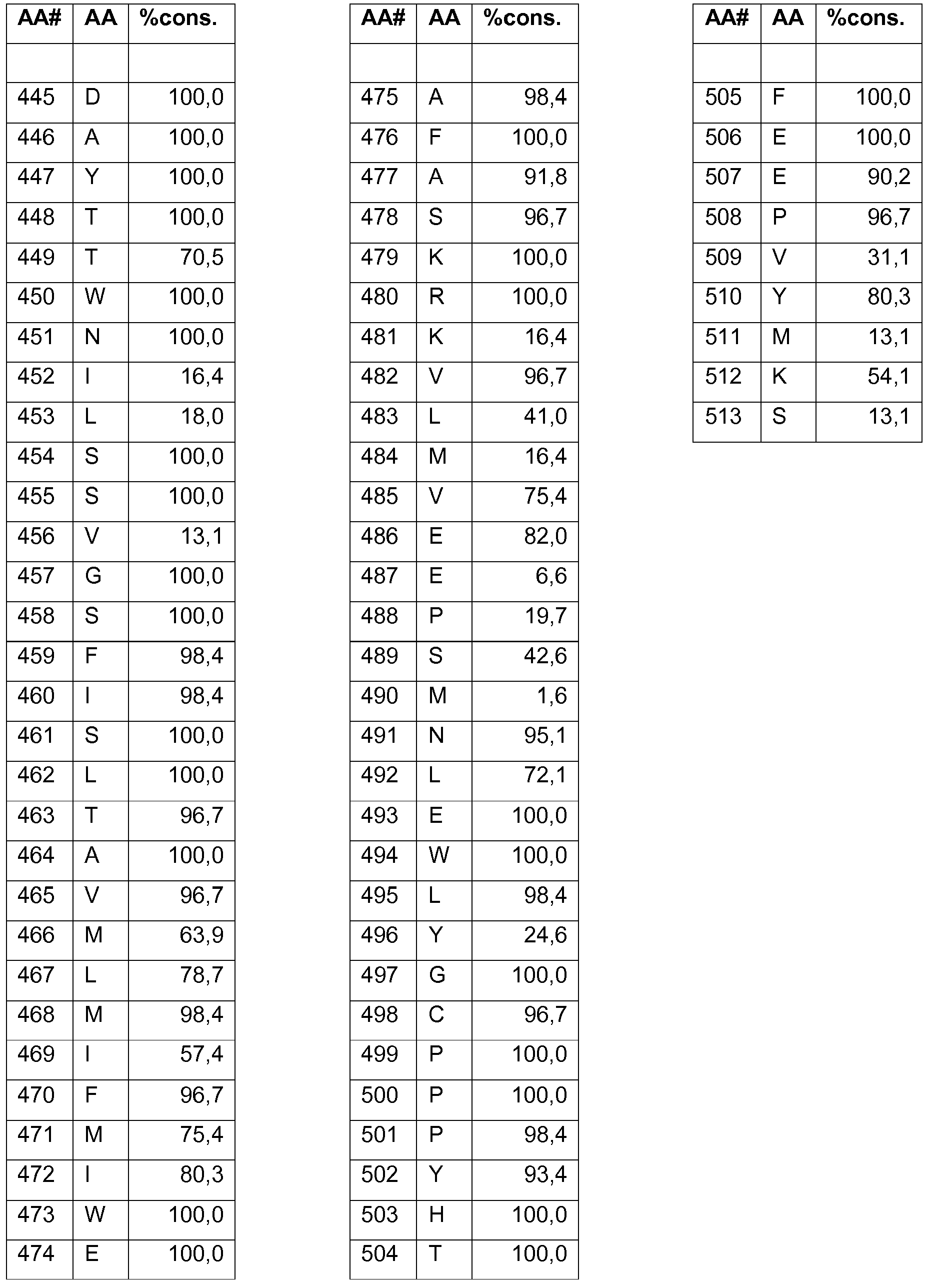

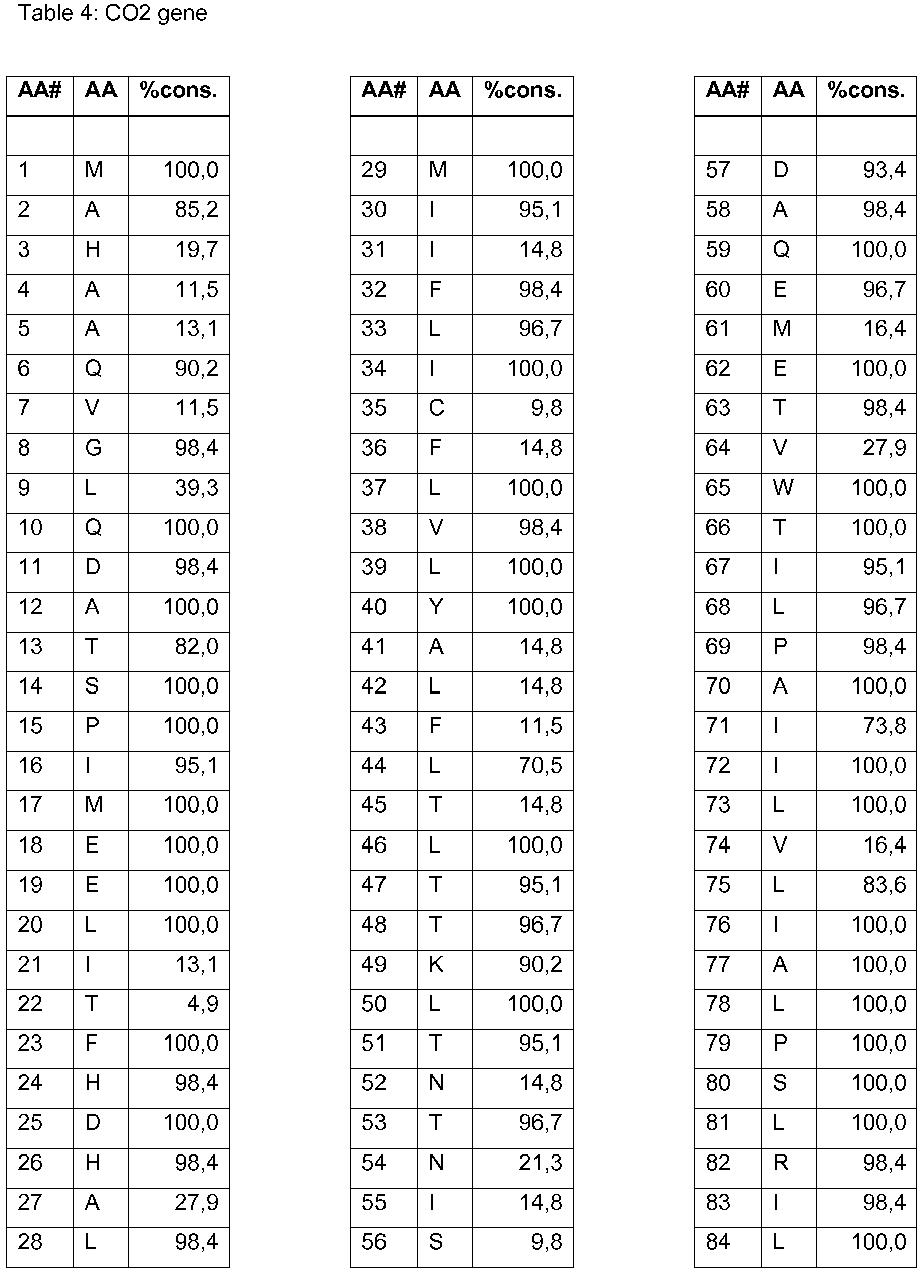

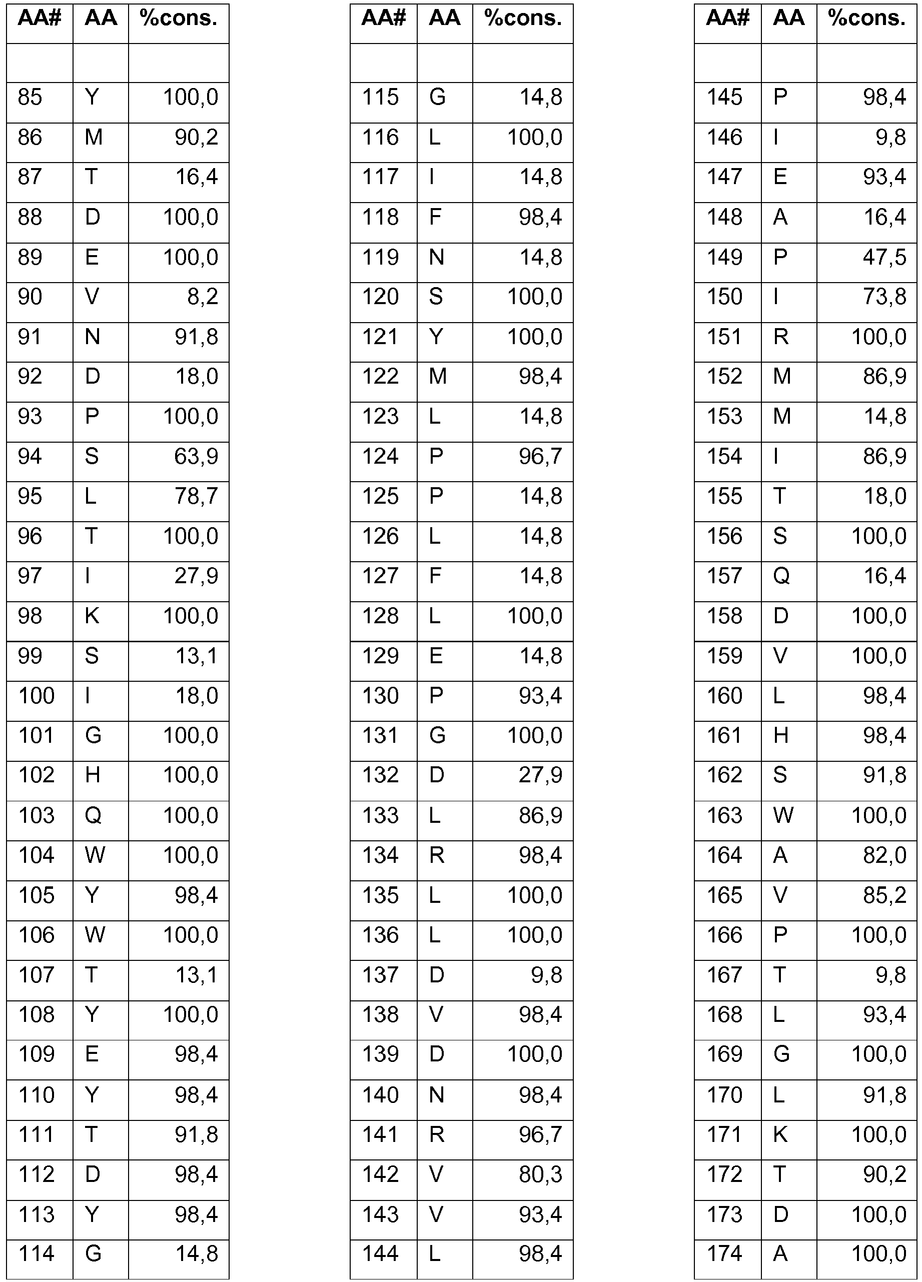

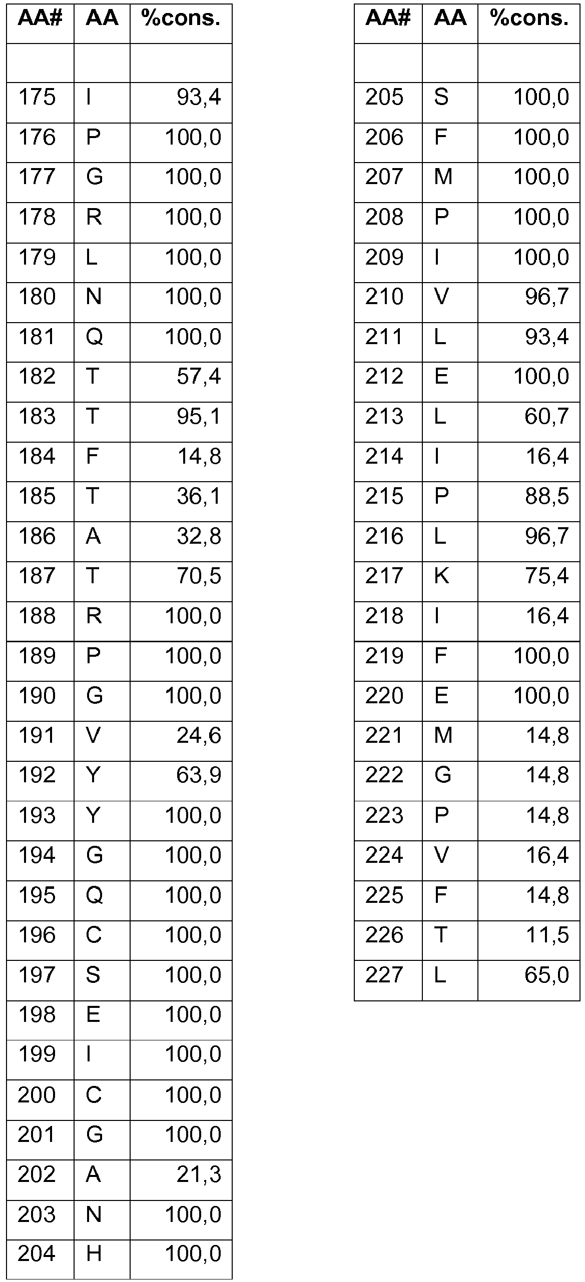

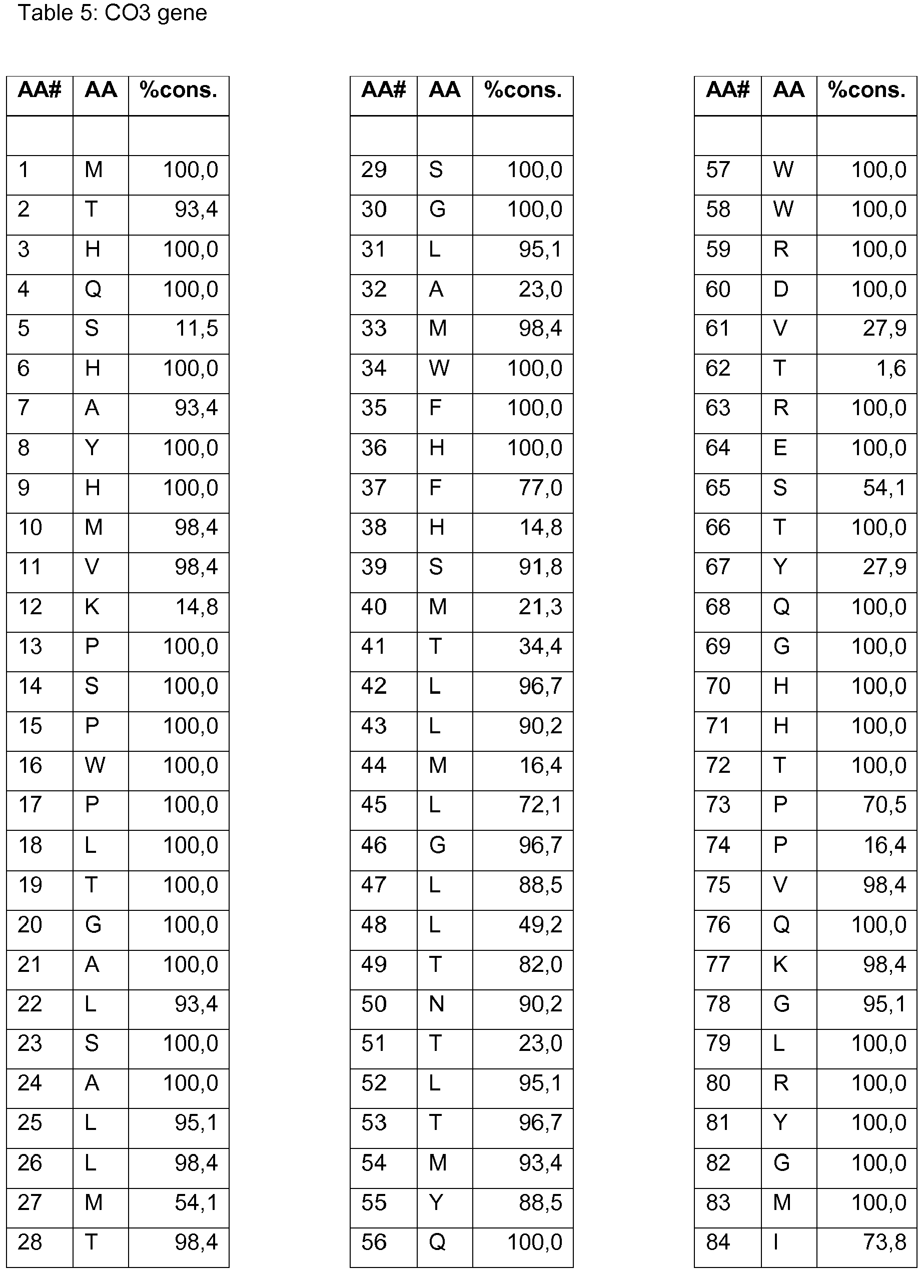

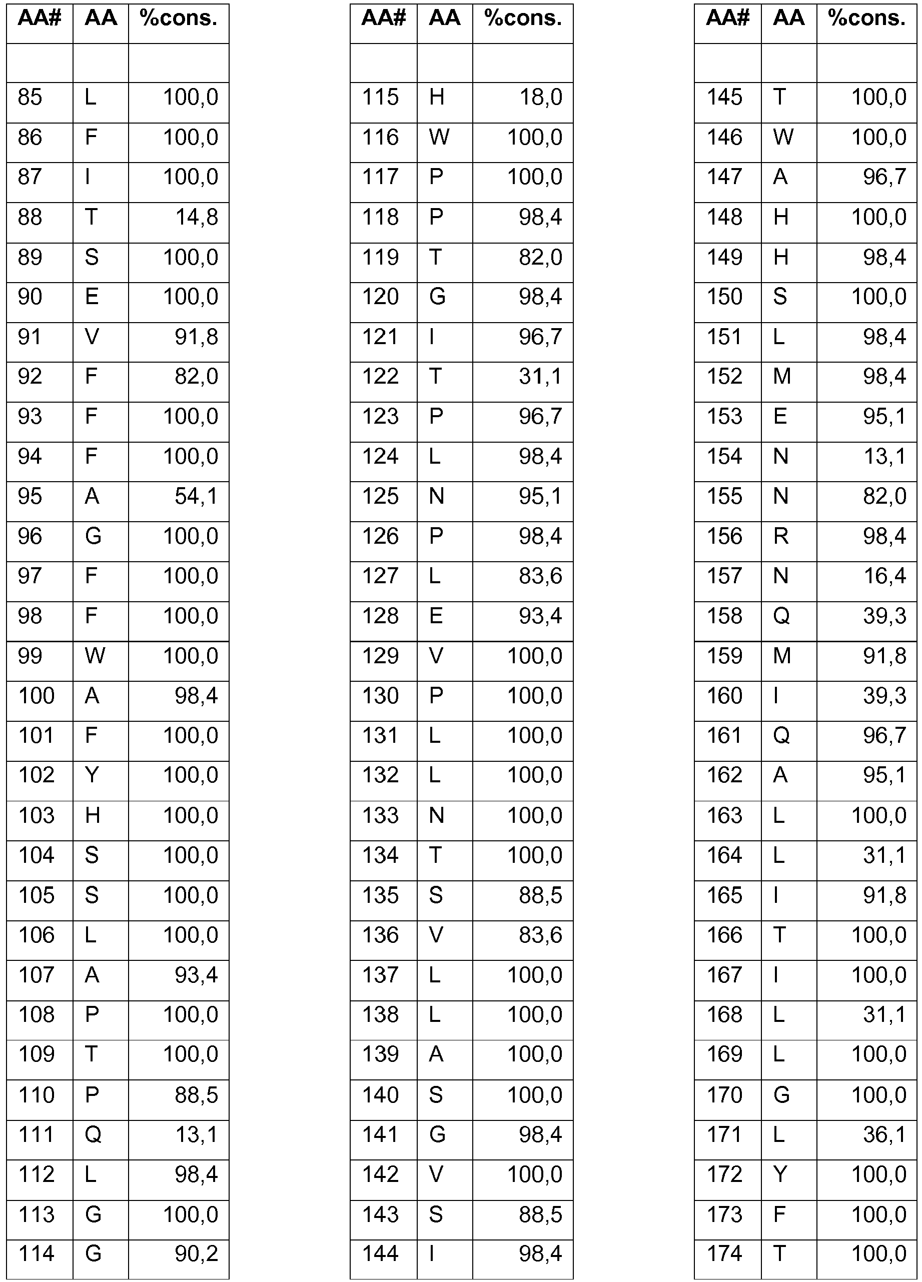

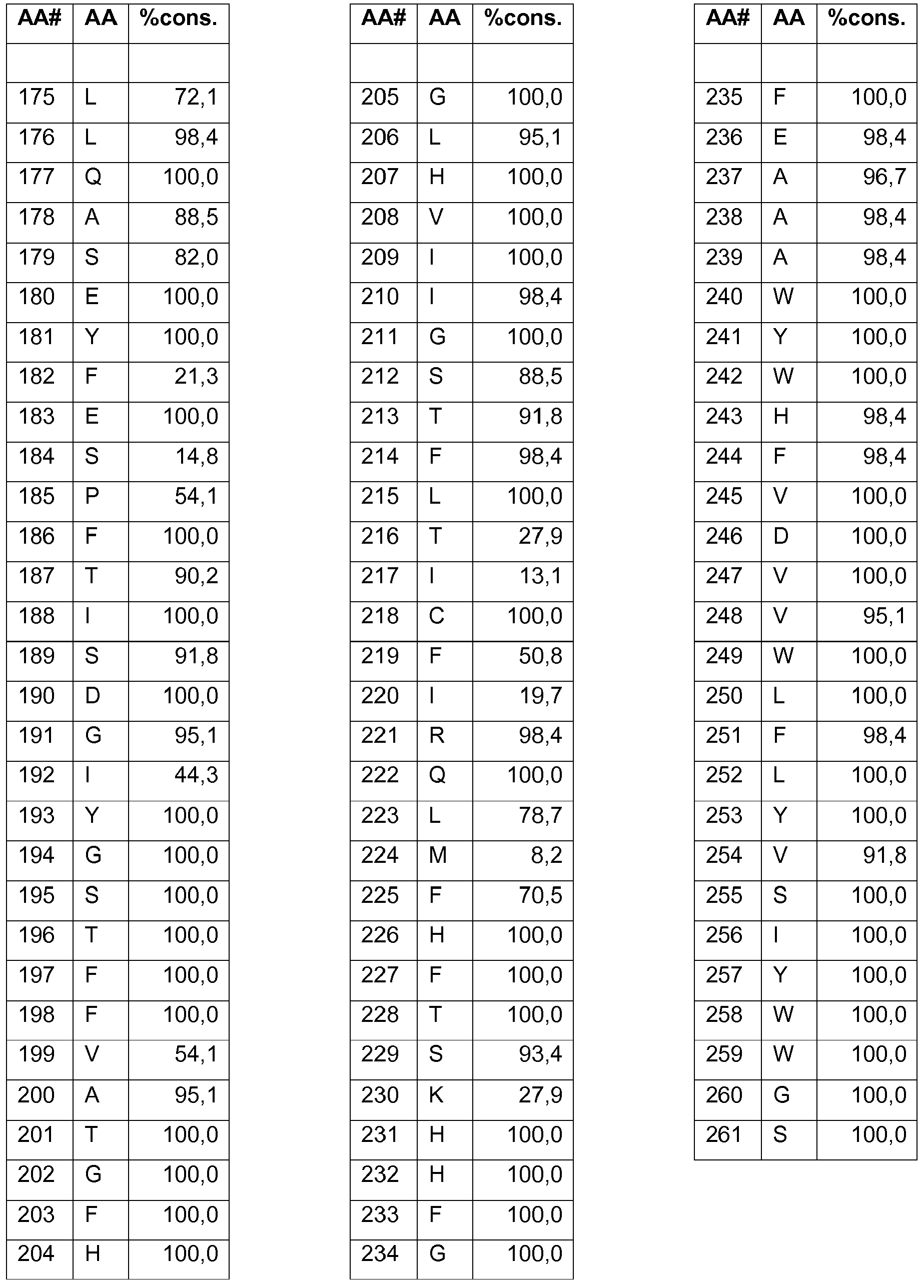

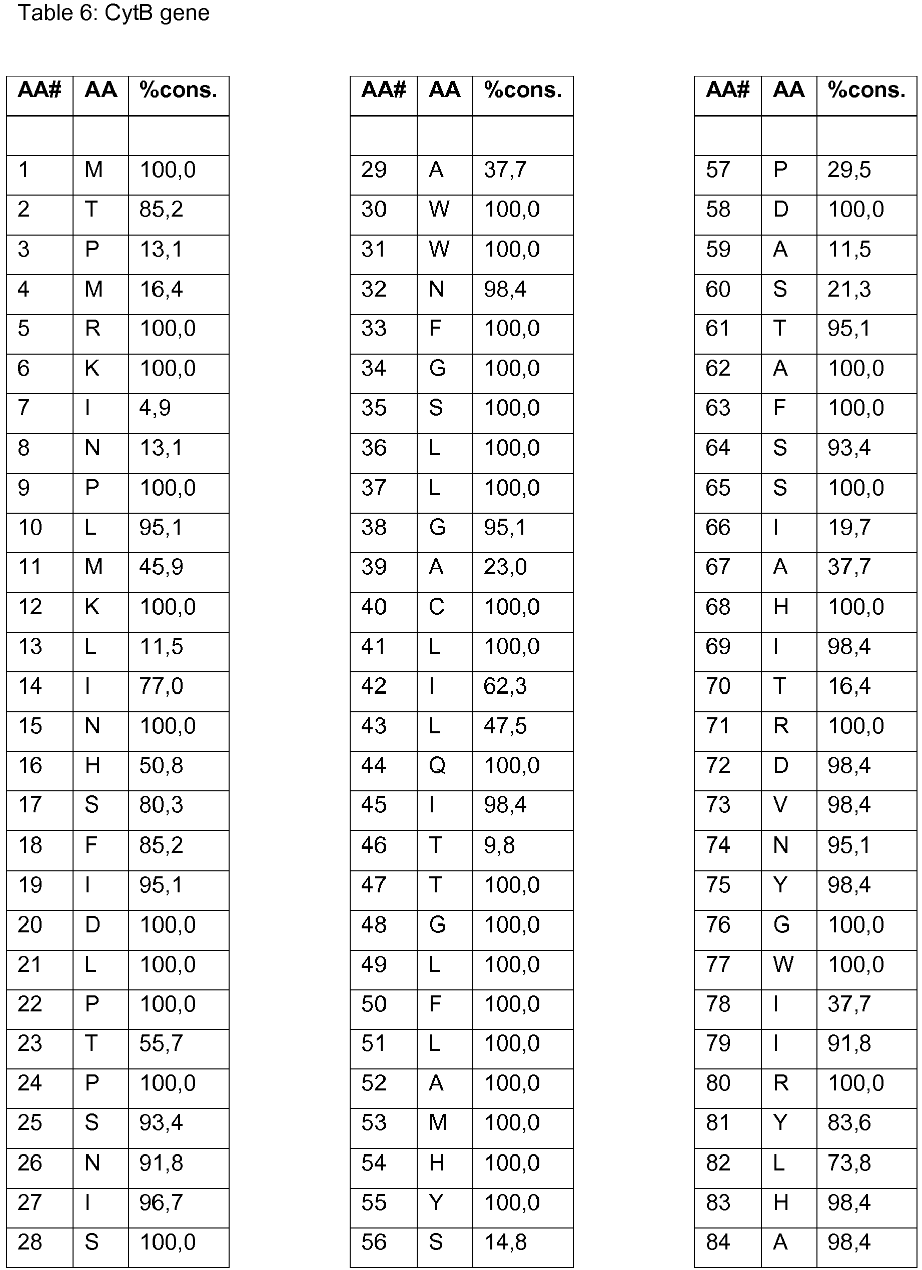

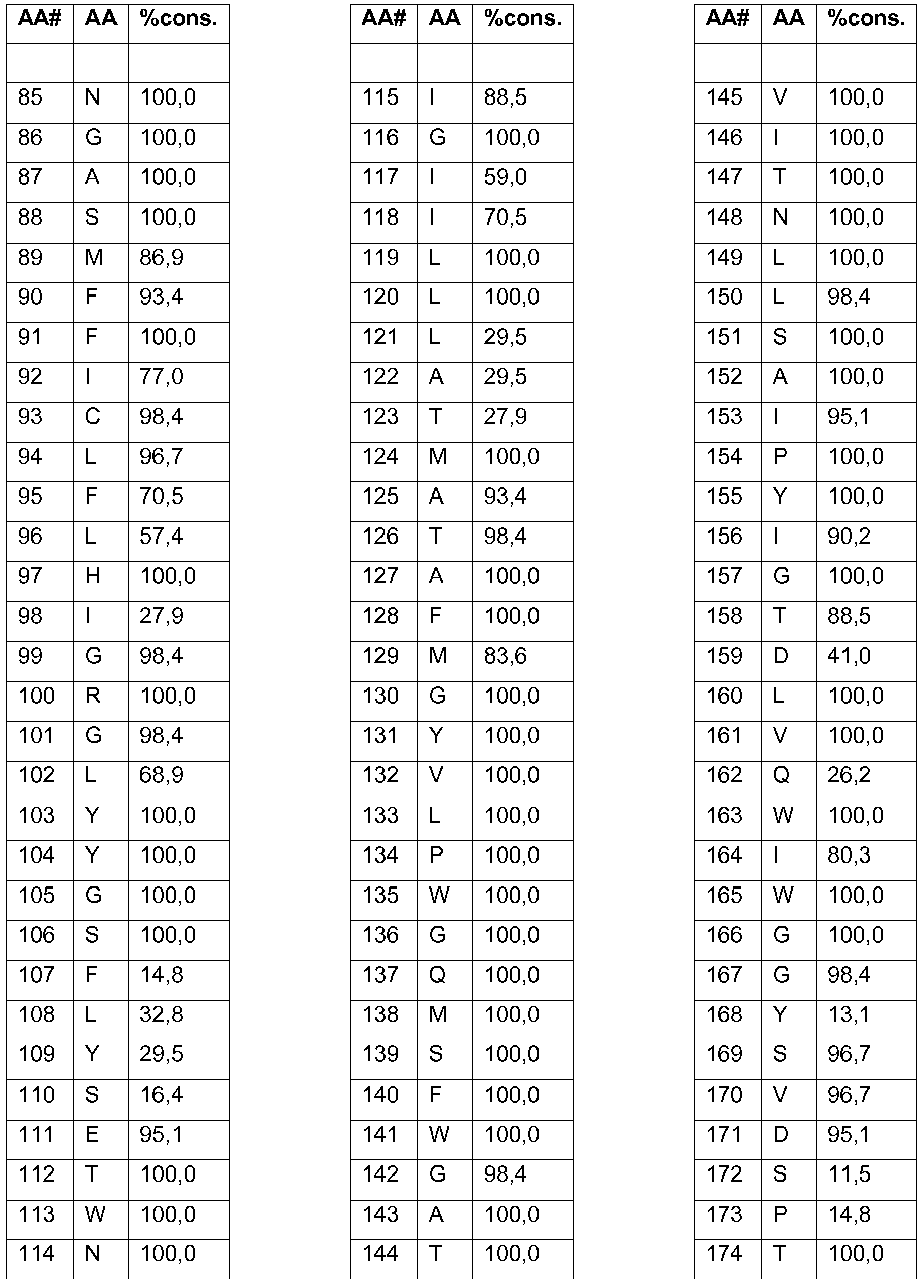

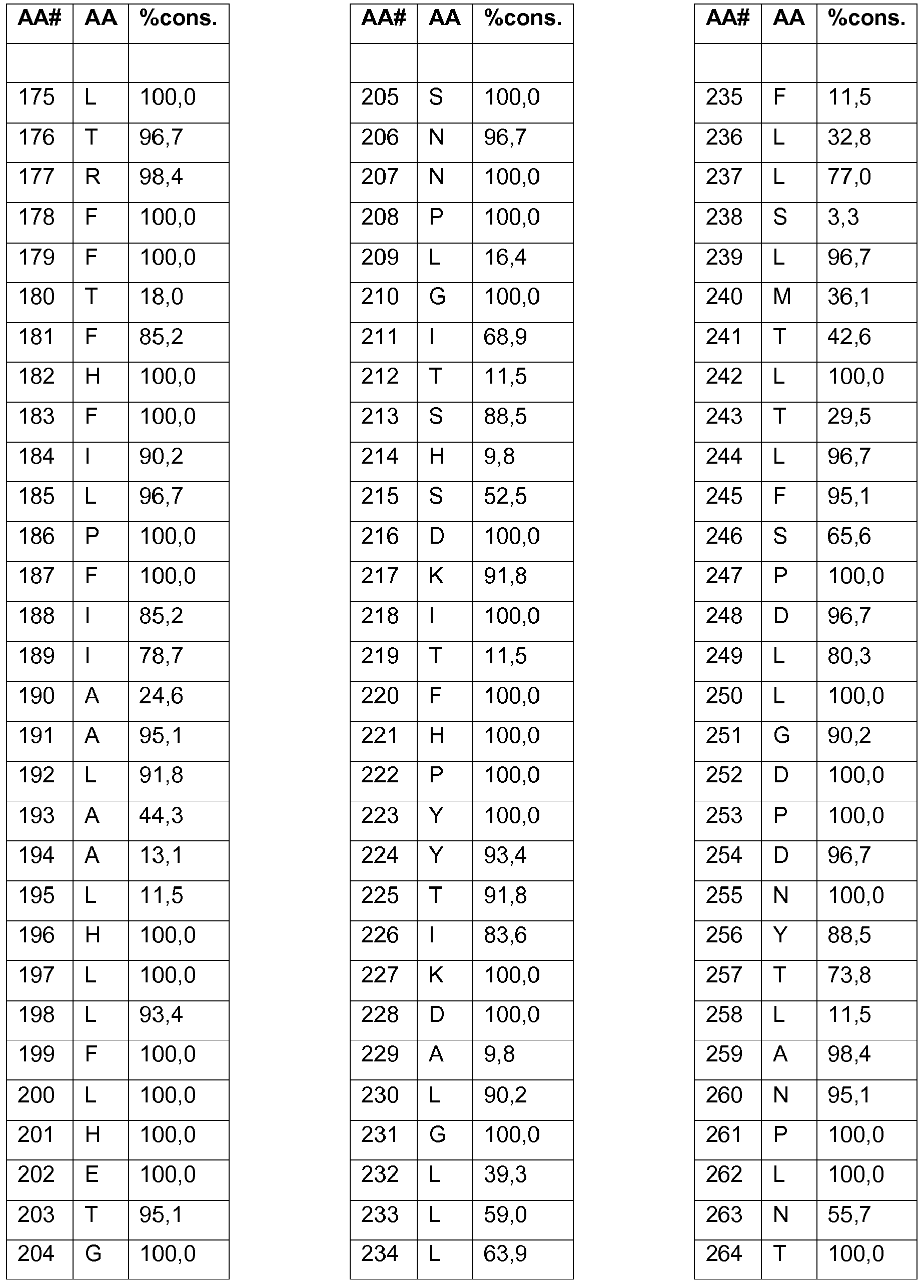

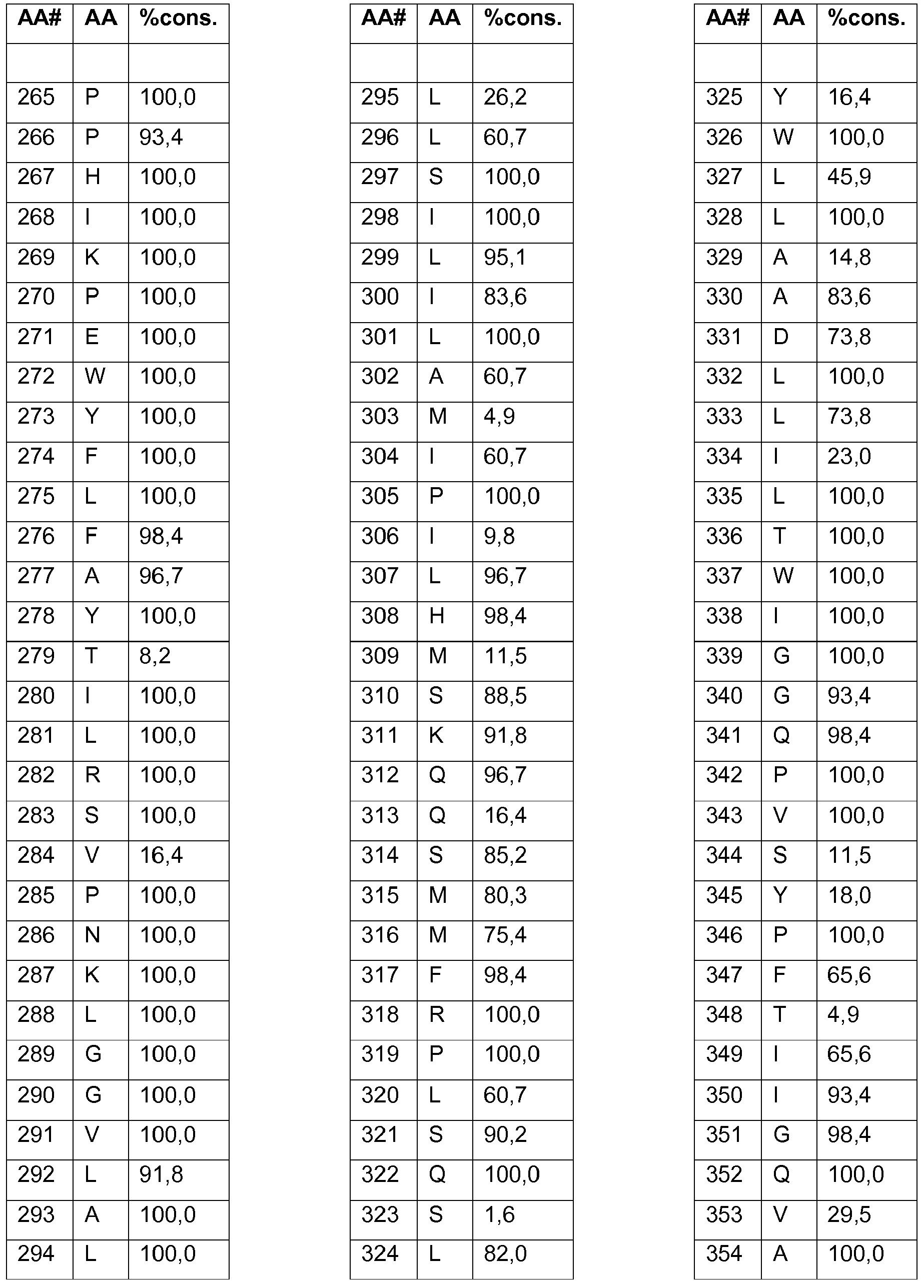

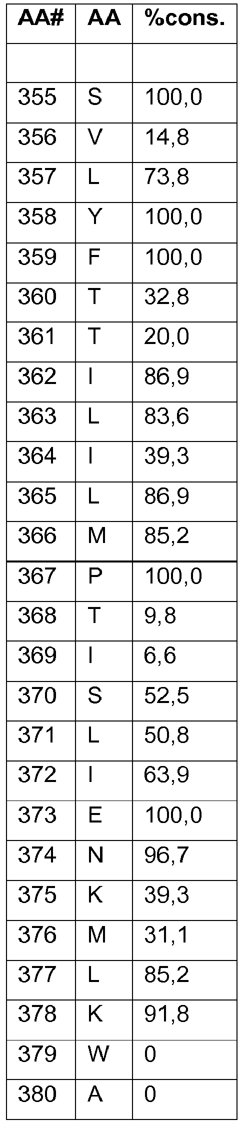

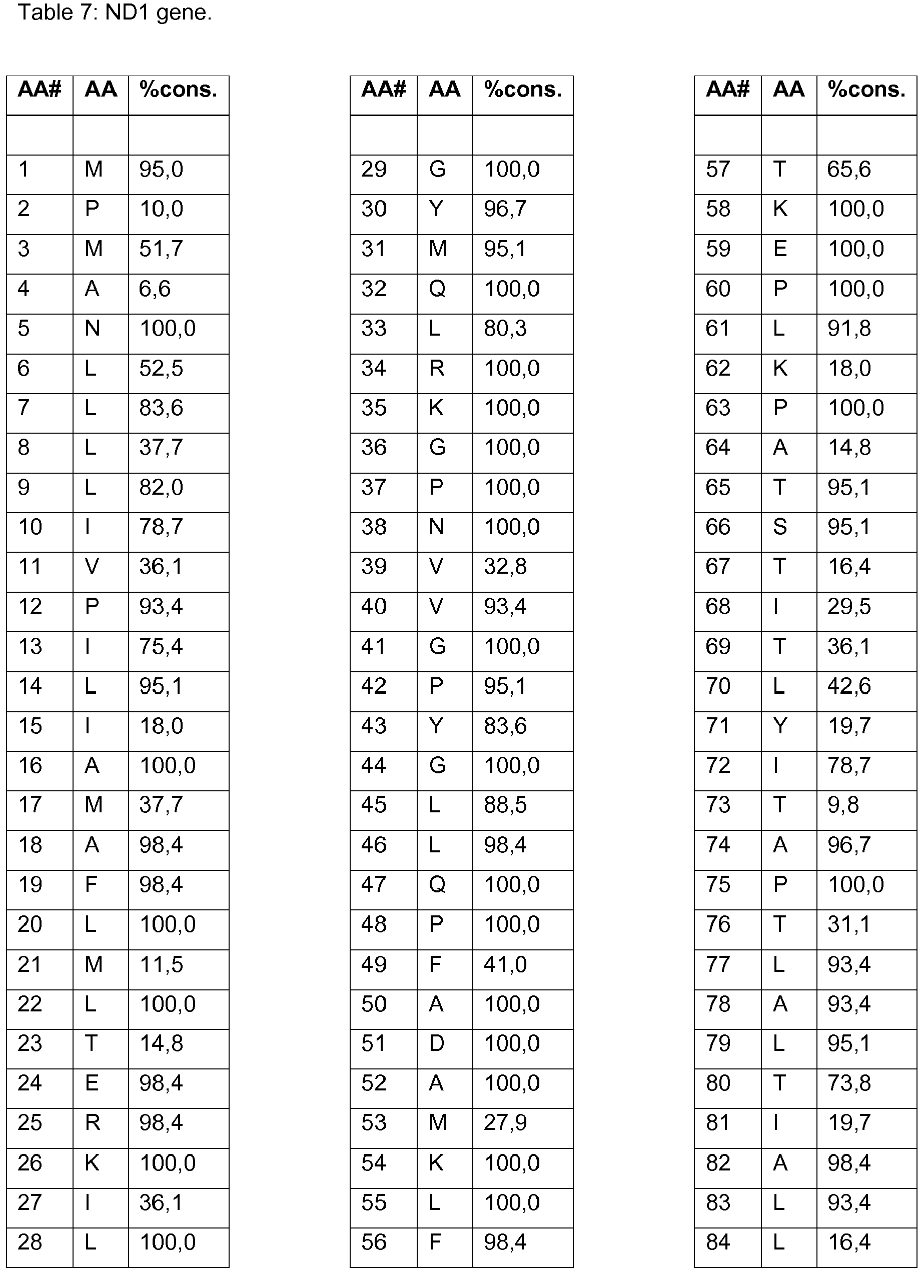

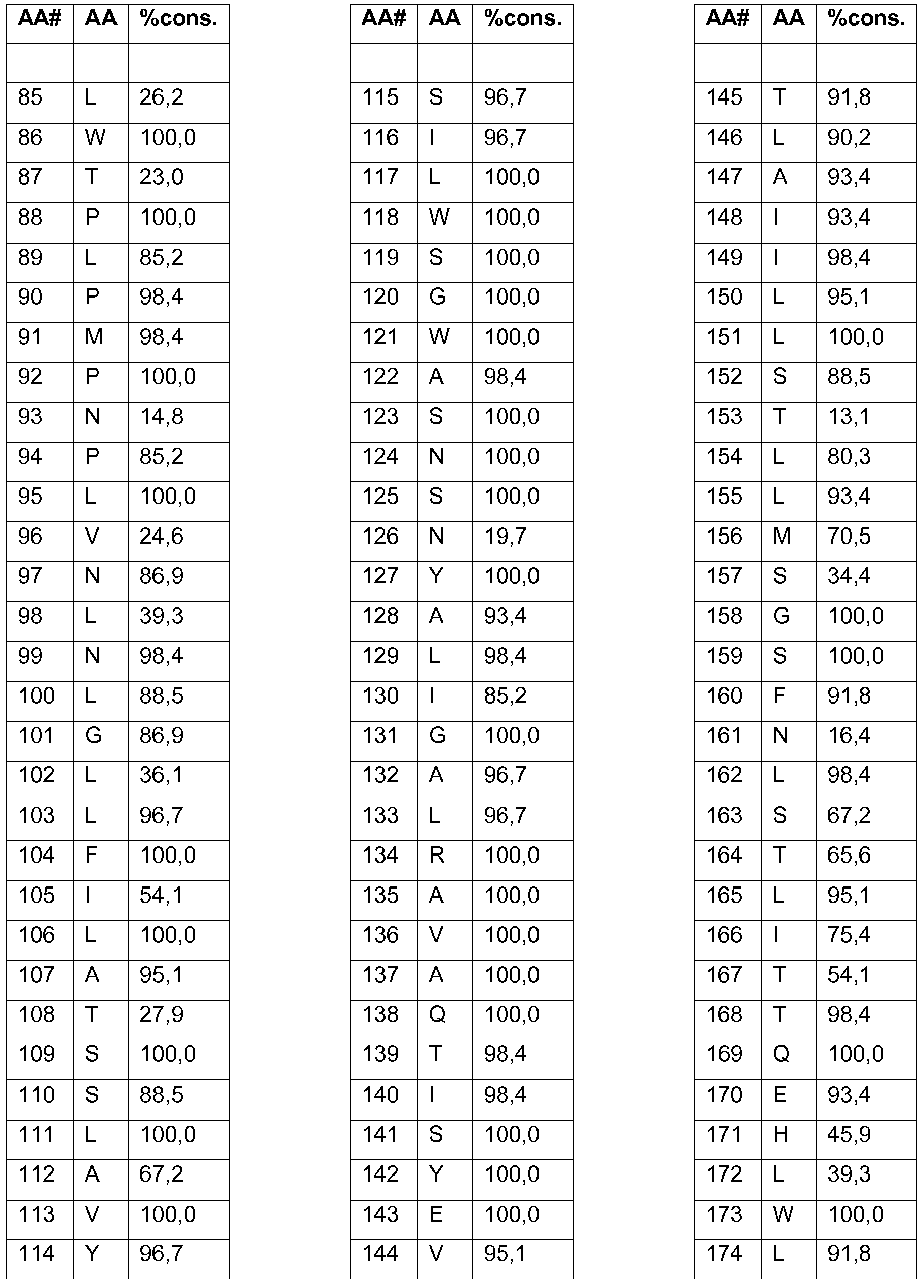

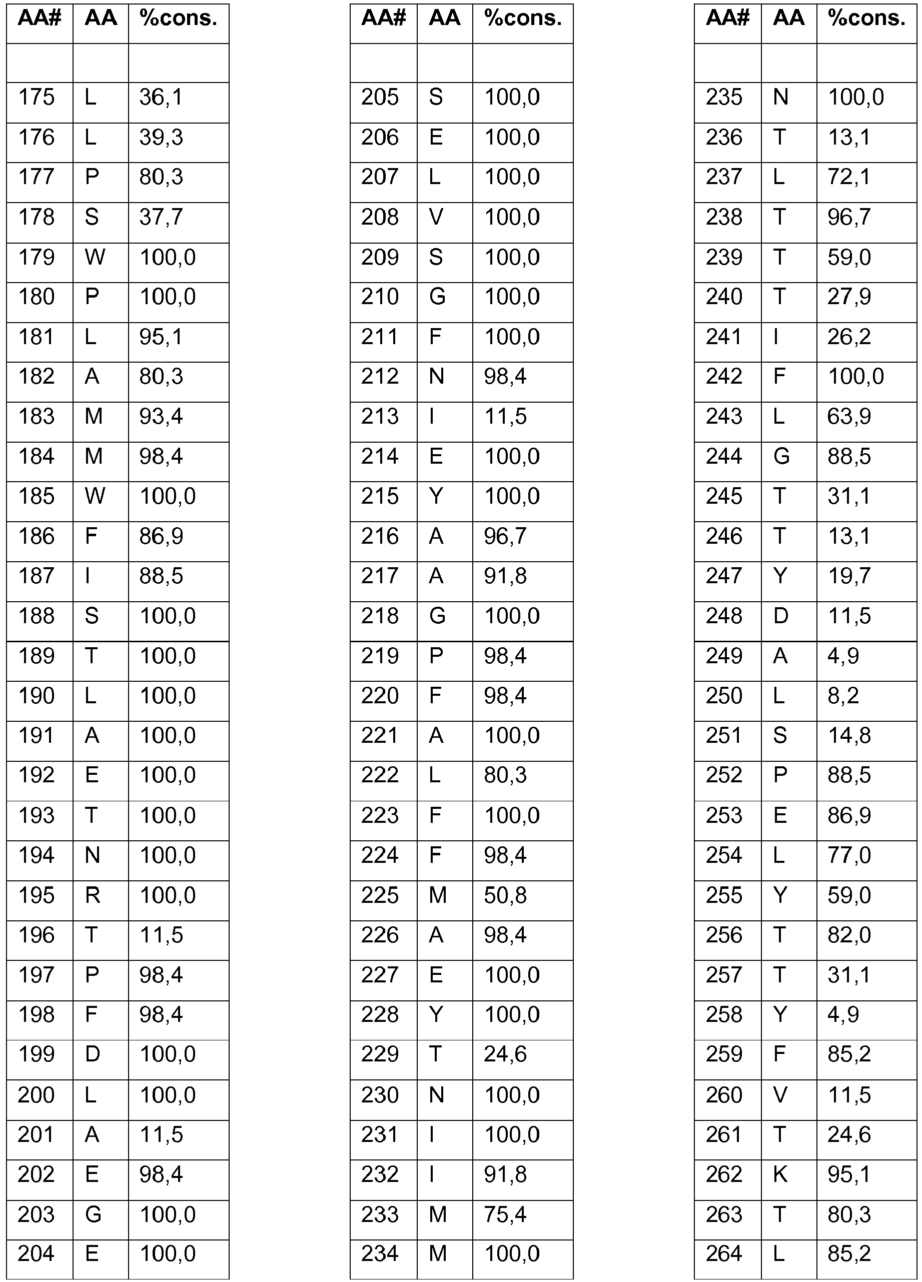

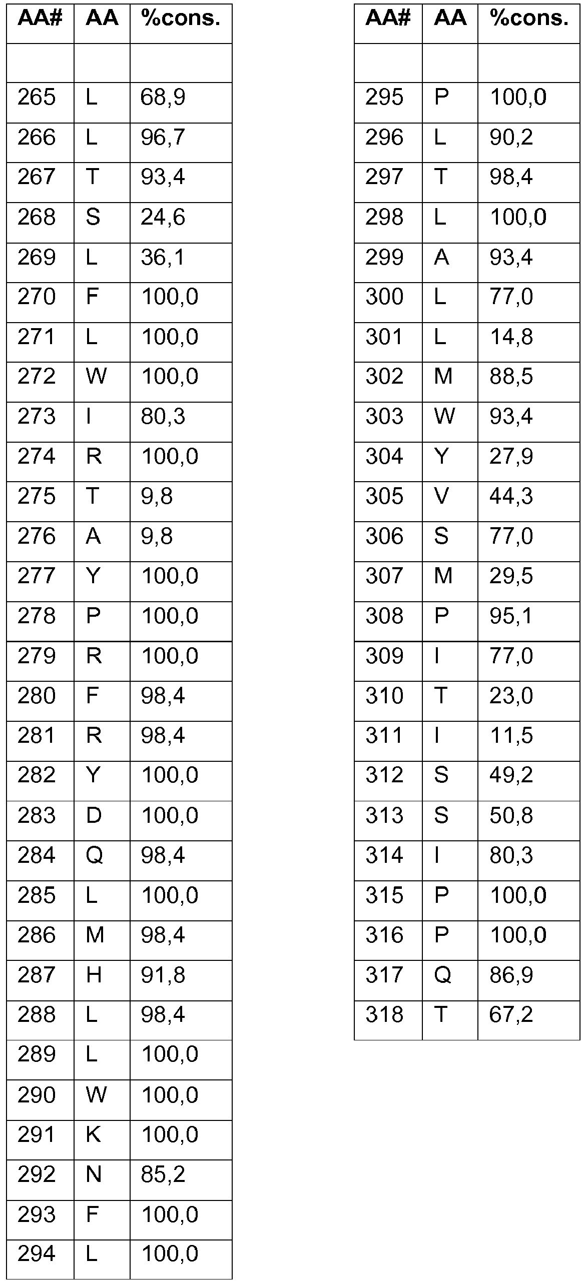

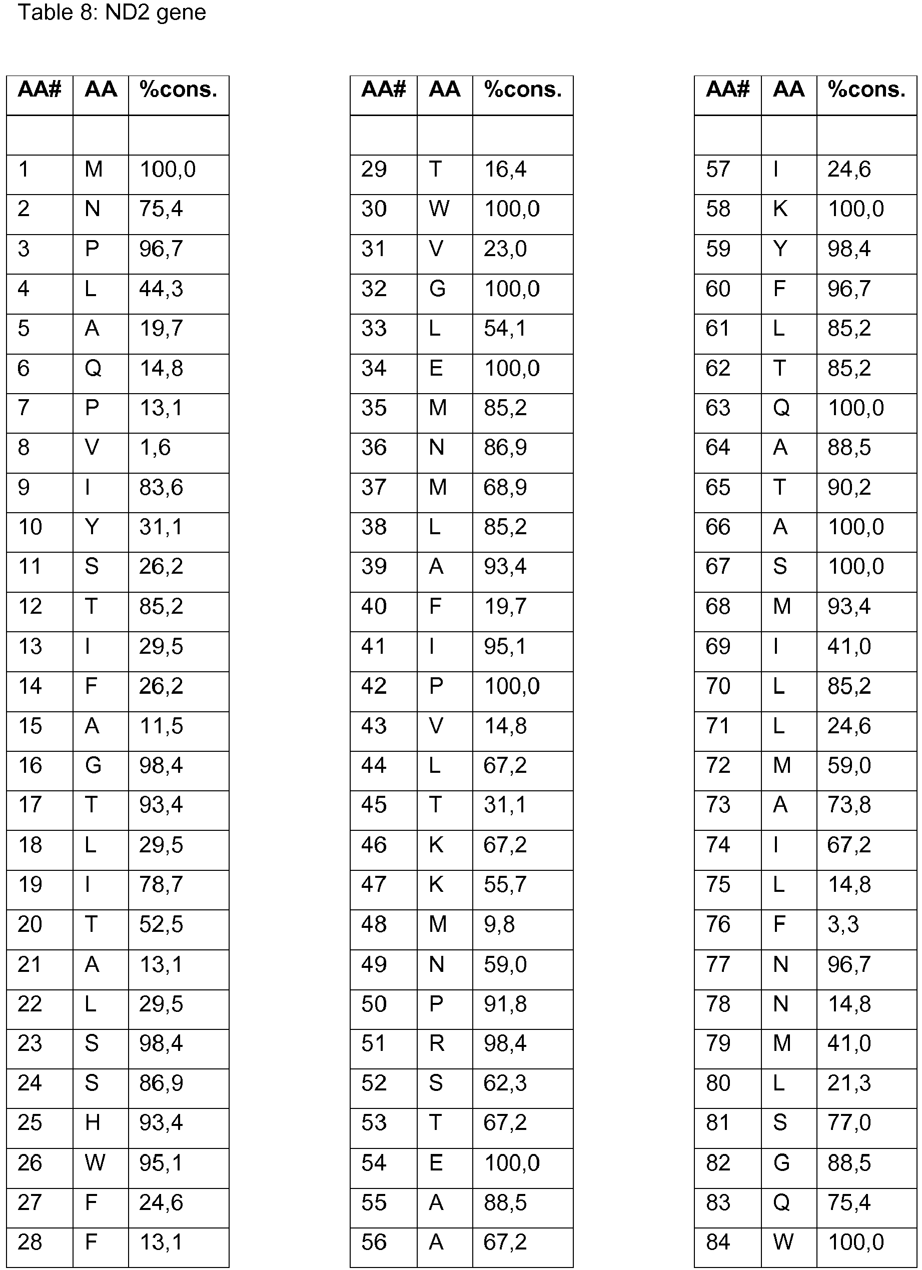

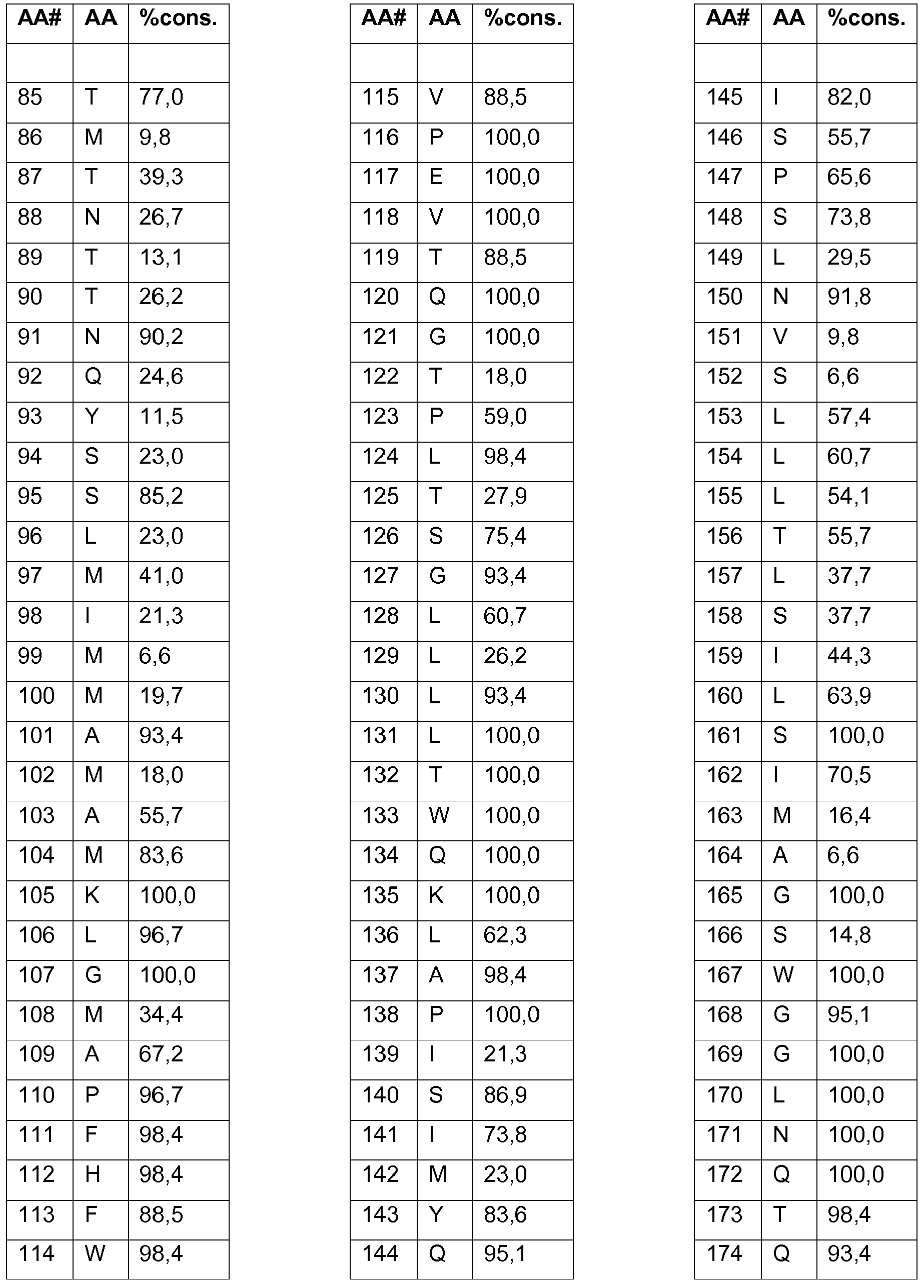

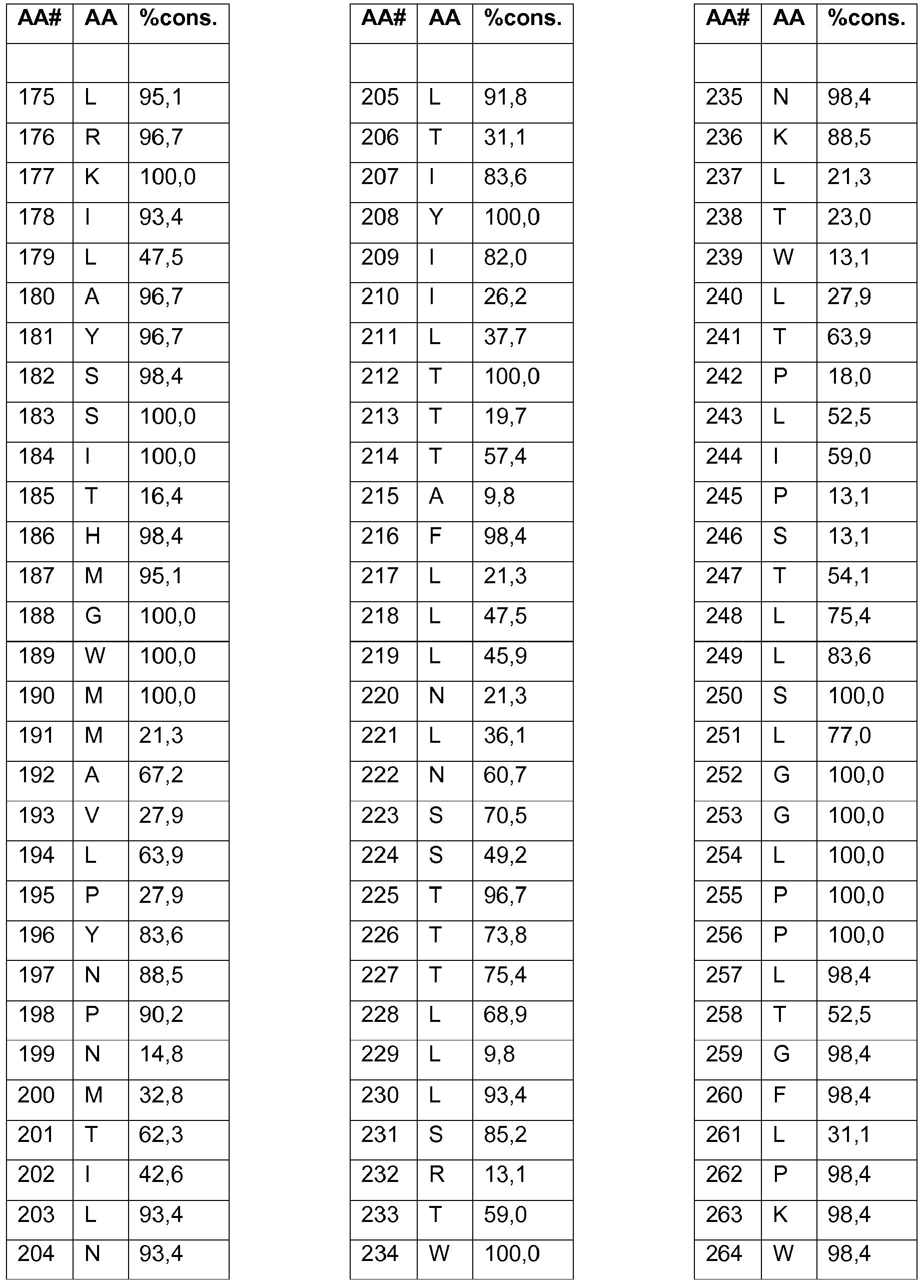

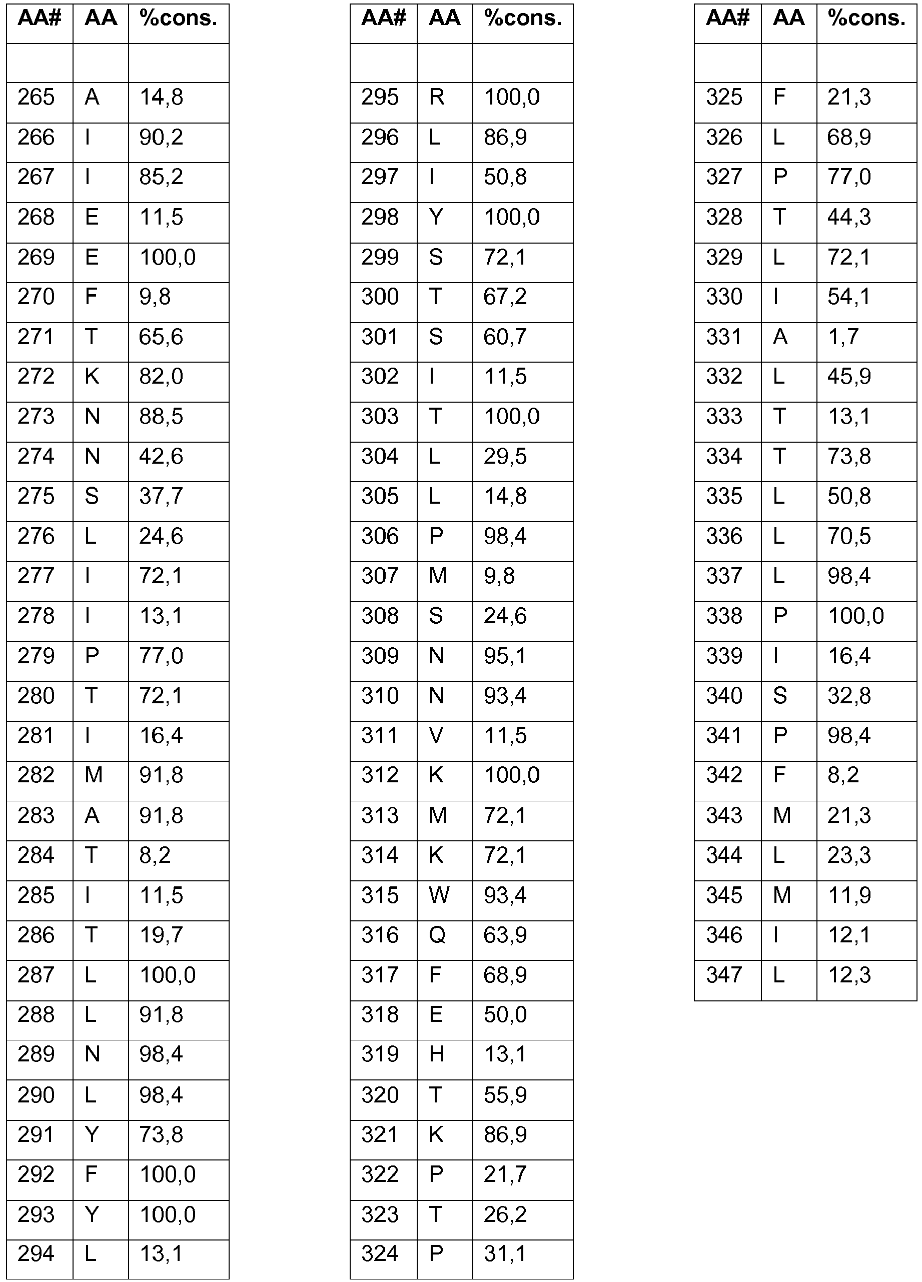

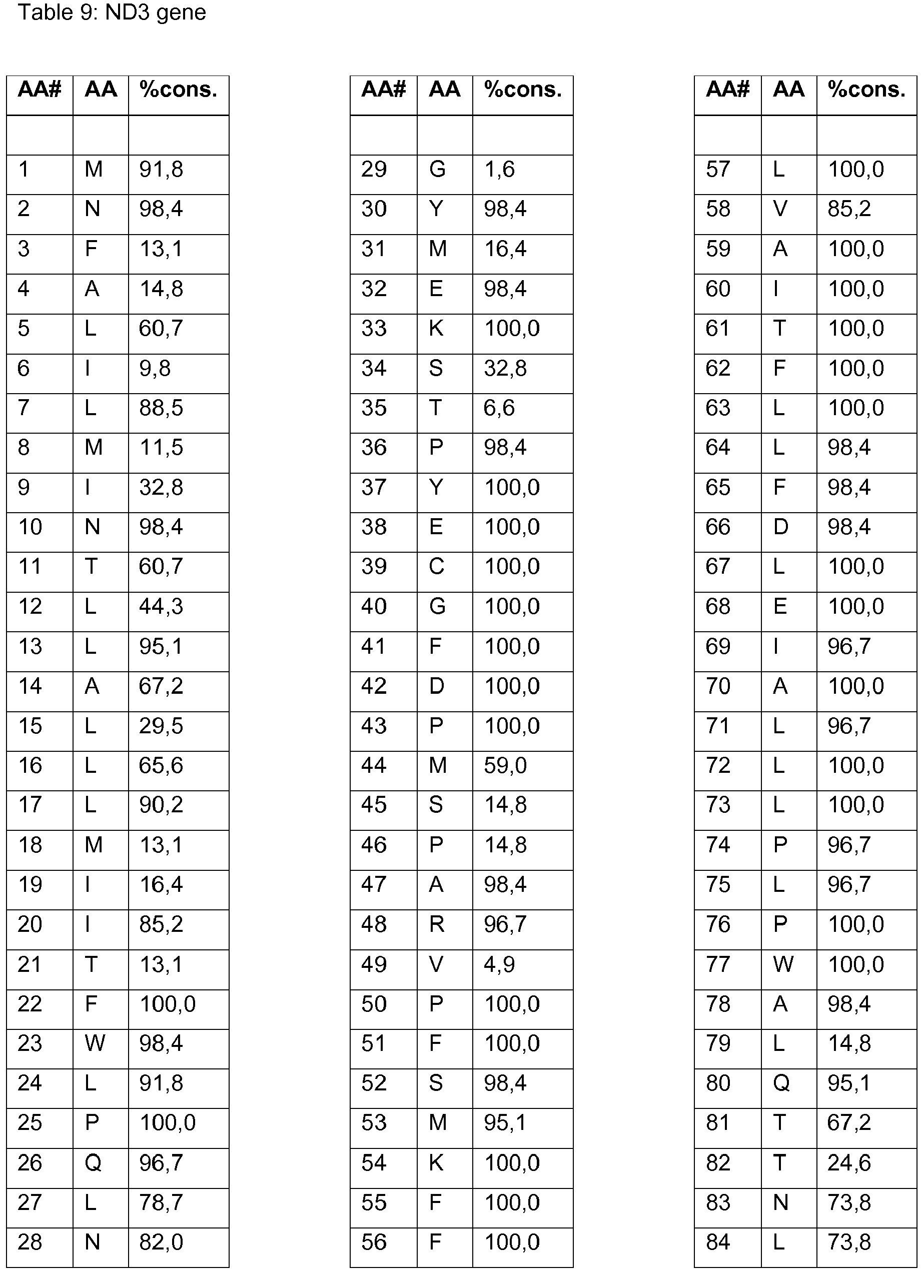

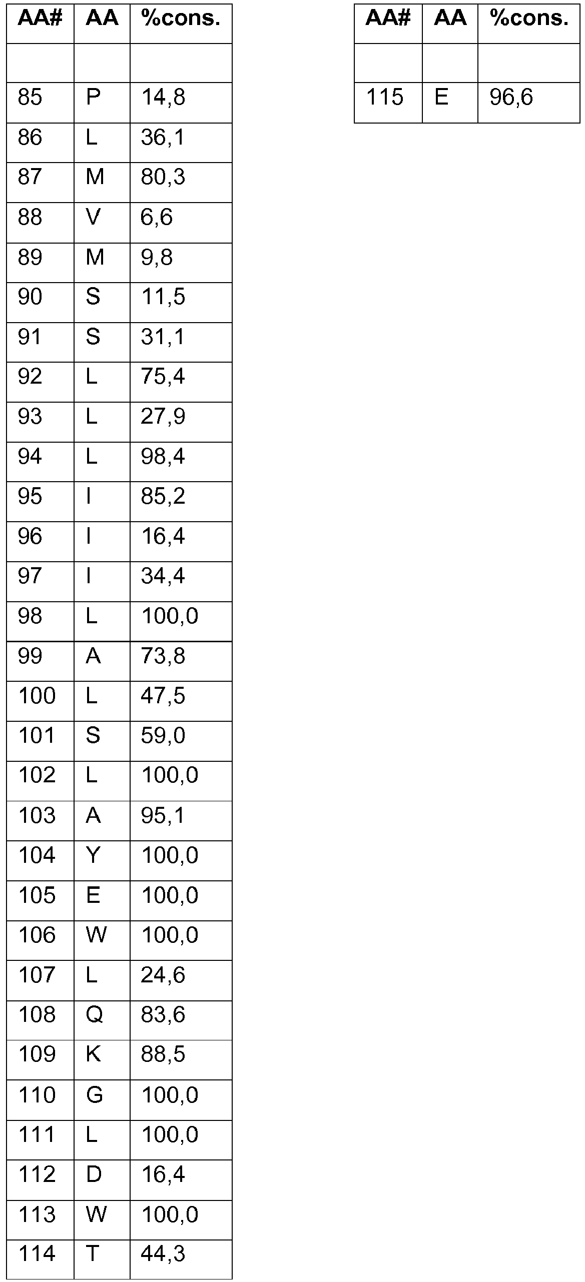

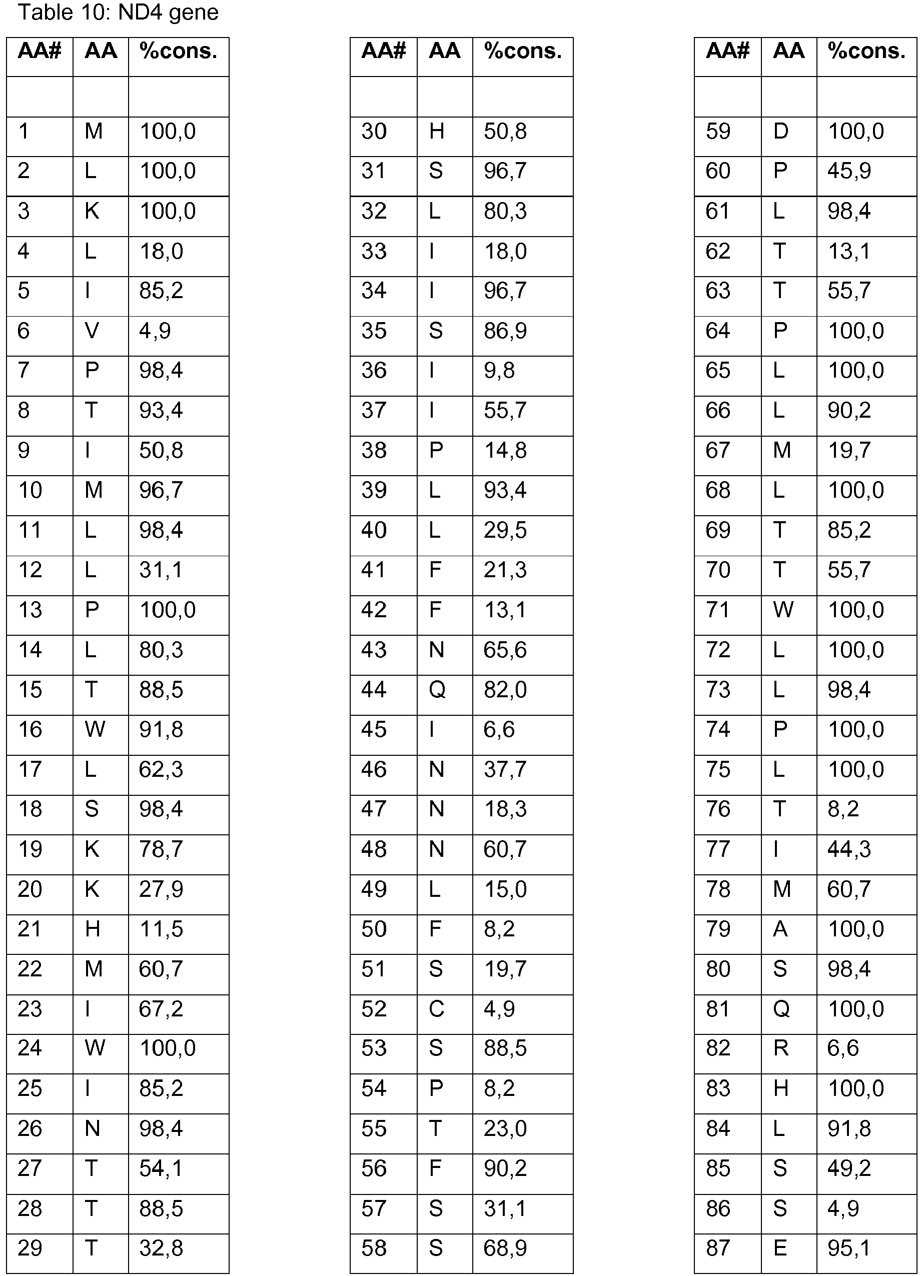

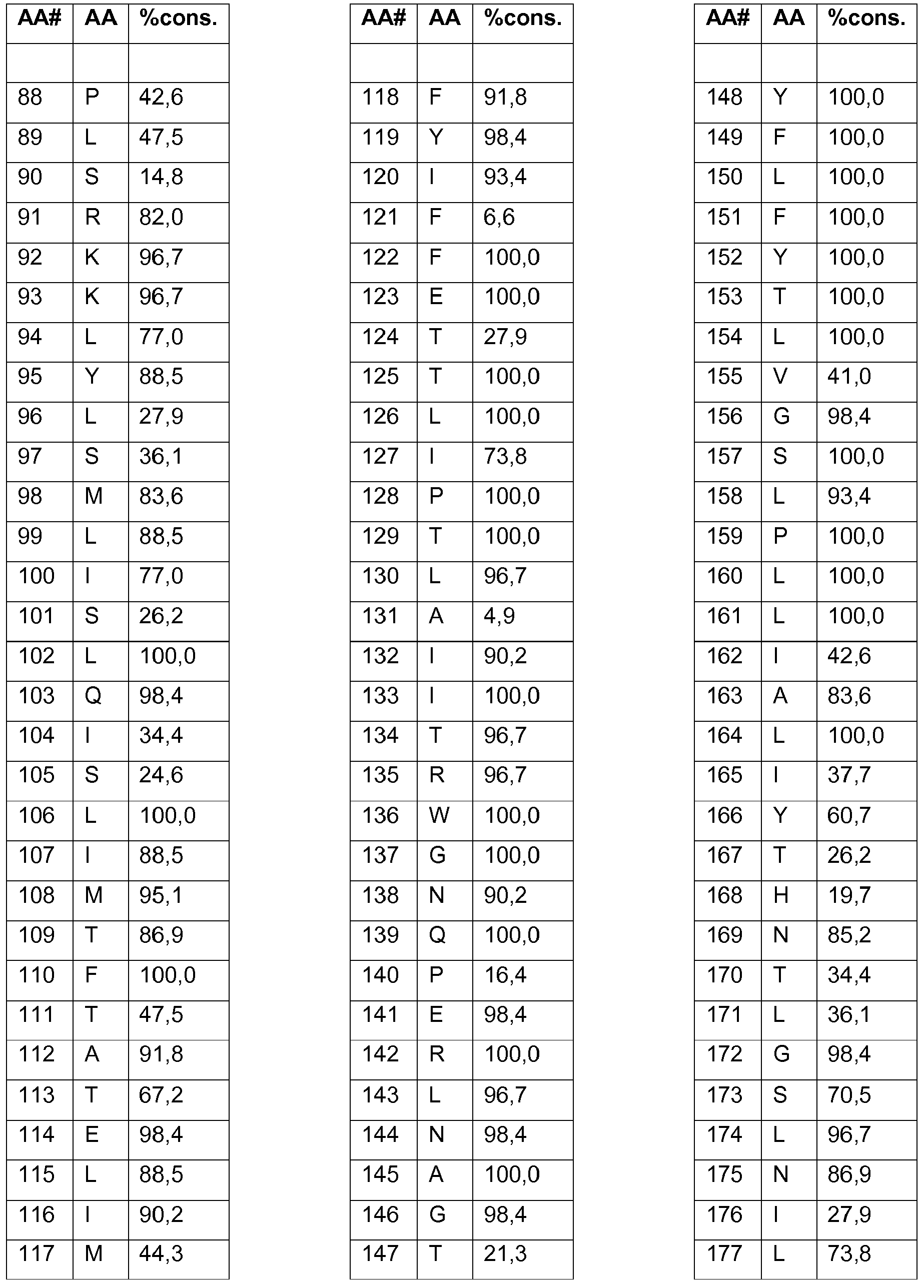

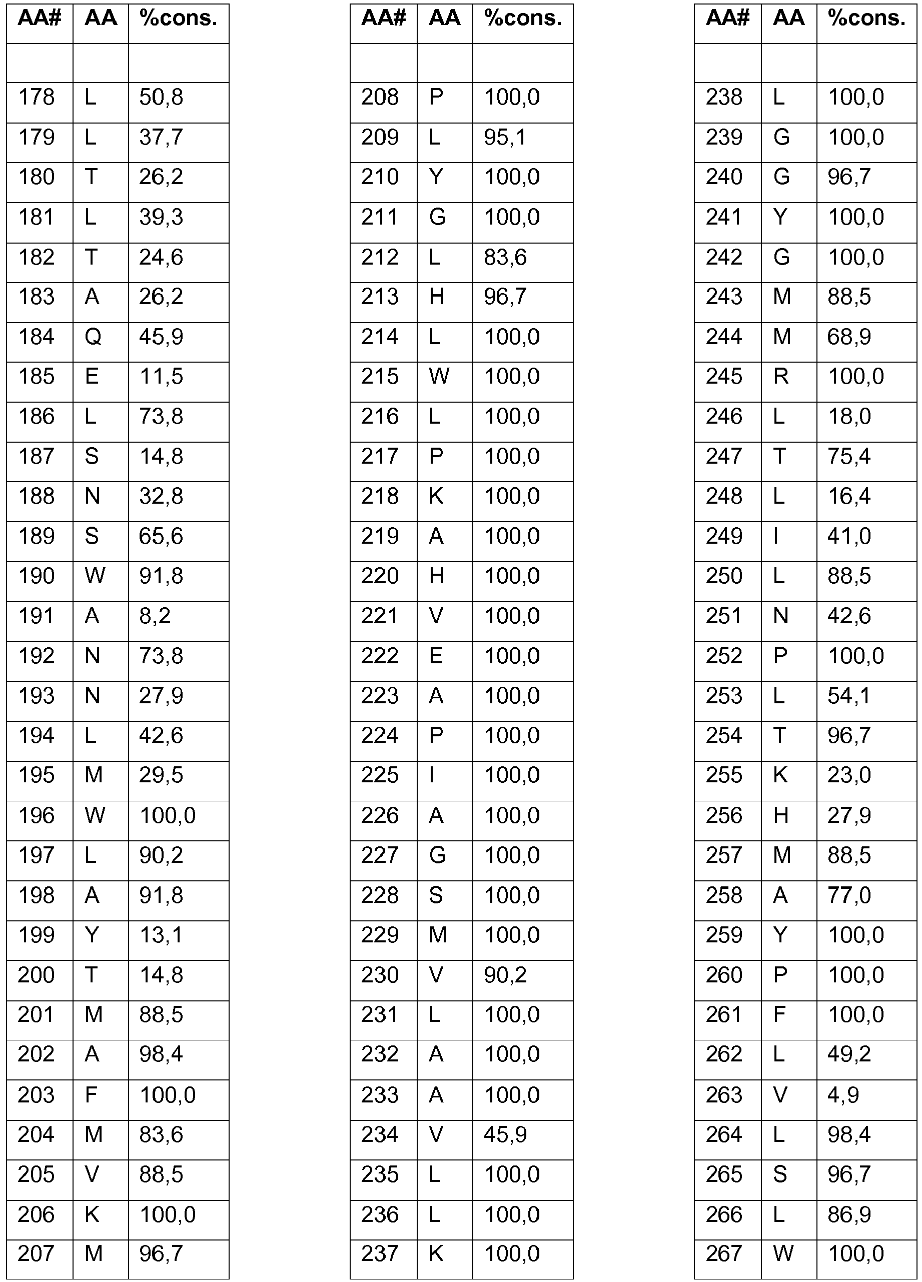

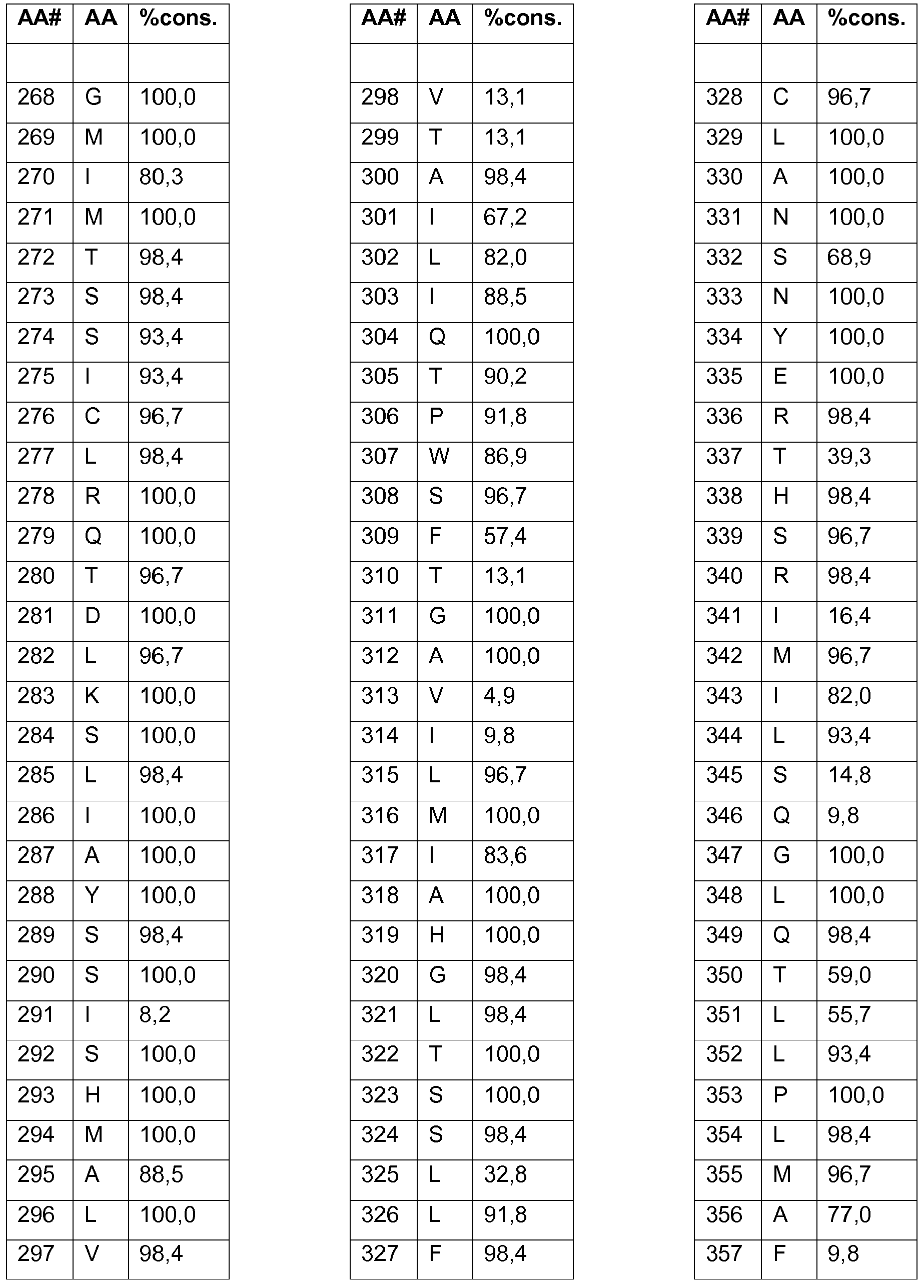

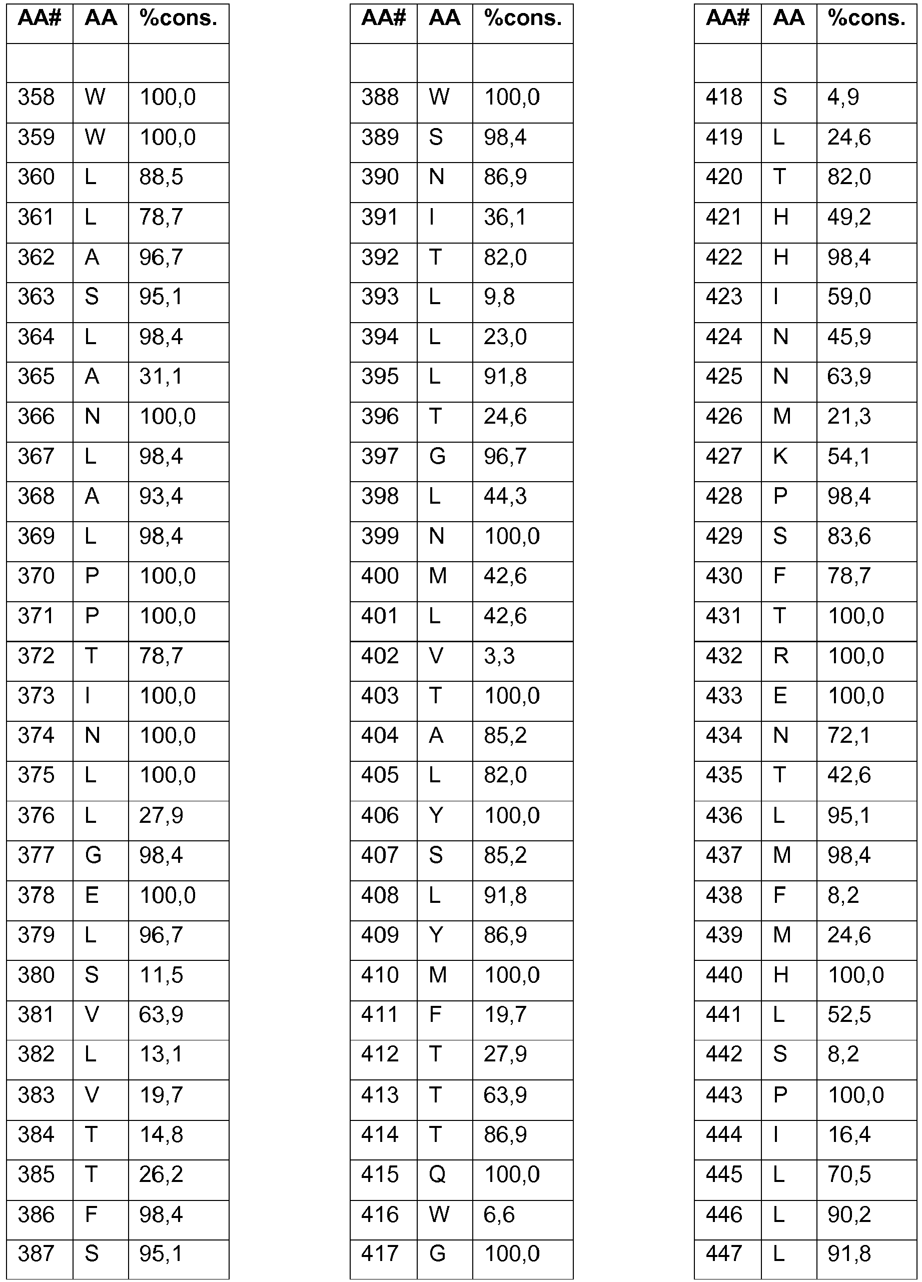

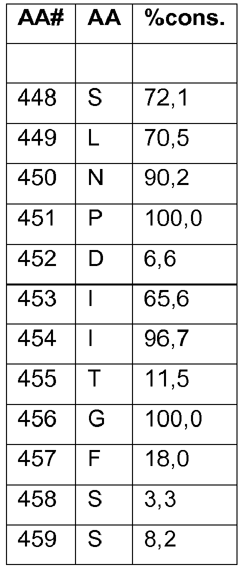

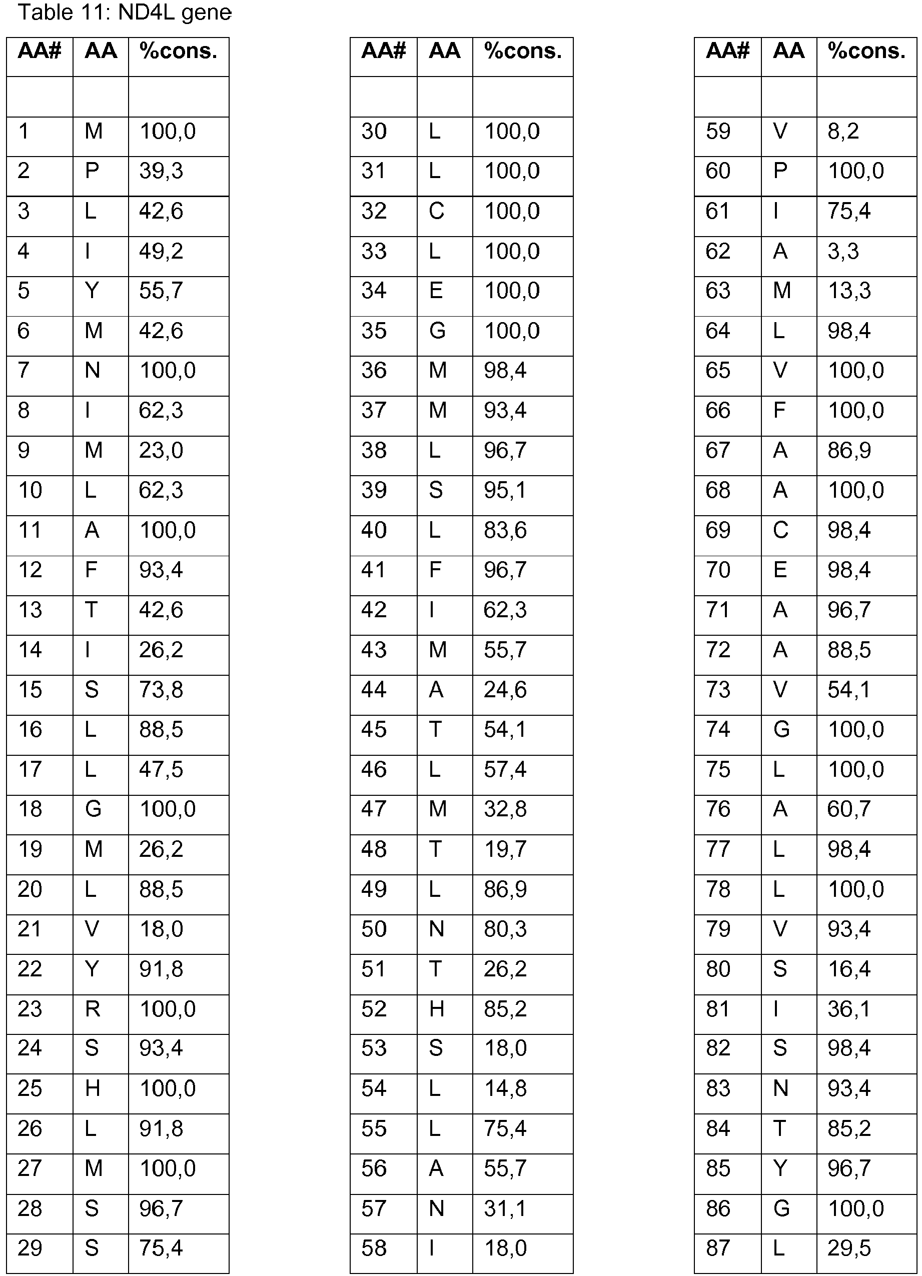

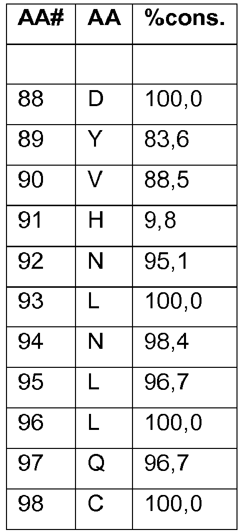

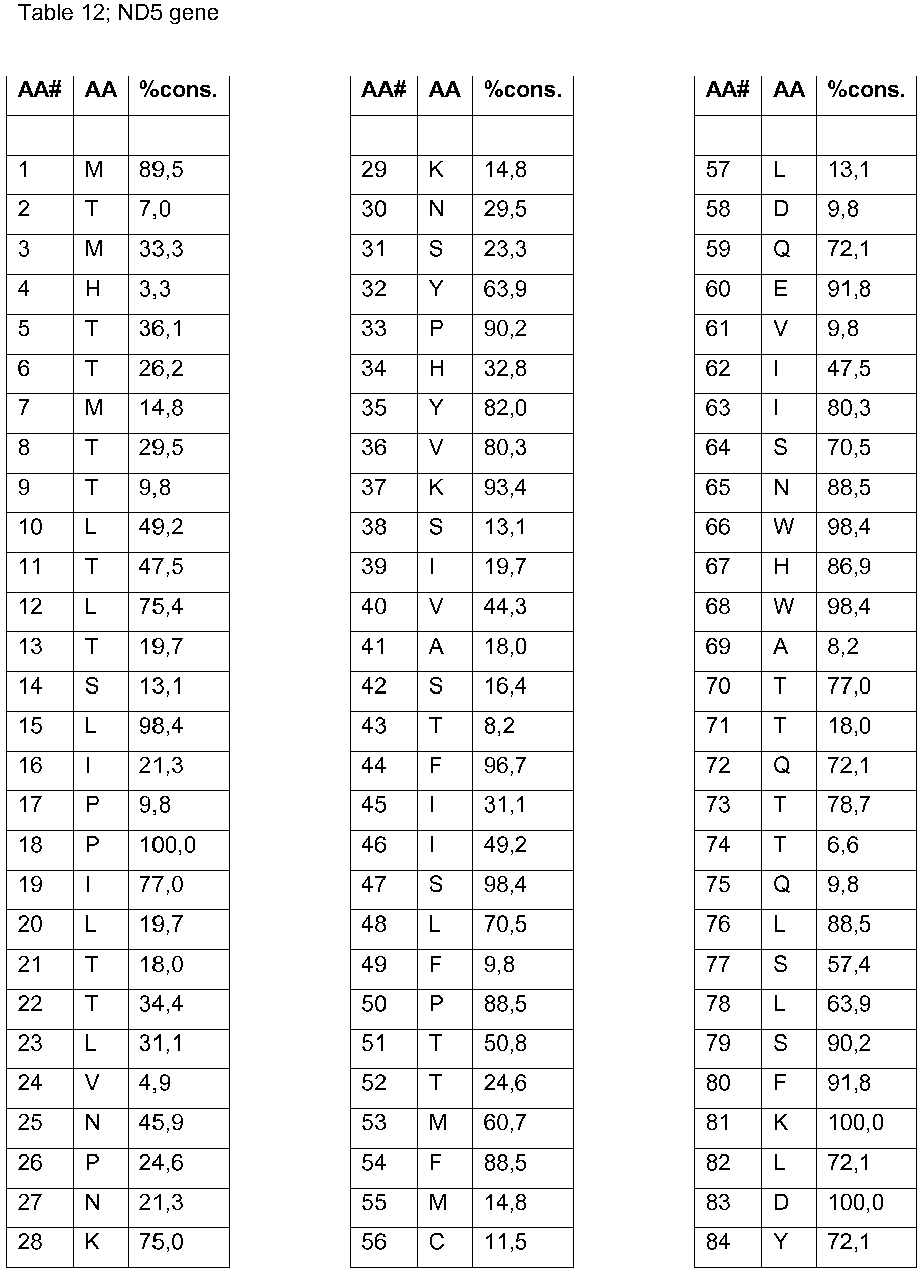

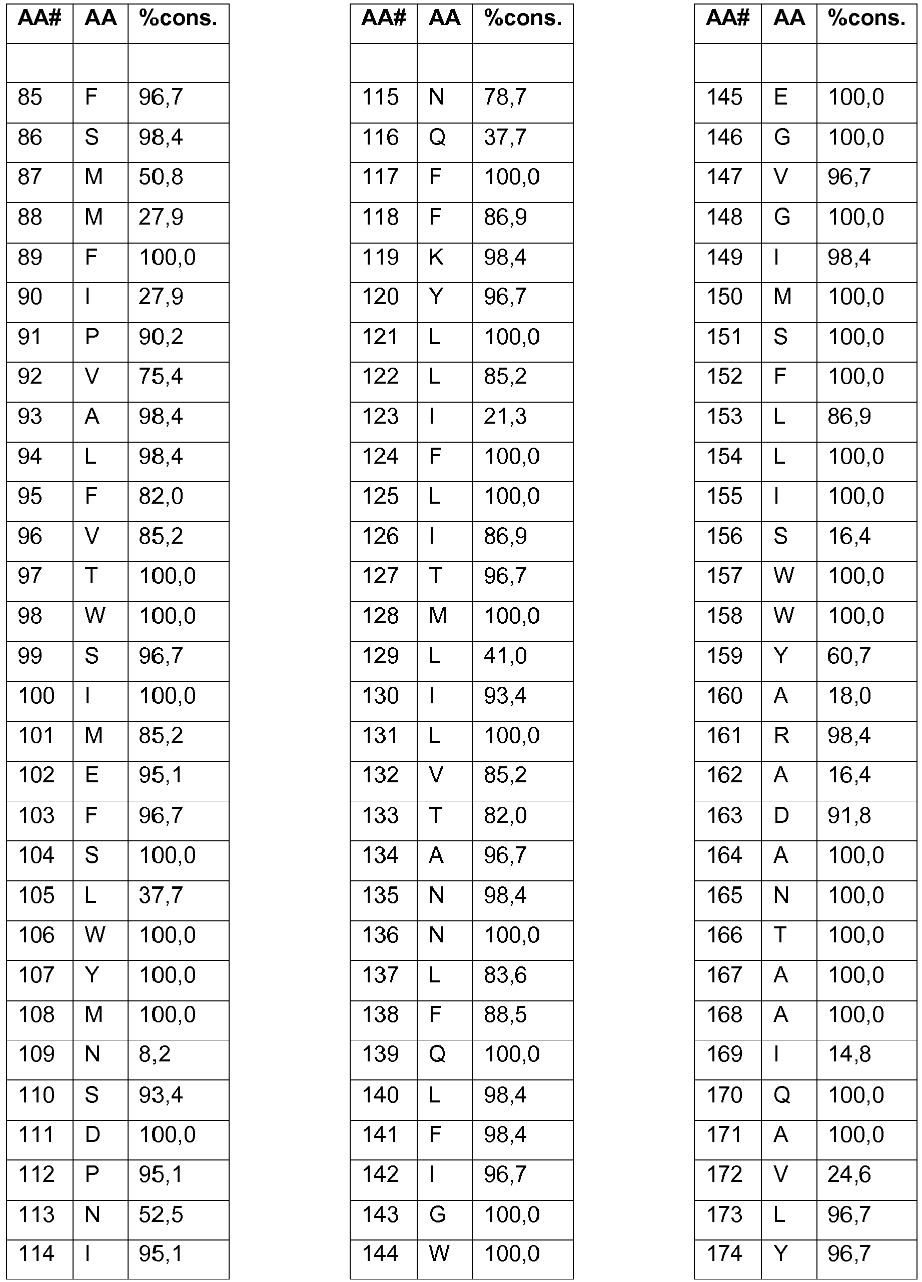

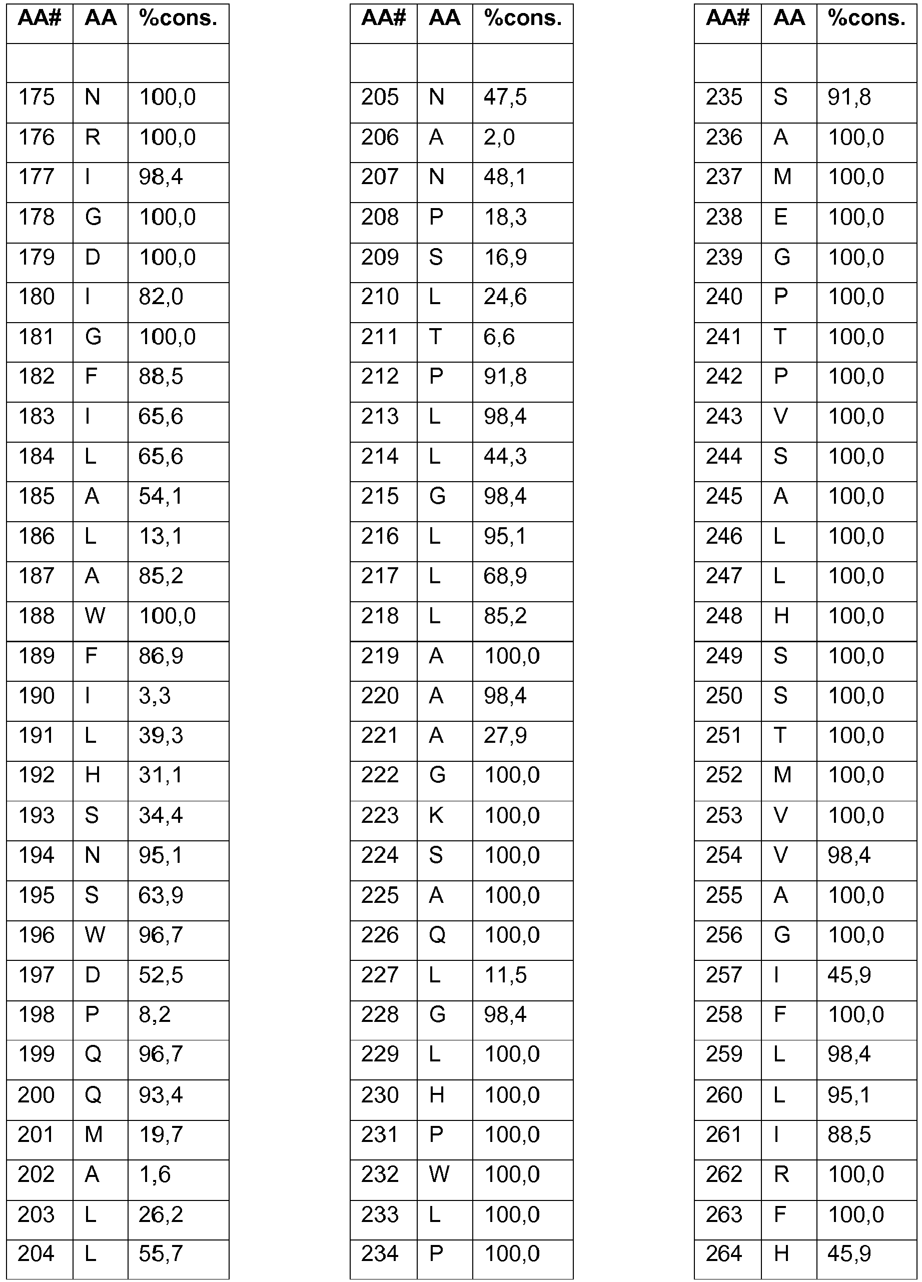

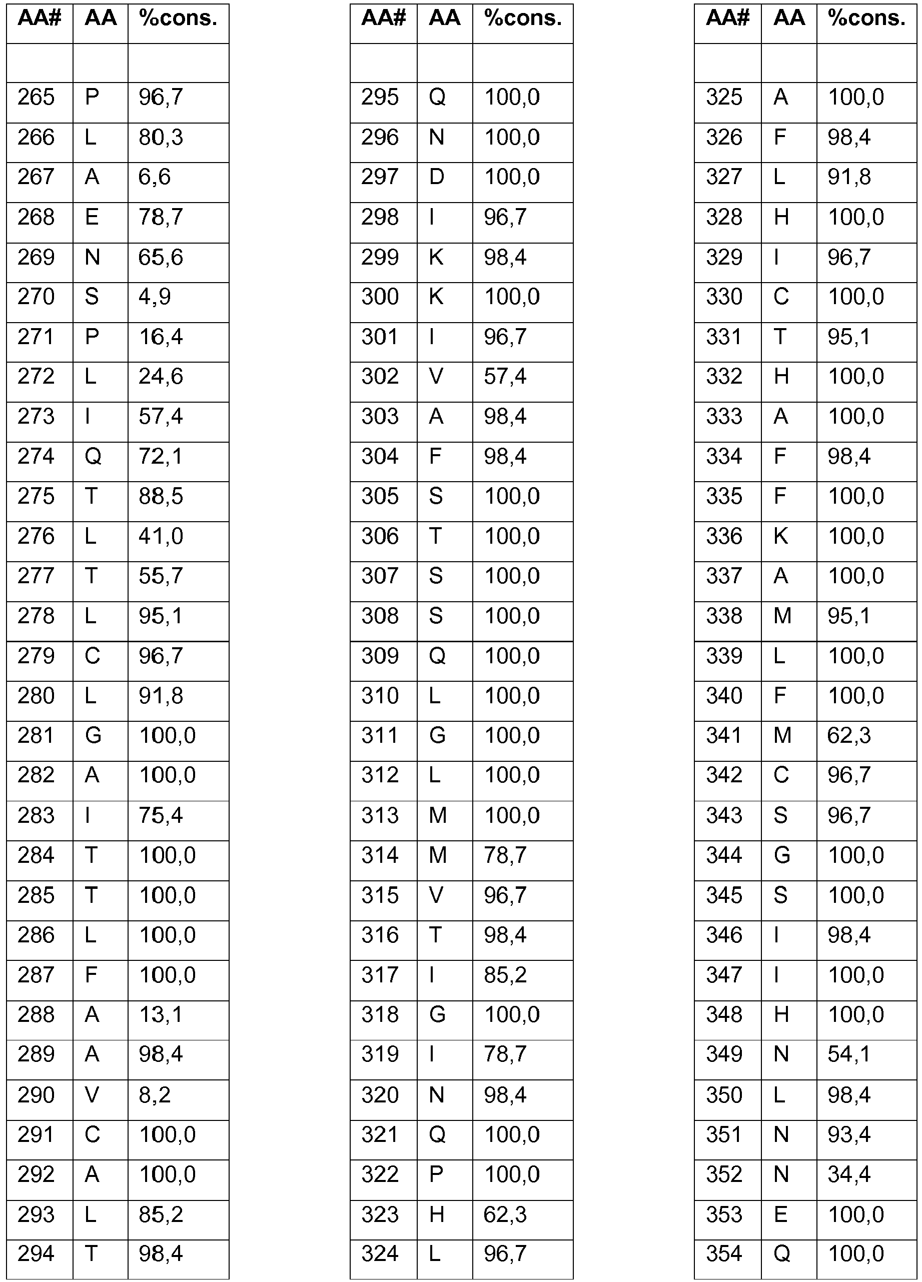

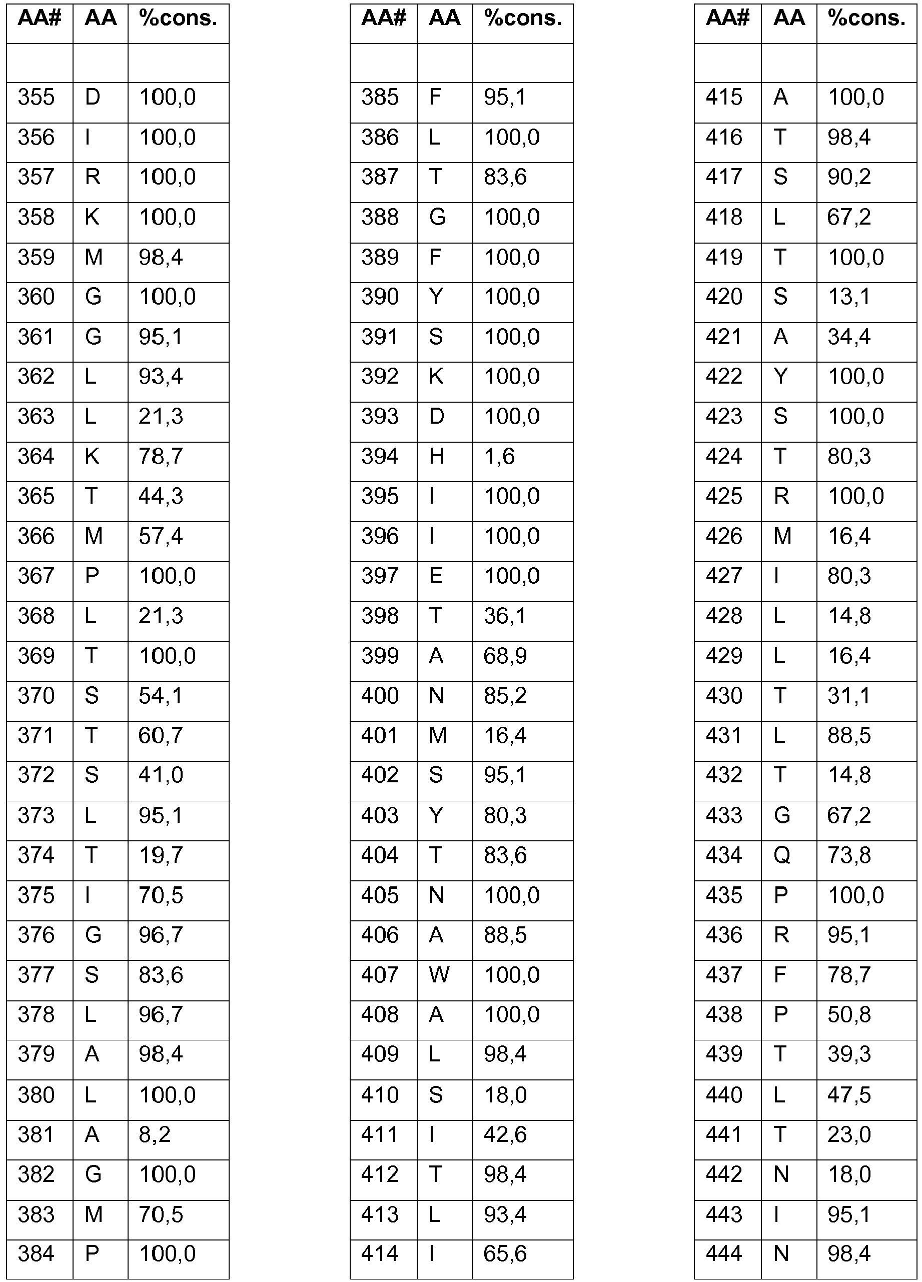

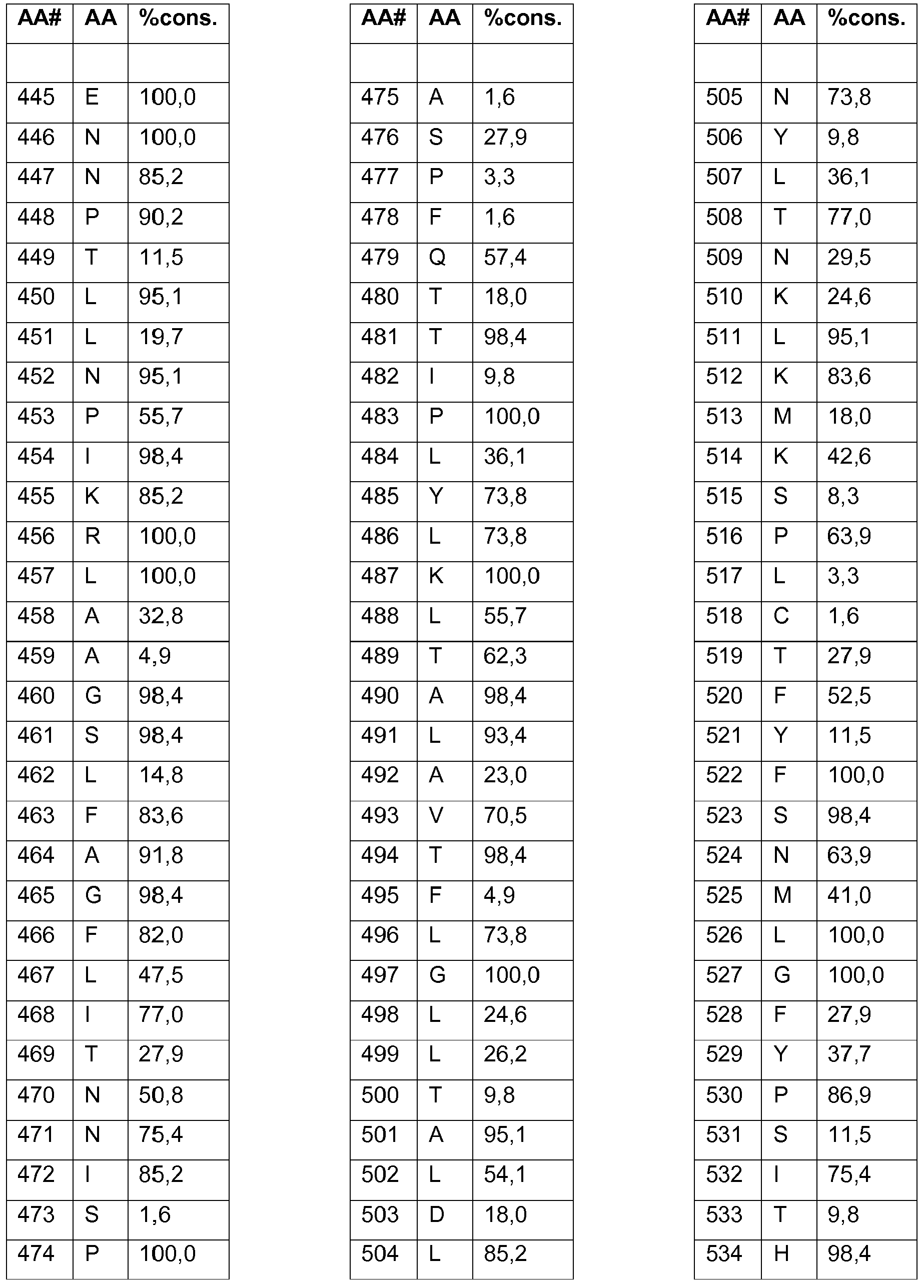

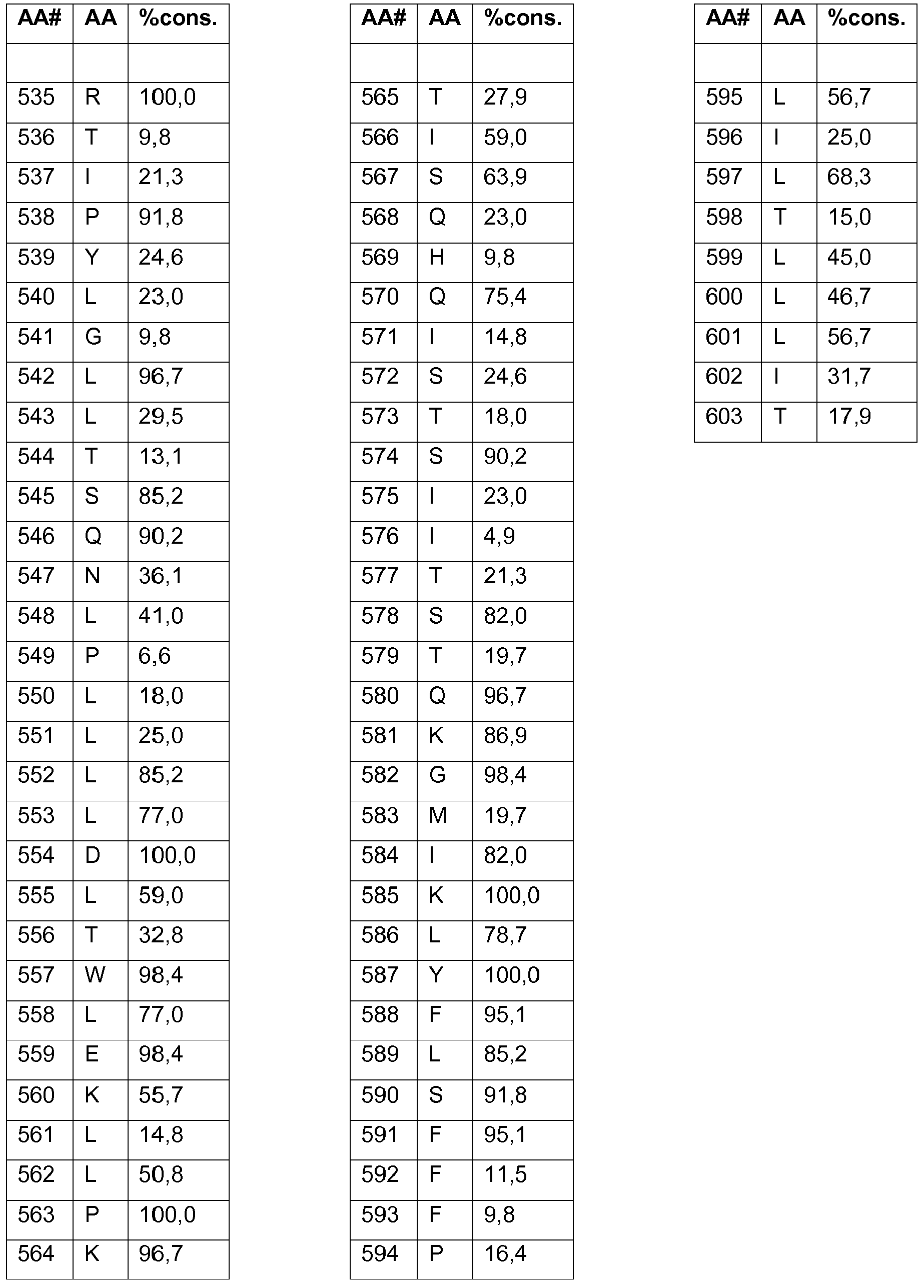

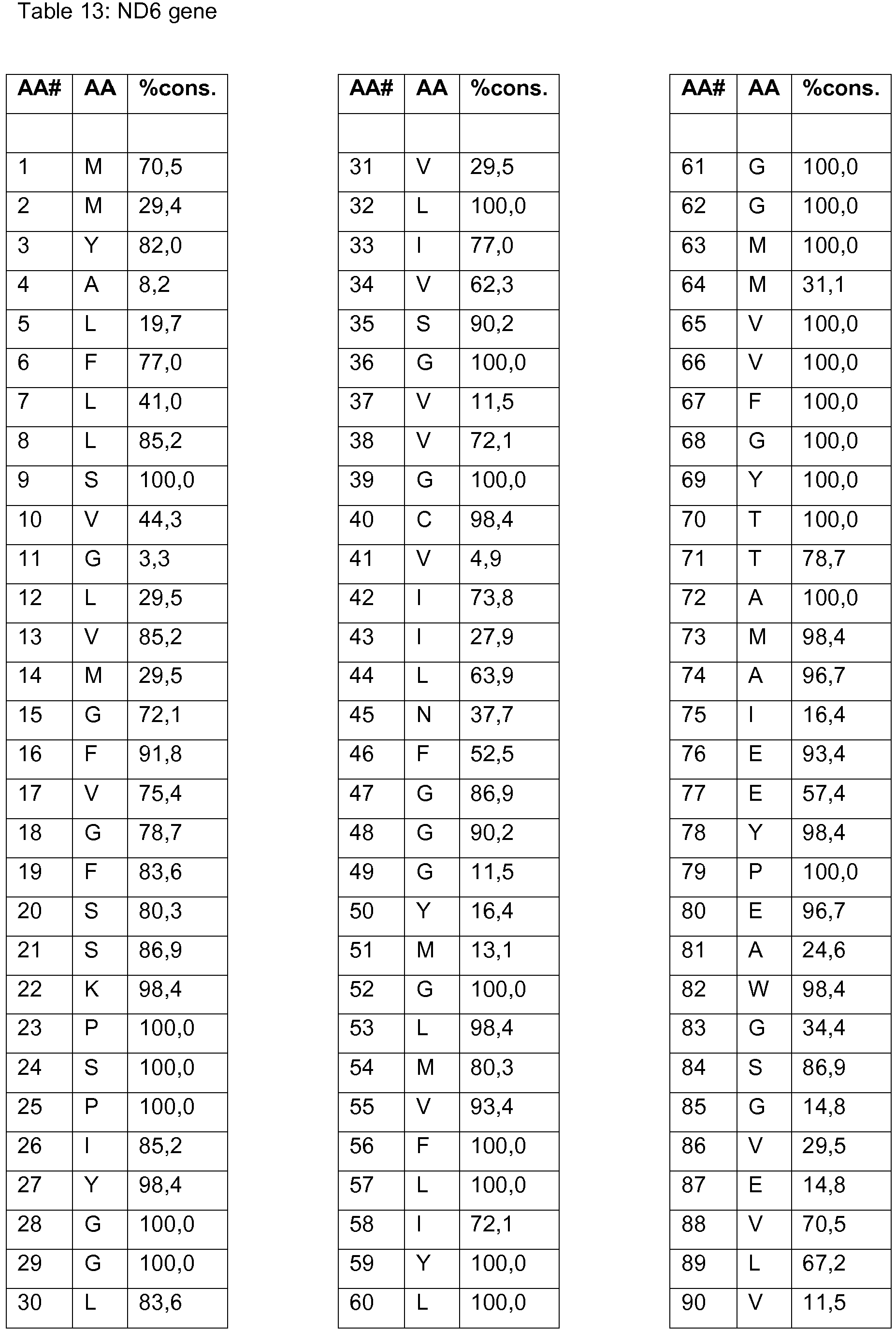

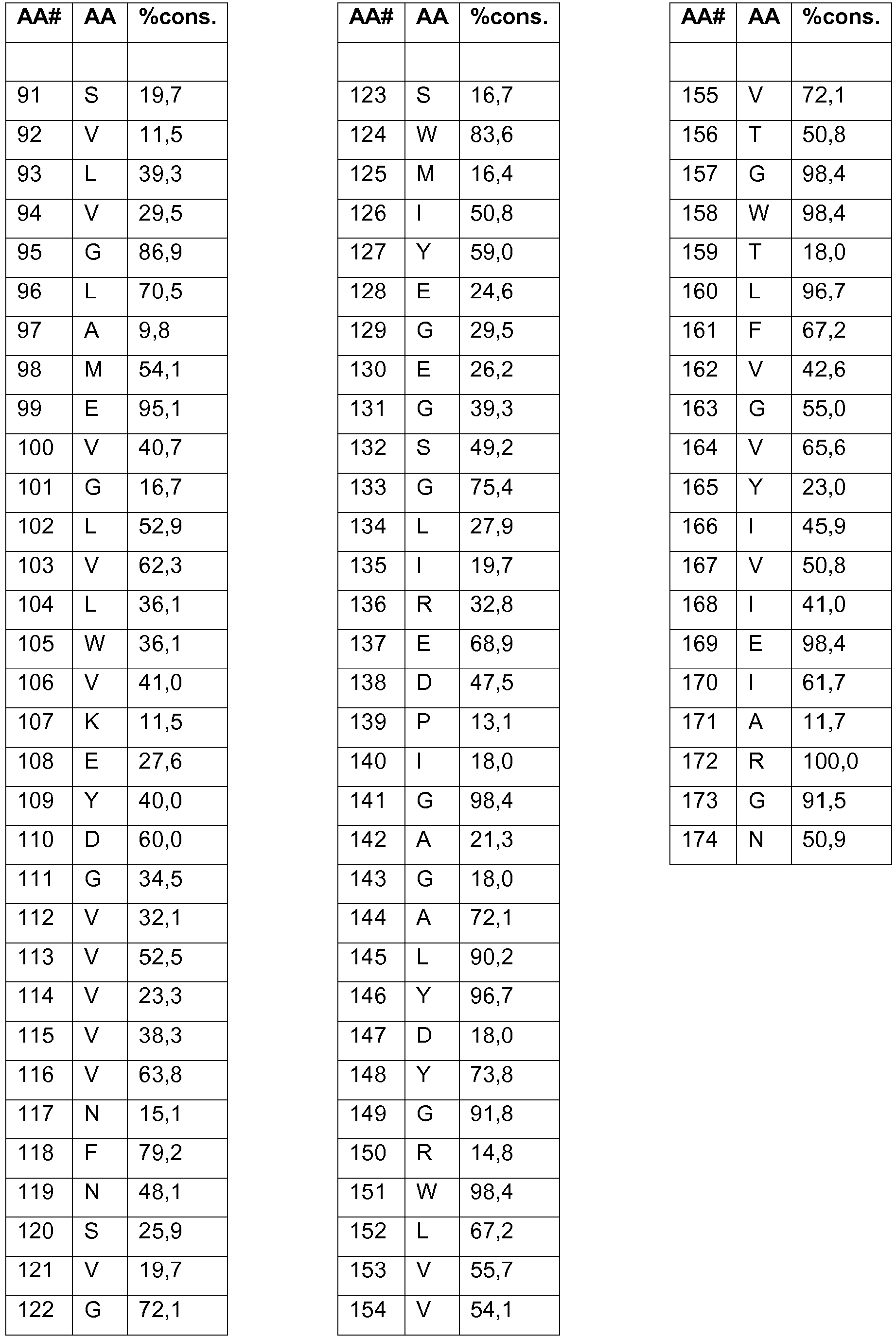

**Table 14; Determination of amino acid positions in a mitochondrial gene.**

| Gene Name | First Coding Nucleotide Position (FCNP) | Last Coding Nucleotide Position | Amino Acids | SEQ ID NO: |
|---|---|---|---|---|
| ND1 | 3307 | 4260 | 1 - 318 | 2 |
| ND2 | 4470 | 5510 | 1 - 347 | 3 |
| CO1 | 5904 | 7442 | 1 - 513 | 4 |
| CO2 | 7586 | 8266 | 1 - 227 | 5 |
| ATP8 | 8366 | 8569 | 1 - 68 | 6 |
| ATP6 | 8527 | 9204 | 1 - 226 | 7 |
| CO3 | 9207 | 9989 | 1 - 261 | 8 |
| ND3 | 10059 | 10403 | 1 - 115 | 9 |
| ND4L | 10470 | 10763 | 1 - 98 | 10 |
| ND4 | 10760 | 12136 | 1 - 459 | 11 |
| ND5 | 12337 | 14145 | 1 - 603 | 12 |
| ND6 | 14673 | 14152 | 1 - 174 | 13 |
| CytB | 14747 | 15886 | 1 - 380 | 14 |

**Table 15 Nucleotide positions of loops of tRNAs**

| **tRNA** | **D-loop** | **Anti-CD loop** | **T-loop** |
|---|---|---|---|
| Phe | 590-598 | 609-615 | 628-636 |
| Val | 1615-1620 | 1631-1637 | 1652-1657 |
| Ile | 4275-4280 | 4290-4296 | 4312-4318 |
| Met | 4415-4419 | 4430-4436 | 4451-4456 |
| Trp | 5525-5531 | 5542-5548 | 5561-5568 |
| Asp | 7531-7535 | 7546-7552 | 7567-7572 |
| Lys | 8307-8311 | 8321-8327 | 8343-8351 |
| Gly | 10004-10008 | 10019-10025 | 10039-10046 |
| Arg | 10418-10422 | 10433-10439 | 10453-10457 |
| His | 12151-12155 | 12166-12172 | 12187-12193 |
| Ser (AGY) | 12214-12217 | 12224-12230 | 12245-12252 |
| Thr | 15900-15907 | 15917-15932 | 15937-15941 |
| Pro | 16010-16005 | 15994-15998 | 15974-15968 |
| Glu | 14729-14725 | 14714-14708 | 14693-14687 |
| Tyr | 5879-5874 | 5864-5858 | 5843-5839 |
| Cys | 5814-5810 | 5800-5794 | 5780-5773 |
| Asn | 5717-5708 | 5698-5692 | 5676-5670 |
| Ala | 5642-5638 | 5627-5621 | 5606-5600 |
| Leu (UUR) | 3243-3252 | 32633269 | 3285-3291 |
| Leu (CUN) | 12279-12285 | 12296-12302 | 12317-12323 |
| Ser (UCN) | 7502-7498 | 7486-7480 | 7465-7459 |
| Gln | 43874380 | 4369-4363 | 4384-4342 |

**Table 16: Raw data from 321 patients**

| **Patient #** | **# non-synonymous variants** | **# non-synonymous variants in genes <=90% conserved** | **# variants tRNA genes** | **Baseline dyspnea score** | **RILT** |
|---|---|---|---|---|---|
| 1 | 3 | 3 | 0 | 2 | YES |
| 2 | 3 | 2 | 0 | 0 | NO |
| 3 | 2 | 2 | 0 | 0 | NO |
| 4 | 0 | 0 | 0 | 3 | YES |
| 5 | 3 | 3 | 0 | 1 | NO |
| 6 | 8 | 7 | 0 | 1 | YES |
| 7 | 6 | 5 | 0 | 0 | NO |
| 8 | 3 | 2 | 0 | 1 | NO |
| 9 | 9 | 8 | 0 | 1 | NO |
| 10 | 2 | 2 | 0 | 1 | YES |
| 11 | 4 | 4 | 0 | 0 | NO |
| 12 | 2 | 2 | 0 | 3 | YES |
| 13 | 4 | 3 | 1 | 0 | NO |
| 14 | 8 | 5 | 1 | 3 | YES |
| 15 | 4 | 4 | 0 | 1 | NO |
| 16 | 8 | 7 | 0 | 0 | NO |
| 17 | 6 | 5 | 1 | 0 | NO |
| 18 | 3 | 3 | 0 | 0 | NO |
| 19 | 6 | 6 | 1 | 2 | YES |
| 20 | 2 | 2 | 0 | 2 | NO |
| 21 | 2 | 2 | 0 | 1 | NO |
| 22 | 10 | 9 | 1 | 0 | NO |
| 23 | 7 | 6 | 1 | 0 | NO |
| 24 | 3 | 2 | 0 | 0 | NO |
| 25 | 4 | 4 | 1 | 1 | YES |
| 26 | 5 | 5 | 1 | 1 | NO |
| 27 | 10 | 10 | 0 | 0 | NO |
| 28 | 7 | 7 | 1 | 1 | YES |
| 29 | 2 | 2 | 0 | 1 | NO |
| 30 | 9 | 8 | 1 | 0 | NO |
| 31 | 6 | 5 | 0 | 1 | NO |
| 32 | 2 | 2 | 0 | 0 | NO |
| 33 | 2 | 2 | 0 | 0 | NO |
| 34 | 2 | 2 | 0 | 1 | NO |
| 35 | 6 | 5 | 1 | 1 | NO |
| 36 | 2 | 2 | 0 | 1 | NO |
| 37 | 2 | 2 | 0 | 1 | YES |
| 38 | 4 | 4 | 0 | 0 | NO |
| 39 | 4 | 3 | 0 | 1 | YES |
| 40 | 10 | 9 | 1 | 2 | YES |
| 41 | 8 | 8 | 0 | 1 | YES |
| 42 | 7 | 7 | 0 | 1 | NO |
| 43 | 3 | 3 | 0 | 1 | NO |
| 44 | 2 | 2 | 0 | 1 | NO |
| 45 | 2 | 2 | 0 | 1 | NO |
| 46 | 6 | 6 | 2 | 0 | NO |
| 47 | 8 | 7 | 0 | 0 | NO |
| 48 | 2 | 2 | 0 | 0 | NO |
| 49 | 7 | 7 | 0 | 0 | NO |
| 50 | 9 | 8 | 1 | 0 | NO |
| 51 | 2 | 2 | 0 | 0 | NO |
| 52 | 8 | 8 | 0 | 2 | YES |
| 53 | 6 | 6 | 0 | 1 | NO |
| 54 | 3 | 3 | 0 | 1 | NO |
| 55 | 4 | 4 | 1 | 1 | NO |
| 56 | 9 | 9 | 0 | 0 | NO |
| 57 | 2 | 2 | 0 | 1 | NO |
| 58 | 7 | 6 | 0 | 1 | NO |
| 59 | 3 | 2 | 0 | 1 | NO |
| 60 | 3 | 3 | 0 | 0 | NO |
| 61 | 6 | 5 | 1 | 1 | NO |
| 62 | 7 | 6 | 1 | 0 | NO |
| 63 | 11 | 11 | 0 | 1 | NO |
| 64 | 4 | 4 | 1 | 0 | NO |
| 65 | 10 | 10 | 1 | 1 | YES |
| 66 | 3 | 3 | 0 | 0 | NO |
| 67 | 5 | 5 | 1 | 2 | YES |
| 68 | 6 | 5 | 0 | 1 | YES |
| 69 | 3 | 2 | 0 | 1 | YES |
| 70 | 2 | 2 | 1 | 0 | NO |
| 71 | 3 | 3 | 0 | 1 | NO |
| 72 | 9 | 9 | 0 | 1 | NO |
| 73 | 2 | 2 | 0 | 0 | NO |
| 74 | 3 | 2 | 0 | 0 | YES |
| 75 | 6 | 5 | 1 | 0 | NO |
| 76 | 6 | 5 | 1 | 1 | YES |
| 77 | 2 | 2 | 0 | 0 | NO |
| 78 | 7 | 6 | 0 | 1 | NO |
| 79 | 3 | 3 | 0 | 0 | NO |
| 80 | 3 | 2 | 0 | 0 | NO |
| 81 | 11 | 10 | 0 | 0 | YES |
| 82 | 3 | 3 | 0 | 1 | NO |
| 83 | 6 | 5 | 0 | 0 | NO |
| 84 | 3 | 3 | 0 | 0 | NO |
| 85 | 6 | 6 | 0 | 1 | NO |
| 86 | 2 | 2 | 0 | 1 | NO |
| 87 | 10 | 10 | 0 | 1 | NO |
| 88 | 7 | 7 | 1 | 2 | YES |
| 89 | 10 | 11 | 0 | 3 | YES |
| 90 | 2 | 2 | 0 | 1 | NO |
| 91 | 6 | 6 | 0 | 3 | YES |
| 92 | 6 | 5 | 0 | 1 | YES |
| 93 | 3 | 4 | 1 | 0 | NO |
| 94 | 2 | 2 | 0 | 0 | NO |
| 95 | 2 | 2 | 0 | 1 | NO |
| 96 | 14 | 13 | 0 | 0 | YES |
| 97 | 3 | 3 | 0 | 1 | NO |
| 98 | 3 | 3 | 0 | 0 | NO |
| 99 | 4 | 3 | 0 | 0 | NO |
| 100 | 8 | 8 | 1 | 0 | NO |
| 101 | 9 | 9 | 1 | 1 | NO |
| 102 | 4 | 3 | 0 | 0 | NO |
| 103 | 2 | 2 | 0 | 0 | NO |
| 104 | 3 | 3 | 0 | 0 | NO |
| 105 | 7 | 6 | 2 | 0 | NO |
| 106 | 2 | 2 | 0 | 2 | NO |
| 107 | 2 | 2 | 0 | 0 | NO |
| 108 | 3 | 2 | 0 | 0 | NO |
| 109 | 2 | 2 | 1 | 1 | YES |
| 110 | 3 | 3 | 0 | 1 | NO |
| 111 | 10 | 9 | 1 | 0 | NO |
| 112 | 6 | 6 | 0 | 0 | NO |
| 113 | 3 | 4 | 0 | 0 | YES |
| 114 | 8 | 7 | 0 | 0 | NO |
| 115 | 2 | 2 | 0 | 2 | YES |
| 116 | 2 | 2 | 0 | 1 | NO |
| 117 | 6 | 5 | 0 | 0 | NO |
| 118 | 2 | 2 | 0 | 1 | YES |
| 119 | 2 | 2 | 1 | 1 | YES |
| 120 | 6 | 5 | 1 | 1 | NO |
| 121 | 6 | 5 | 1 | 1 | YES |
| 122 | 6 | 5 | 1 | 2 | NO |
| 123 | 2 | 2 | 0 | 1 | YES |
| 124 | 4 | 3 | 0 | 1 | NO |
| 125 | 4 | 4 | 0 | 1 | NO |
| 126 | 3 | 3 | 1 | 1 | YES |
| 127 | 7 | 6 | 1 | 0 | NO |
| 128 | 3 | 2 | 0 | 1 | NO |
| 129 | 9 | 6 | 1 | NA | NO |
| 130 | 2 | 2 | 0 | 0 | NO |
| 131 | 10 | 10 | 0 | 0 | NO |
| 132 | 3 | 3 | 0 | 0 | NO |
| 133 | 5 | 4 | 2 | 0 | NO |
| 134 | 2 | 2 | 0 | 0 | NO |
| 135 | 2 | 2 | 0 | 0 | NO |
| 136 | 3 | 3 | 0 | 0 | NO |
| 137 | 3 | 3 | 0 | 1 | NO |
| 138 | 7 | 6 | 1 | 0 | NO |
| 139 | 3 | 2 | 0 | 0 | NO |
| 140 | 8 | 7 | 0 | 1 | NO |
| 141 | 6 | 5 | 0 | 2 | YES |
| 142 | 2 | 2 | 1 | 0 | NO |
| 143 | 6 | 5 | 1 | 3 | NO |
| 144 | 5 | 3 | 2 | 2 | NO |
| 145 | 8 | 7 | 0 | 0 | NO |
| 146 | 3 | 3 | 0 | 1 | NO |
| 147 | 4 | 4 | 1 | 0 | NO |
| 148 | 2 | 2 | 0 | NA | NO |
| 149 | 6 | 5 | 0 | 0 | NO |
| 150 | 2 | 2 | 0 | 0 | NO |
| 151 | 8 | 8 | 0 | 0 | NO |
| 152 | 3 | 2 | 0 | 2 | NO |
| 153 | 2 | 2 | 0 | 1 | NO |
| 154 | 2 | 2 | 0 | 1 | NO |
| 155 | 5 | 5 | 1 | 1 | NO |
| 156 | 14 | 13 | 0 | 1 | NO |
| 157 | 7 | 6 | 1 | 0 | NO |
| 158 | 9 | 7 | 1 | 1 | NO |
| 159 | 3 | 3 | 0 | 0 | NO |
| 160 | 3 | 2 | 0 | 0 | NO |
| 161 | 2 | 2 | 0 | 0 | NO |
| 162 | 2 | 2 | 1 | 1 | NO |
| 163 | 3 | 2 | 0 | 0 | NO |
| 164 | 6 | 5 | 0 | 0 | YES |
| 165 | 6 | 5 | 1 | 1 | NO |
| 166 | 3 | 3 | 0 | 1 | NO |
| 167 | 6 | 5 | 0 | 1 | NO |
| 168 | 6 | 5 | 0 | 0 | NO |
| 169 | 6 | 5 | 1 | 1 | NO |
| 170 | 3 | 2 | 0 | 1 | NO |
| 171 | 7 | 6 | 0 | 0 | NO |
| 172 | 2 | 2 | 0 | 1 | NO |
| 173 | 7 | 7 | 0 | 2 | YES |
| 174 | 2 | 2 | 0 | 1 | NO |
| 175 | 8 | 7 | 1 | 0 | NO |
| 176 | 3 | 3 | 0 | 1 | NO |
| 177 | 9 | 8 | 1 | 2 | YES |
| 178 | 6 | 5 | 1 | 1 | YES |
| 179 | 3 | 2 | 0 | 1 | NO |
| 180 | 7 | 6 | 2 | 1 | YES |
| 181 | 2 | 2 | 0 | 2 | YES |
| 182 | 2 | 2 | 0 | 1 | NO |
| 183 | 3 | 3 | 0 | 0 | NO |
| 184 | 2 | 2 | 0 | 1 | NO |
| 185 | 2 | 2 | 1 | 1 | NO |
| 186 | 8 | 8 | 0 | 1 | NO |
| 187 | 2 | 2 | 0 | 0 | NO |
| 188 | 3 | 3 | 1 | 0 | YES |
| 189 | 6 | 5 | 0 | 2 | NO |
| 190 | 6 | 5 | 0 | 0 | NO |
| 191 | 2 | 2 | 0 | 1 | NO |
| 192 | 2 | 2 | 0 | 4 | YES |
| 193 | 2 | 2 | 0 | 1 | YES |
| 194 | 8 | 7 | 1 | 1 | YES |
| 195 | 2 | 2 | 0 | 0 | NO |
| 196 | 2 | 2 | 0 | 3 | NO |
| 197 | 3 | 2 | 0 | 1 | YES |
| 198 | 3 | 2 | 0 | 1 | NO |
| 199 | 4 | 4 | 1 | 3 | YES |
| 200 | 8 | 7 | 0 | 0 | YES |
| 201 | 11 | 8 | 0 | 0 | NO |
| 202 | 3 | 3 | 0 | 1 | NO |
| 203 | 7 | 6 | 1 | 0 | NO |
| 204 | 2 | 2 | 0 | NA | NO |
| 205 | 9 | 8 | 1 | 1 | YES |
| 206 | 4 | 4 | 1 | 0 | NO |
| 207 | 3 | 3 | 1 | 1 | YES |
| 208 | 9 | 8 | 1 | NA | NO |
| 209 | 6 | 5 | 1 | 1 | NO |
| 210 | 6 | 5 | 2 | 2 | YES |
| 211 | 10 | 10 | 0 | 1 | YES |
| 212 | 3 | 2 | 0 | 0 | NO |
| 213 | 2 | 2 | 0 | 1 | NO |
| 214 | 2 | 2 | 0 | 1 | NO |
| 215 | 2 | 2 | 0 | 0 | NO |
| 216 | 4 | 4 | 1 | 2 | YES |
| 217 | 4 | 4 | 1 | 0 | NO |
| 218 | 6 | 5 | 0 | 2 | NO |
| 219 | 3 | 2 | 0 | 1 | NO |
| 220 | 2 | 2 | 0 | 2 | NO |
| 221 | 5 | 4 | 1 | 2 | YES |
| 222 | 7 | 7 | 0 | 1 | YES |
| 223 | 8 | 5 | 1 | 1 | NO |
| 224 | 2 | 2 | 0 | 0 | NO |
| 225 | 3 | 3 | 0 | NA | NO |
| 226 | 9 | 9 | 0 | 1 | NO |
| 227 | 2 | 2 | 0 | 0 | NO |
| 228 | 2 | 2 | 1 | 0 | NO |
| 229 | 2 | 2 | 1 | 0 | NO |
| 230 | 7 | 7 | 1 | 3 | YES |
| 231 | 4 | 4 | 0 | 0 | NO |
| 232 | 6 | 5 | 1 | 1 | YES |
| 233 | 2 | 2 | 0 | 1 | NO |
| 234 | 2 | 2 | 0 | 1 | NO |
| 235 | 7 | 6 | 2 | 2 | YES |
| 236 | 2 | 2 | 0 | 0 | NO |
| 237 | 7 | 7 | 1 | 0 | YES |
| 238 | 7 | 6 | 0 | 1 | NO |
| 239 | 2 | 2 | 0 | 0 | NO |
| 240 | 3 | 3 | 0 | 0 | NO |
| 241 | 7 | 5 | 3 | 1 | NO |
| 242 | 2 | 2 | 0 | 0 | NO |
| 243 | 9 | 9 | 0 | 0 | NO |
| 244 | 3 | 2 | 0 | 1 | NO |
| 245 | 6 | 6 | 1 | 1 | YES |
| 246 | 2 | 2 | 0 | 1 | NO |
| 247 | 3 | 2 | 0 | 1 | NO |
| 248 | 3 | 3 | 0 | 0 | NO |
| 249 | 4 | 3 | 0 | 1 | NO |
| 250 | 6 | 4 | 1 | 1 | YES |
| 251 | 6 | 5 | 1 | 1 | YES |
| 252 | 6 | 5 | 0 | 2 | NO |
| 253 | 2 | 2 | 0 | 1 | NO |
| 254 | 9 | 9 | 0 | 3 | YES |
| 255 | 2 | 2 | 0 | 1 | NO |
| 256 | 0 | 0 | 0 | 1 | YES |
| 257 | 6 | 6 | 2 | 4 | YES |
| 258 | 11 | 8 | 0 | 1 | NO |
| 259 | 3 | 3 | 1 | 1 | NO |
| 260 | 4 | 3 | 1 | 1 | NO |
| 261 | 11 | 11 | 0 | 1 | NO |
| 262 | 6 | 5 | 0 | 0 | YES |
| 263 | 2 | 2 | 0 | 1 | YES |
| 264 | 5 | 4 | 2 | 1 | NO |
| 265 | 2 | 2 | 0 | 0 | NO |
| 266 | 7 | 7 | 1 | 0 | NO |
| 267 | 3 | 3 | 0 | 1 | NO |
| 268 | 7 | 6 | 2 | 2 | NO |
| 269 | 6 | 6 | 2 | 1 | NO |
| 270 | 6 | 6 | 2 | 1 | NO |
| 271 | 2 | 2 | 0 | 1 | YES |
| 272 | 2 | 2 | 0 | 1 | NO |
| 273 | 6 | 5 | 0 | 1 | YES |
| 274 | 2 | 2 | 0 | 0 | NO |
| 275 | 2 | 2 | 1 | 2 | NO |
| 276 | 2 | 2 | 0 | 1 | NO |
| 277 | 2 | 2 | 0 | 0 | NO |
| 278 | 5 | 4 | 1 | 1 | NO |
| 279 | 2 | 2 | 0 | 3 | YES |
| 280 | 2 | 2 | 1 | 1 | NO |
| 281 | 7 | 6 | 2 | 1 | NO |
| 282 | 3 | 3 | 0 | 1 | NO |
| 283 | 3 | 2 | 0 | 0 | NO |
| 284 | 2 | 2 | 0 | 0 | NO |
| 285 | 5 | 5 | 1 | 1 | NO |
| 286 | 5 | 5 | 1 | 1 | NO |
| 287 | 2 | 2 | 0 | 1 | NO |
| 288 | 7 | 6 | 1 | 1 | NO |
| 289 | 7 | 6 | 1 | 2 | YES |
| 290 | 8 | 7 | 1 | 0 | NO |
| 291 | 3 | 3 | 0 | 3 | YES |
| 292 | 2 | 2 | 0 | 1 | NO |
| 293 | 6 | 5 | 2 | 1 | YES |
| 294 | 6 | 5 | 1 | 2 | NO |
| 295 | 5 | 5 | 0 | 1 | NO |
| 296 | 3 | 3 | 0 | 0 | NO |
| 297 | 6 | 6 | 0 | 1 | NO |
| 298 | 3 | 3 | 0 | 0 | YES |
| 299 | 10 | 10 | 0 | 1 | NO |
| 300 | 6 | 6 | 0 | 1 | NO |
| 301 | 4 | 3 | 0 | 0 | NO |
| 302 | 3 | 3 | 0 | 0 | YES |
| 303 | 2 | 2 | 0 | 0 | NO |
| 304 | 8 | 8 | 0 | 2 | YES |
| 305 | 3 | 3 | 0 | 0 | NO |
| 306 | 2 | 2 | 0 | 0 | NO |
| 307 | 2 | 2 | 0 | 1 | NO |
| 308 | 10 | 10 | 0 | 0 | NO |
| 309 | 9 | 7 | 1 | 0 | NO |
| 310 | 7 | 5 | 1 | 0 | NO |
| 311 | 3 | 2 | 0 | 0 | NO |
| 312 | 8 | 6 | 1 | 0 | NO |
| 313 | 3 | 3 | 0 | 0 | NO |
| 314 | 2 | 2 | 0 | 1 | NO |
| 315 | 4 | 4 | 0 | 2 | YES |
| 316 | 5 | 5 | 1 | 0 | NO |
| 317 | 3 | 3 | 0 | 0 | NO |
| 318 | 6 | 5 | 1 | 1 | NO |
| 319 | 3 | 3 | 0 | 1 | NO |
| 320 | 4 | 3 | 1 | 0 | NO |
| 321 | 2 | 2 | 0 | 0 | NO |

### Examples

### Example 1: Patient population and DNA isolation.

Patient were recruited from MAASTRO Clinic (n=321) and Ghent University Hospital (n=66). Lung toxicity was scored using the Common Terminology Criteria for Adverse Events version 3.0 for dyspnea before (baseline) and up to six months after (maximum) radiotherapy (RT). The study was approved by the ethics committees of both centers, and all study participants provided written informed consent.

Total (nuclear and mitochondrial) cellular DNA was isolated from blood of patients collected before radiotherapy according to standard procedures.

### Example 2: Sequence determination.

GeneChip® Mitochondria Resequencing 2.0 Arrays (Affymetrix, Santa Clara, CA, USA) were used to determine the mtDNA sequence of the DNA samples according to the manufacturer's protocol.

After generation of the cell intensity (CEL) files by GeneChip® Operating Software 1.4 (GCOS 1.4), raw sequence data was analyzed by the Sequence Pilot - module SeqC (JSI medical systems) with the standard parameters. The eventual mtDNA sequences were compared with the revised Cambridge reference sequence (SEQ ID NO: 1) to list all homoplasmic variants. The haplogroups were determined as described previously (Voets, A.M., et al. Large scale mtDNA sequencing reveals sequence and functional conservation as major determinants of homoplasmic mtDNA variant distribution. Mitochondrion 11, 964-972 (2011)).

### Example 3: Statistical analysis

Multivariate logistic regression analyses models were built with α = 0.05. The model performance for predicting outcome was evaluated by calculating the area under the curve of the receiver operating characteristic curve using a 10-fold cross-validation (out-of-sample) procedure. The maximum value of the AUC is 1.0, indicating a perfect discrimination, whereas 0.5 indicates a random chance to correctly discriminate outcome with the model. P values for AUCs being different from 0.5 were calculated using 500 bootstraps and a 1-sided Student's t-test. The final model output was represented by a nomogram (lasonos, A., Schrag, D., Raj, G.V. & Panageas, K.S. How to build and interpret a nomogram for cancer prognosis. J Clin Oncol 26, 1364-1370 (2008)).

The endpoint for the model was the occurrence of dyspnea≥2 within six months after RT.

## Claims

1. In vitro method for predicting the risk of developing radiation induced lung toxicity comprising the steps of:
a. obtaining mitochondrial DNA from a sample of a subject,
b. determining the number of non-synonymous variations present in at least one gene encoding a mitochondrial protein,
c. attributing a value to the number of non-synonymous variations,
wherein a higher value corresponds to a higher risk for developing radiation induced lung toxicity.

2. In vitro method according to claim 1 wherein said non-synonymous variations occur at positions that are less than 100% conserved, such as 99%, 98%, 97%, 96%, 95%, 94%, 93%, 92%, 91%, 90%, 89%, 88%, 87%, 86%, 85%, 84%, 83%, 82%, 81 %, 80% or less conserved.

3. In vitro method according to claim 2 wherein wherein said non-synonymous variations occur at positions that are less than 90% conserved.

4. In vitro method according to any one of claims 1 - 3 wherein the subject is diagnosed with lung cancer.

5. In vitro method according to any one of claims 1 - 4, additionally comprising a step of attributing a value to the baseline dyspnea score of the subject and adding that value to the value obtained from the number of non-synonymous variations to obtain an aggregated value, wherein a higher aggregated value corresponds to a higher risk of developing radiation induced lung toxicity.

6. In vitro method according to any one of claims 1 - 5, additionally comprising a step of determining the number of variations in tRNA loop positions, attributing a value to the number of variations in tRNA loop positions and adding that value to the value obtained from the number of non-synonymous variations or the aggregated value.

7. In vitro method according to any one of claims 1 - 6 wherein a value is attributed to the dose of chemotherapy that the subject is receiving wherein the value is proportional to the dose of chemotherapy, and adding that value to the value obtained from the number of non-synonymous variations or the aggregated value.

8. In vitro method according to any one of claims 1 - 7 wherein said at least one gene encoding a mitochondrial protein is 2 genes or more than 2 genes, such as 3 or 4, 5, 6, 7, 8, 9, 10, 11, 12 or all genes encoding a mitochondrial protein.

9. In vitro method according to any one of claims 1 - 8 wherein the sample comprises nucleated cells.

10. In vitro method according to claim 9 wherein the sample is a blood sample or a tissue sample.

11. In vitro method according to any one of claims 1 - 10 wherein the sample is taken from the subject before the start of the radiation therapy.
